(19) Europäisches Patentamt
European Patent Office
Office européen des brevets

(11) **EP 2 048 153 A1**

(12) **EUROPEAN PATENT APPLICATION**
published in accordance with Art. 153(4) EPC

(43) Date of publication:
**15.04.2009 Bulletin 2009/16**

(21) Application number: **07791500.7**

(22) Date of filing: **27.07.2007**

(51) Int Cl.:
***C07H 19/01*** *(2006.01)* ***A61K 31/7048*** *(2006.01)*
***A61P 3/04*** *(2006.01)* ***A61P 3/10*** *(2006.01)*
***A61P 43/00*** *(2006.01)*

(86) International application number:
**PCT/JP2007/064806**

(87) International publication number:
**WO 2008/013280 (31.01.2008 Gazette 2008/05)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **27.07.2006 JP 2006205153**

(71) Applicant: **CHUGAI SEIYAKU KABUSHIKI KAISHA**
**Tokyo, 115-8543 (JP)**

(72) Inventors:
- **SATO, Tsutomu**
**Gotenba-shi, Shizuoka 412-8513 (JP)**
- **HONDA, Kiyofumi**
**Gotenba-shi, Shizuoka 412-8513 (JP)**
- **KAWAI, Takahiro**
**Gotenba-shi, Shizuoka 412-8513 (JP)**
- **AHN, Koo Hyeon**
**Hwasung-kun, Kyunggi-do 445-380 (KR)**

(74) Representative: **Vossius & Partner**
**Siebertstraße 4**
**81675 München (DE)**

(54) **SUBSTITUTED SPIROKETAL DERIVATIVE AND USE THEREOF AS DRUG FOR TREATING DIABETES**

(57) The present invention provides a compound represented by Formula (II):

[Formula 1]

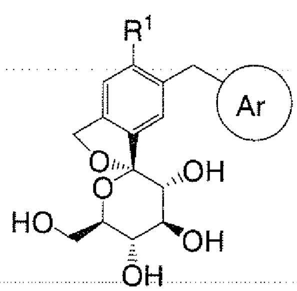

(II)

wherein $R^1$ is a chlorine atom, a fluorine atom, a methyl group or an ethynyl group;
Ar is a group represented by the following Formula (a), Formula (b), Formula (c) or Formula (d):

EP 2 048 153 A1

[Formula 2]

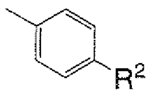

(a)

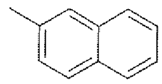

(b)

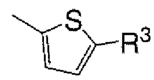

(c)

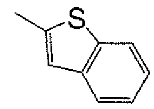

(d)

wherein $R^2$ is a $C_{1-6}$ alkyl group which may be substituted with one or more halogen atoms, a $C_{1-6}$ alkoxy group which may be substituted with one or more halogen atoms, a $C_{1-3}$ alkylthio group, a halogen atom, a $C_{1-3}$ alkylcarbonyl group or a $C_{2-5}$ alkynyl group which may be substituted with $-OR^4$;
$R^3$ is a hydrogen atom or a $C_{1-3}$ alkyl group;
$R^4$ is a hydrogen atom or a $C_{1-3}$ alkyl group;
provided that Ar is a group represented by Formula (a) when $R^1$ is a fluorine atom, methyl group or an ethynyl group, and that $R^2$ is methoxy group, an ethoxy group, an isopropyl group, a propyl group, a trifluoromethyl group, a trifluoromethoxy group, 2-fluoroethyl group or 1-propynyl group when $R^1$ is a methyl group or a pharmaceutically acceptable salt or a solvate thereof and a pharmaceutical agent, a pharmaceutical composition and so on comprising the compound.

## Description

[TECHNICAL FIELD]

The present invention relates to spiroketal derivatives useful as pharmaceutical agents, prodrugs thereof and pharmacologically acceptable salts thereof. Particularly, the present invention relates to spiroketal derivatives which inhibit $Na^+$-glucose cotransporter 2 (SGLT2) and are thereby useful as preventive or therapeutic agents for diabetes such as insulin-dependent diabetes (Type 1 diabetes), non-insulin-dependent diabetes (Type 2 diabetes), diabetic complications and diseases caused by hyperglycemia such as obesity, prodrugs thereof and salts thereof.

[BACKGROUND ART]

In late years, the number of diabetic patients has been increasing due to westernization of dietary habits, chronic lack of exercise and so on. Decrease in insulin secretion and insulin sensitivity is observed in diabetic patients, which is caused by chronic hyperglycosemia, further causes elevation of blood sugar level and leads to aggravation of symptoms. Biguanide drugs, sulphonylurea drugs, glycosidase inhibitors, insulin sensitizers, etc., have been used as therapeutic drugs for diabetes. However, side effects such as lactic acidosis as for biguanide drugs, hypoglycemia as for sulphonylurea drugs, diarrhea as for glycosidase inhibitors have been reported, and now the development of therapeutic drugs for diabetes according to new action mechanism different from these drugs is eagerly demanded.

It has been reported that Phloridzin, which is a naturally-occurring glucose derivative, inhibits sodium dependent glucose cotransporter 2 (SGLT2) occurring in SI site of the renal proximal tubule, and thereby inhibits reabsorption of excessive glucose in the kidney, promotes glucose excretion, and exhibits hypoglycemic action (refer to Non-Patent Document 1). Thereafter, up to the present, studies on the therapeutic drugs for diabetes based on SGLT2 inhibition has been extensively performed.

For example, compounds usable as inhibitors of SGLT2 are reported in JP 2000-080041 A (Patent Document 1), WO01/068660 (Patent Document 2), WO04/007517 (Patent Document 3), etc. However, Phloridzin and the compounds described in the above-mentioned patent applications are considered to be problematic in that when they are orally administered, they are readily hydrolyzed by glycosidase and the like present in the small intestine and the pharmacological effect thereof immediately disappears. In addition, as for Phloridzin, there has been reported that phloretin, which is the aglycone moiety thereof, strongly inhibits a sugar transporter of the facilitated diffusion type and causes bad influences such that the glucose concentration in the brain decreases when phloretin is administered to a rat vein (for example, refer to Non-Patent Document 2).

Therefore, attempts to convert the compounds to prodrugs have been made for the purpose of preventing such decomposition and improving absorption efficiency. However, although it is desirable that the administered prodrugs are suitably metabolized and changed into an active compound in or in the vicinity of the target organ, there are so various metabolic enzymes in the living body and there are so many differences among individuals that stable action cannot be developed in many cases. Attempts to convert the glycoside bond of the compound to a carbon-carbon bond have been also made (refer to Patent Documents 4 to 21), but further improvement is demanded in the characteristics as pharmaceutical agents including activity and metabolic stability.

[Patent Document 1]
JP 2000-080041 A
[Patent Document 2]
International Publication WO01/06860
[Patent Document 3]
International Publication WO04/007517
[Patent Document 4]
US Patent Application Pub. No. 2001/041,674
[Patent Document 5]
US Patent Application Pub. No. 2002/137,903
[Patent Document 6]
International Publication WO01/027,128
[Patent Document 7]
International Publication WO02/083066
[Patent Document 8]
International Publication WO04/013118
[Patent Document 9]
International Publication WO03/099836

[Patent Document 10]
International Publication WO04/080990
[Patent Document 11]
US Patent Application Pub. No. 2005/0,209,166
[Patent Document 12]
International Publication WO05/085237
[Patent Document 13]
International Publication WO05/085265
[Patent Document 14]
International Publication WO05/012318
[Patent Document 15]
International Publication WO05/012326
[Patent Document 16]
US Patent Application Pub. No. 2006/0,063,722
[Patent Document 17]
US Patent Application Pub. No. 2006/0,035,841
[Patent Document 18]
US Patent Application Pub. No. 2006/0,074,031
[Patent Document 19]
International Publication WO06/002912
[Patent Document 20]
International Publication WO06/008038
[Patent Document 21]
International Publication WO06/010557
[Non-Patent Document 1]
J. Clin. Invest., Vol. 93, page 397, 1994
[Non-Patent Document 2]
Stroke, Vol. 14, page 388, 1983

[DISCLOSURE OF THE INVENTION]

[PROBLEMS TO BE SOLVED BY THE INVENTION]

An object of the present invention is to provide a spiroketal derivative having preferable characteristics as pharmaceutical agents. Particularly, an object of the present invention is to provide a spiroketal derivative having preferable characteristics as pharmaceutical agents such as high SGLT2 selectivity and strong and sustained hypoglycemic action as well as little concern about safety. Another object of the present invention is to provide a pharmaceutical composition used for prevention or treatment of diabetes such as insulin-dependent diabetes (Type 1 diabetes) and non-insulin-dependent diabetes (Type 2 diabetes), diabetic complications and diseases caused by hyperglycemia such as obesity.

[MEANS FOR SOLVING THE PROBLEMS]

We have filed a patent application for spiroketal derivatives represented by Formula (I):
[Formula 1]

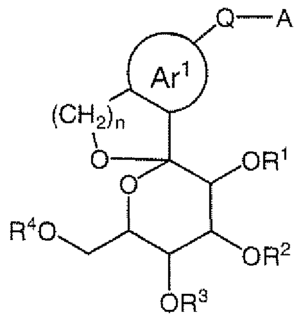

(I)

(specification of PCT/JP2006/301284, WO2006/080421). The present inventors have conducted intensive studies about these spiroketal derivatives so as to achieve the above-mentioned objects and consequently have found that spiroketal derivatives represented by Formula (II) in particularly have excellent characteristics preferable as pharmaceutical agents and thus completed the present invention.

More specifically, they have found that spiroketal derivatives represented by Formula (II) have high SGLT2 selectivity and strong and sustained hypoglycemic action as well as preferable features in terms of safety.

According to one aspect of the present invention, compounds described in the following (1) to (9) are provided.

(1) A compound represented by Formula (II):

[Formula 2]

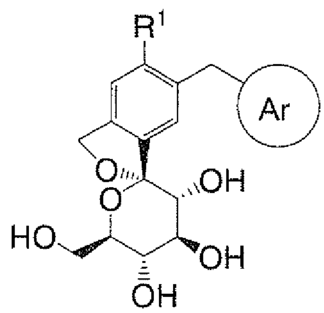

(II)

wherein $R^1$ is a chlorine atom, a fluorine atom, a methyl group or an ethynyl group;
Ar is a group represented by the following Formula (a), Formula (b), Formula (c) or Formula (d):

[Formula 3]

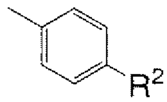
(a)

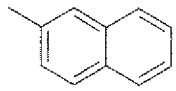
(b)

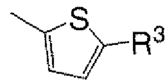
(c)

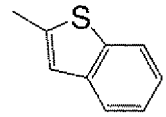
(d)

wherein $R^2$ is a $C_{1-6}$ alkyl group which may be substituted with one or more halogen atoms, a $C_{1-6}$ alkoxy group which may be substituted with one or more halogen atoms, a $C_{1-3}$ alkylthio group, a halogen atom, a $C_{1-3}$ alkylcarbonyl group or a $C_{2-5}$ alkynyl group which may be substituted with $-OR^4$;
$R^3$ is a hydrogen atom or a $C_{1-3}$ alkyl group;
$R^4$ is a hydrogen atom or a $C_{1-3}$ alkyl group;
provided that Ar is a group represented by Formula (a) when $R^1$ is a fluorine atom, methyl group or an ethynyl group, and that $R^2$ is a methoxy group, an ethoxy group, an isopropyl group, a propyl group, a trifluoromethyl group, a trifluoromethoxy group, a 2-fluoroethyl group or a 1-propynyl group when $R^1$ is a methyl group or a pharmaceutically acceptable salt or a solvate thereof.

(2) A compound represented by Formula (III):

[Formula 4]

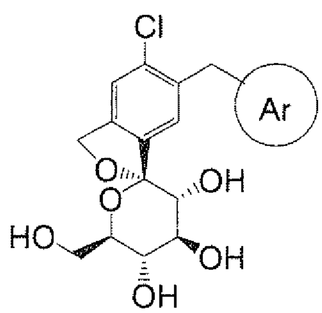
(III)

wherein Ar is a group represented by the following Formula (a), Formula (b), Formula (c) or Formula (d):
[Formula 5]

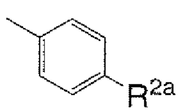
(a)

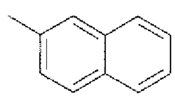
(b)

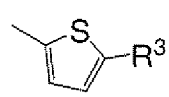
(c)

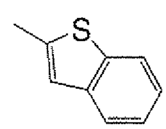
(d)

wherein $R^{2a}$ is a methyl group, an ethyl group, a propyl group, an isopropyl group, a tert-butyl group, a methoxy group, an ethoxy group, a trifluoromethoxy group, a trifluoromethyl group, a 2-fluoroethyl group, a 2,2-di fluoroethyl group, a methylthio group, a chloro group, an acetyl group or an ethynyl group,

$R^3$ is a hydrogen atom or an ethyl group or a pharmaceutically acceptable salt or a solvate thereof.

(3) A compound represented by Formula (IV):

[Formula 6]

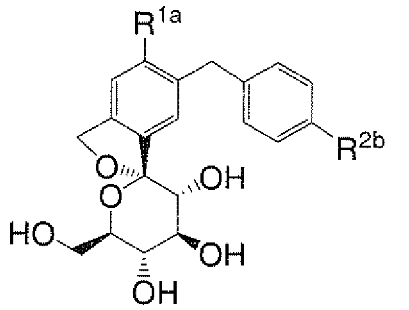

(IV)

wherein $R^{1a}$ is a fluorine atom or an ethynyl group; $R^{2b}$ is a methyl group, an ethyl group, an isopropyl group, a methoxy group or an ethoxy group or a pharmaceutically acceptable salt or a solvate thereof.

(4) A compound represented by Formula (V):

[Formula 7]

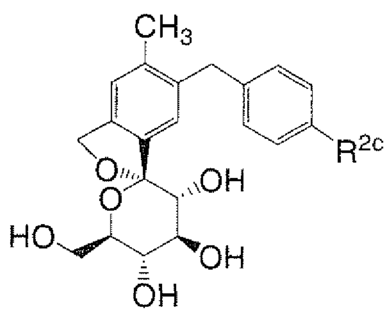

(V)

wherein $R^{2c}$ is a methoxy group, an ethoxy group, a propyl group, an isopropyl group, a trifluoromethyl group, a trifluoromethoxy group, a 2-fluoroethyl group or a 1-propynyl group or a pharmaceutically acceptable salt or a solvate thereof.

(5) A compound represented by Formula (VI):

[Formula 8]

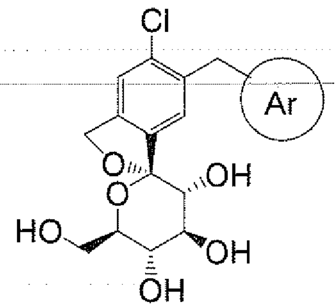

(VI)

wherein Ar is a group represented by the following Formula (a) or Formula (b):

[Formula 9]

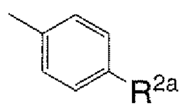

(a)

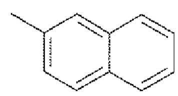

(b)

wherein $R^{2a}$ is an ethyl group, a propyl group, an isopropyl group, an ethoxy group or a 2-fluoroethyl group or a pharmaceutically acceptable salt or a solvate thereof.

(6) A compound represented by Formula (VII):

[Formula 10]

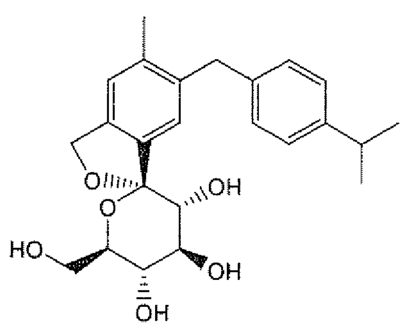

or a pharmaceutically acceptable salt or a solvate thereof.

(7) A compound selected from

(1S,3'R,4'S,5'S,6'R)-5-chloro-6-[(4-ethylphenyl)methyl]-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-[(4-methylphenyl)methyl]-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-1(4-(methylthio)phenyl)methyl]-spiro[isobenzofuran-1(3H),2'-[2H]pyran]-3',4',5'-triol;

(1S,3'R,4'S,5'S,6'R)-5-chloro-3',4',5',6'-tetrahydro-6'-(hydroxymethyl)-6-[(4-methoxyphenyl)methyl]-spiro[isobenzofuran-1(3H),2'-[2H]pyran]-3',4',5'-triol;
(1S,3'R,4'S,5'S,6'R)-5-chloro-6-[(4-ethoxyphenyl)methyl]-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-6-[(4-chlorophenyl)methyl]-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-(2-naphthylmethyl)-spiro[isobenzofuran-1(3H),2'-[2H]pyran]-3',4',5'-triol;
(1S,3'R,4'S,5'S,6'R)-5-chloro-6-[(4-(2-fluoroethyl)phenyl)methyl]-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-[(4-propylphenyl)methyl]-spiro[isobenzofuran-1(3H),2'-[2H]pyran]-3',4',5'-triol;
(1S,3'R,4'S,5-S,6'R)-5-chloro-6-[(4-(2,2-difluoroethyl)phenyl)methyl]-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-6-[(4-trifluoromethoxyphenyl)methyl]-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-6-[(4-acetylphenyl)methyl]-5-chloro-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-6-[(4-trifluoromethylphenyl)methyl]-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-6-[(4-tert-butylphenyl)methyl]-5-chloro-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-[(4-isopropylphenyl)methyl]-spiro[isobenzofuran-1(3H),2'-[2H]pyran]-3',4',5'-triol;
(1S,3'R,4'S,5'S,6'R)-6-[(4-ethylphenyl)methyl]-5-fluoro-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-6-[(4-ethoxyphenyl)methyl]-5-fluoro-3',4',5',6'-tetrahydro-6'-(hydroxymethyl)-spiro[isobenzofuran-1(3H),2'-[2H]pyran]-3',4',5'-triol; (1S,3'R,4'S,5'S,6'R)-5-fluoro-3',4',5',6'-tetrahydro-6'-(hydroxymethyl)-6-[(4-methylphenyl)methyl]-spiro[isobenzofuran-1(3H),2'-[2H]pyran]-3',4',5'-triol;
(1S,3'R,4'S,5'S,6'R)-5-fluoro-3',4',5',6'-tetrahydro-6'-(hydroxymethyl)-6-[(4-isopropylphenyl)methyl]-spiro[isobenzofuran-1(3H),2'-[2H]pyran]-3',4',5'-triol;
(1S,3'R,4'S,5'S,6'R)-5-fluoro-3',4',5',6'-tetrahydro-6'-(hydroxymethyl)-6-[(4-methoxyphenyl)methyl]-spiro[isobenzofuran-1(3H),2'-[2H]pyran]-3',4',5'-triol;
(1S,3'R,4'S,5'S,6'R)-3',4',5',6'-tetrahydro-6'-(hydroxymethyl)-6-[(4-isopropylphenyl)methyl]-5-methyl-spiro[isobenzofuran-1(3H),2'-[2H]pyran]-3',4',5'-triol;
(1S,3'R,4'S,5'S,6'R)-6-[(4-(2-fluoroethyl)phenyl)methyl]-5-methyl-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-3',4',5',6'-tetrahydro-6'-(hydroxymethyl)-6-[(4-methoxyphenyl)methyl]-5-methyl-spiro[isobenzofuran-1(3H),2'-[2H]pyran]-3',4',5'-triol;
(1S,3'R,4'S,5'S,6'R)-6-[(4-ethoxyphenyl)methyl]-3',4',5',6'-tetrahydro-6'-(hydroxymethyl)-5-methyl-spiro[isobenzofuran-1(3H),2'-[2H]pyran]-3',4',5'-triol;
(1S,3'R,4'S,5'S,6'R)-3',4',5',6'-tetrahydro-6'-(hydroxymethyl)-5-methyl-6-[(4-propylphenyl)methyl]-spiro[isobenzofuran-1(3H),2'-[2H]pyran]-3',4',5'-triol;
(1S,3'R,4'S,5'S,6'R)-6-[(4-trifluoromethylphenyl)methyl]-3',4',5',6'-tetrahydro-6'-(hydroxymethyl)-5-methyl-spiro[isobenzofuran-1(3H),2'-[2H]pyran]-3',4',5'-triol;
(1S,3'R,4'S,5'S,6'R)-6-[(4-trifluoromethoxyphenyl)methyl]-3',4',5',6'-tetrahydro-6'-(hydroxymethyl)-5-methyl-spiro[isobenzofuran-1(3H),2'-[2H]pyran]-3',4',5'-triol;
(1S,3'R,4'S,5'S,6'R)-6-[(benzothiophen-2-yl)methyl]-5-chloro-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-6-[(5-ethylthiophen-2-yl)methyl]-3',4',5',6'-tetrahydro-6'-(hydroxymethyl)-spiro[isobenzofuran-1(3H),2'-[2H]pyran]-3',4',5'-triol
(1S,3'R,4'S,5'S,6'R)-5-chloro-3',4',5',6'-tetrahydro-6'-(hydroxymethyl)-6-[(thiophen-2-yl)methyl]-spiro[isobenzofuran-1(3H),2'-[2H]pyran]-3',4',5'-triol;
(1S,3'R,4'S,5'S,6'R)-6-[(4-ethylphenyl)methyl]-5-ethynyl-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-6-[(4-ethynylphenyl)methyl]-3',4',5',6'-tetrahydro-6'-(hydroxymethyl)-spiro[isobenzofuran-1(3H),2'-[2H]pyran]-3',4',5'-triol; and
(1S,3'R,4'S,5'S,6'R)-3',4',5',6'-tetrahydro-6'-(hydroxymethyl)-5-methyl-6-[(4-(propyn-1-yl)phenyl)methyl]-spiro[isobenzofuran-1(3H),2'-[2H]pyran]-3',4',5'-triol;
or a pharmaceutically acceptable salt or a solvate thereof.

(8) A compound selected from

(1S,3'R,4'S,5'S,6'R)-5-chloro-6-[(4-ethylphenyl)methyl]-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-[(4-methylphenyl)methyl]-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-[(4-(methylthio)phenyl)methyl]-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-[(4-methoxyphenyl)methyl]-spiro[isobenzofuran-1(3H),2'-[2H]pyran]-3',4',5'-triol;
(1S,3'R,4'S,5'S,6'R)-5-chloro-6-[(4-ethoxyphenyl)methyl]-3',4',5',6'-tetrahydro-6'-(hydroxymethyl)-spiro[isobenzofuran-1(3H),2'-[2H]pyran]-3',4',5'-trlol;
(1S,3'R,4'S,5'S,6'R)-5-chloro-3',4',5',6'-tetrahydro-6'-(hydroxymethyl)-6-(2-naphthylmethyl)-spiro[isobenzofuran-1(3H),2'-[2H]pyran]-3',4',5'-triol;
(1S,3'R,4'S,5'S,6'R)-5-chloro-6-[(4-(2-fluoroethyl)phenyl)methyl]-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-[(4-propylphenyl)methyl]-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-[(4-isopropylphenyl)methyl]-spiro[isobenzofuran-1(3H),2'-[2H]pyran]-3',4',5'-triol;
(1S,3'R,4'S,5'S,6'R)-3',4',5',6'-tetrahydro-6'-(hydroxymethyl)-6-[(4-isopropylphenyl)methyl]-5-methyl-spiro[isobenzofuran-1(3H),2'-[2H]pyran]-3',4',5'-triol;
(1S,3'R,4'S,5'S,6'R)-6-[(benzothiophen-2-yl)methyl]-5-chloro-3',4',5',6'-tetrahydro-6'-(hydroxymethyl)-spiro[isobenzofuran-1(3H),2'-[2H]pyran]-3',4',5'-triol; and
(1S,3'R,4'S,5'S,6'R)-5-chloro-6-[(5-ethylthiophen-2-yl)methyl]-3',4',5',6'-tetrahydro-6'-(hydroxymethyl)-spiro[isobenzofuran-1(3H),2'-[2H]pyran]-3',4',5'-triol;
or a pharmaceutically acceptable salt or a solvate thereof.

(9) A compound selected from

(1S,3'R,4'S,5'S,6'R)-5-chloro-6-[(4-ethylphenyl)methyl]-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-(2-naphthylmethyl)-spiro[isobenzofuran-1(3H),2'-[2H]pyran]-3',4',5'-triol;
(1S,3'R,4'S,5'S,6'R)-5-chloro-6-[(4-(2-fluoroethyl)phenyl)methyl]-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-[(4-propylphenyl)methyl]-spiro[isobenzofuran-1(3H),2'-[2H]pyran]-3',4',5'-triol;
(1S,3-R,4'S,5'S,6'R)-5-chloro-6-[(4-ethoxyphenyl)methyl]-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-[(4-isopropylphenyl)methyl]-spiro[isobenzofuran-1(3H),2'-[2H]pyran]-3',4',5'-triol; and
(1S,3'R,4'S,5'S,6'R)-3',4',5',6'-tetrahydro-6'-(hydroxymethyl)-6-[(4-isopropylphenyl)methyl]-5-methyl-spiro[isobenzofuran-1(3H),2'-[2H]pyran]-3',4',5'-triol;
or a pharmaceutically acceptable salt or a solvate thereof.

According to another aspect of the present invention, pharmaceutical compositions containing a compound described in the above (1) to (9) or a pharmaceutically acceptable salt or a solvate thereof which is used as a $Na^+$-glucose cotransporter inhibitor are provided.

According to still another aspect of the present invention, pharmaceutical compositions containing a compound described in the above (1) to (9) or a pharmaceutically acceptable salt or a solvate thereof which is used for prevention or treatment of diabetes, hyperglycemia, diabetic complications and obesity are provided. In one embodiment of this aspect, diabetes is insulin-dependent diabetes (Type 1 diabetes) or non-insulin-dependent diabetes (Type 2 diabetes).

According to further another aspect of the present invention, a method for preventing or treating diabetes (for example, insulin-dependent diabetes (Type 1 diabetes) or non-insulin-dependent diabetes (Type 2 diabetes)), hyperglycemia, diabetic complications or obesity comprising an effective amount of a compound described in the above (1) to

(9) or a pharmaceutically acceptable salt or a solvate thereof is provided.

As for $R^1$, $R^2$ and $R^3$ defined for the compounds of the present invention:

$R^1$ is a chlorine atom, a fluorine atom, a methyl group, an ethynyl group, and a chlorine atom and a methyl group are particularly preferable.

As a $C_{1-6}$ alkyl group of the $C_{1-6}$ alkyl group which may be substituted with one or more halogen atoms in $R^2$, a methyl group, an ethyl group, a propyl group, an isopropyl group or a tert-butyl is preferable.

As a halogen atom of the $C_{1-6}$ alkyl group which may be substituted with one or more halogen atoms in $R^2$, a fluorine atom is preferable.

As a $C_{1-6}$ alkyl group which may be substituted with one or more halogen atoms in $R^2$, a methyl group, an ethyl group, a propyl group, an isopropyl group, a tert-butyl group, a 2-fluoroethyl group, a trifluoromethyl group or a 2,2-difluoroethyl group is preferable.

As a $C_{1-6}$ alkoxy group of the $C_{1-6}$ alkoxy group which may be substituted with one or more halogen atoms in $R^2$, a methoxy group or an ethoxy group is preferable.

As a halogen atom of the $C_{1-6}$ alkoxy group which may be substituted with one or more halogen atoms in $R^2$, a fluorine atom is preferable.

As a $C_{1-6}$ alkoxy group which may be substituted with one or more halogen atoms in $R^2$, a methoxy group, an ethoxy group or a trifluoromethoxy group is preferable.

As a $C_{1-3}$ alkylthio group in $R^2$, a methylthio group is preferable.

As a halogen atom in $R^2$, a chlorine atom is preferable.

As a $C_{1-3}$ alkylcarbonyl group in $R^2$, an acetyl group is preferable.

As a $C_{2-5}$ alkynyl group of the $C_{2-5}$ alkynyl group which may be substituted with $-OR^4$ in $R^2$, an ethynyl group or a 1-propynyl group is preferable.

As $-OR^4$ of the $C_{2-5}$ alkynyl group which may be substituted with $-OR^4$ in $R^2$, a hydroxyl group or a methoxy group is preferable.

As a $C_{2-5}$ alkynyl group which may be substituted with $-OR^4$ in $R^2$, an ethynyl group or a 1-propynyl group is preferable.

As $R^2$, a methyl group, an ethyl group, a propyl group, an isopropyl group, a tert-butyl group, a 2-fluoroethyl group, a trifluoromethyl group, a 2,2-difluoroethyl group, a methoxy group, an ethoxy group, a trifluoromethoxy group, a methylthio group, a chlorine atom, an acetyl group, an ethynyl group or a 1-propynyl group is preferable.

As a $C_{1-3}$ alkyl group in $R^3$, an ethyl group is preferable.

As $R^3$, an ethyl group is preferable.

The "$C_{1-6}$ alkyl group" in the present specification means a linear or branched alkyl group having 1 to 6 carbon atoms, and includes, for example, a methyl group, an ethyl group, a n-propyl group, an 1-propyl group, a n-butyl group, a s-butyl group, an i-butyl group, a t-butyl group, a n-pentyl group, a 3-methylbutyl group, a 2-methylbutyl group, a 1-methylbutyl group, a 1-ethylpropyl group, a n-hexyl group, a 4-methylpentyl group, a 3-methylpentyl group, a 2-methylpentyl group, a 1-methylpentyl group, a 3-ethylbutyl group and a 2-ethylbutyl group. Examples of preferable $C_{1-6}$ alkyl group include a linear or branched alkyl group having 1 to 3 carbon atoms, and a methyl group and an ethyl group are particularly preferable. The "$C_{1-3}$ alkyl group" is a linear or branched alkyl group having 1 to 3 carbon atoms and specifically means a methyl group, an ethyl group, an n-propyl group and an i-propyl group.

The "$C_{1-6}$ alkyl group which may be substituted with one or more halogen atoms" in the present specification means a group in which any of the hydrogen atoms in the above "$C_{1-6}$ alkyl group" may be substituted with one or more halogen atoms, and includes, for example, a fluoromethyl group, a difluoromethyl group, a trifluoromethyl group, a 1-fluoroethyl group, a 2-fluoroethyl group, a 2,2,2-trifluoroethyl group, a pentafluoroethyl group, a heptafluoropropyl group, a chloromethyl group, a dichloromethyl group, a trichloromethyl group, a 1-chloroethyl group, a 2-chloroethyl group, a 2,2,2-trichloroethyl group, a pentachloroethyl group, a heptachloropropyl group, a bromomethyl group, a dibromomethyl group, a tribromomethyl group, an iodomethyl group, a diiodomethyl group, a triiodomethyl group, a bromochloromethyl group, a chloroiodomethyl group, a 3-chloropropyl group, a 3-bromopropyl group, a 3-iodopropyl group, a 2-chloro-1-methylethyl group, a 2-bromopropyl group, a 1-chloro-2,2,2-trifluoroethyl group, a 1-bromo-2,2,2-trifluoroethyl group, and preferred examples are a trifluoromethyl group and a 2-fluoroethyl group.

The "$C_{1-6}$ alkoxy group" in the present specification means an alkyloxy group having a linear or branched alkyl group having 1 to 6 carbon atoms as an alkyl moiety, and includes, for example, , a methoxy group, an ethoxy group, a n-propoxy group, an i-propoxy group, a n-butoxy group, a s-butoxy group, an i-butoxy group, a t-butoxy group, a n-pentoxy group, a 3-methylbutoxy group, a 2-methylbutoxy group, a 1-methylbutoxy group, a 1-ethylpropoxy group, a n-hexyloxy group, a 4-methylpentoxy group, a 3-methylpentoxy group, a 2-methylpentoxy group, a 1-methylpentoxy group, a 3-ethylbutoxy group.

The "$C_{1-6}$ alkoxy group which may be substituted with one or more halogen atoms" in the present specification

means a group in which any of the hydrogen atoms in the above "$C_{1-6}$ alkoxy group" may be substituted with one or more halogen atoms, and includes, for example, a fluoromethoxy group, a difluoromethoxy group, a trifluoromethoxy group, a 1-fluoroethoxy group, a 2-fluoroethoxy group, a 2,2,2-trifluoroethoxy group, a pentafluoroethoxy group, a heptafluoropropoxy group, a chloromethoxy group, a dichloromethoxy group, a trichloromethoxy group, a 1-chloroethoxy group, a 2-chloroethoxy group, a 2,2,2-trichloroethoxy group, a pentachloroethoxy group, a heptachloropropoxy group, a bromomethoxy group, a dibromomethoxy group, a tribromomethoxy group, an iodomethoxy group, a diiodomethoxy group, a triiodomethoxy group, a bromochloromethoxy group, a chloroiodomethoxy group, a 3-chloropropoxy group, a 3-bromopropoxy group, a 3-iodopropoxy group, a 2-chloro-1-methylethoxy group, a 2-bromopropoxy group, a 1-chloro-2,2,2-trifluoroethoxy group, a 1-bromo-2,2,2-trifluoroethoxy group, and preferred examples are a trifluoromethoxy group and a 2,2,2-trifluoroethoxy group.

The "$C_{1-3}$ alkylthio group" in the present specification means an alkylthio group having a linear or branched alkyl group having 1 to 3 carbon atoms as an alkyl moiety, and specidically includes a methylthio group, an ethylthio group, a n-propylthio group and an i-propylthio group, and a preferred example is a methylthio group.

The "halogen atom" in the present specification includes, for example, a fluorine atom, a chlorine atom, a bromine atom and an iodine atom.

The "$C_{1-3}$ alkylcarbonyl group" in the present specification means an alkylcarbonyl group having a linear or branched alkyl group having 1 to 3 carbon atoms as an alkyl moiety, and specifically includes an acetyl group, a propionyl group, a butyryl group and an isobutyryl group, and a preferred example is an acetyl group.

The "$C_{2-5}$ alkynyl group" in the present specification includes an ethynyl group, a 1-propynyl group, a 1-butynyl group, a 1-butyne-3-methyl group, 1-pentynyl group, a 2-propynyl group, a 2-butynyl group, a 2-butyne-1-methyl group, a 2-pentynyl group, a 3-butyne-1-methyl group, a 3-butyne-2-methyl group, a 3-pentynyl group, a 4-pentynyl group.

Various stereoisomers of the compound defined as the compounds of the present invention such as tautomers and optical isomers, and mixtures and isolated products thereof are included in the range of the present invention.

The compounds of the present invention may form acid addition salts. The compounds may form salts with a base depending on the kind of the substituent. Such salts specifically include acid addition salts with mineral acids such as hydrochloric acid, hydrobromic acid, hydroiodic acid, sulfuric acid, nitric acid, phosphoric acid; organic acids such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid; and acidic amino acids such as aspartic acid and glutamic acid. The salts formed with bases include salts with inorganic bases such as sodium, potassium, magnesium, calcium and aluminum; salts with organic bases such as methylamine, ethylamine, ethanolamine; salts with basic amino acids such as lysin, ornithine and ammonium salts.

Furthermore, hydrates, pharmaceutically acceptable various solvates and crystal polymorphs are included in the compounds of the present invention.

The compounds of the present invention have strong and sustained hypoglycemic effect and little concern on the safety and other characteristics preferable as pharmaceutical agents. The "strong hypoglycemic action (or effect)" mentioned in the present specification includes, for example, the hypoglycemic action (or effect) achieving 25% or more reduction of blood glucose level at 6 hours after 0.3 mg/kg oral administration of the compound in a study for glucose lowering effect in db/db mice. In addition, "sustained hypoglycemic action (or effect)" mentioned in the present specification includes, for example, the hypoglycemic action (or effect) achieving 25% or more reduction of blood glucose level at 24 hours after administration of the compound in a study for glucose lowering effect in db/db mice. Furthermore, when a compound is mentioned as causing "little concern on the safety", it means, for example, that the compound causes little concern of side effects which can be obstacles in the drugs development such as genotoxic potential and inhibitory action on metabolizing enzymes.

The present invention also includes so-called prodrugs which are compounds metabolized in the living body and converted into the compounds of the above Formula (II) and pharmaceutically acceptable salts thereof. Groups to form prodrugs of the compounds of the present invention include groups described in Prog. Med. Vol. 5, pages 2157-2161 (1985) and groups described in "Iyakuhin no Kaihatsu" ("Development of medicinal drugs"), Vol. 7 (molecular design), pages 163-198, Hirokawa Shoten published in 1990.

The compounds of the present invention can be produced by applying various kinds of a publicly known synthesis method in accordance with characteristics based on the basic structure or the kind of the substituents. Depending on the kind of functional groups, it may be preferable in terms of production technology to protect a functional group with a suitable protecting group at the stage of raw materials or intermediates, and desired compounds can be obtained by removing the protecting group in the later steps. Examples of the functional groups needed to be protected in the production process include a hydroxyl group and a carboxy group and examples of the protecting groups thereof include the protecting groups described in Greene and Wuts, "Protective Groups in Organic Synthesis", second edition. The protecting group to be used and reaction conditions at the time of introducing and removing the protecting group can be appropriately selected based on the conventional technology such as those described in the above-mentioned documents.

The compounds of the present invention have inhibitory activity on sodium dependent glucose cotransporter 2 (SGLT2) involved in glucose reabsorption in the kidney (J. Clin. Invest., Vol. 93, page 397, 1994). Inhibition of SGLT2 suppresses reabsorption of glucose, excretes excessive glucose to outside of the body and thereby leads to therapeutic effect on the diabetes and an effect of improving insulin resistance by correcting hyperglycemia without a burden to pancreatic β cells.

Therefore, according to one aspect of the present invention, pharmaceutical agents to prevent or treat diseases or conditions which can be improved by inhibiting the activity of SGLT2, for example, diabetes, diabetes-related diseases and diabetic complications are provided.

Here, the "diabetes" includes Type 1 diabetes, Type 2 diabetes, and the other types of diabetes by specific causes. The "diabetes-related diseases" includes, for example, obesity, hyperinsulinemia, abnormality of glucose metabolism, hyperlipidemia, hypercholesterolemia, hypertriglyceridemia, lipidosis, hypertension, congestive heart failure, edema, hyperuricemia and gout.

The "diabetic complications" include both acute and chronic complications. Examples of "acute complications" include hyperglycemia (ketoacidosis, etc.), infectious diseases (infection in the skin, soft tissue, biliary system, respiratory system, urinary tract, etc.) and examples of "chronic complication" include microangiopathy (nephropathy, retinopathy), arteriosclerosis (atherosclerosis, myocardial infarction, cerebral infarction, lower limbs arterial occlusion, etc.), neuropathy (in sensory nerve, motor nerves, autonomous nerve, etc.), foot gangrene. Major diabetic complications include diabetic retinopathy, diabetic nephropathy and diabetic neuropathy.

The compounds of the present invention can be used together with therapeutic drugs for diabetes and diabetic complications, which have different action mechanism other than SGLT2 activity inhibitor, antihyperlipemic drugs, or antihypertensive drug, etc. Additive effect can be expected by combining the compounds of the present invention with the other drugs as compared with the effect obtained by singly using the respective drugs for the above-mentioned diseases.

Examples of the "therapeutic drug for diabetes and diabetic complications" which can be used together include insulin sensitivity enhancing drugs (PPARγ agonist, PPARα/γ agonist, PPARδ agonist, PRARα/γ/δ agonist), glycosidase inhibitors, biguanide drugs, insulin secretion enhancers, insulin formulations, glucagon receptor antagonists, insulin receptor kinase enhancers, tripeptidyl peptidase II inhibitors, dipeptidyl peptidase IV inhibitors, protein tyrosine phosphatase-1B inhibitors, glycogen phosphorylase inhibitors, glucose-6-phosphatase inhibitors, gluconeogenesis inhibitors, fructose bisphosphatase inhibitors, pyruvic acid dehydrogenase inhibitors, glucokinase activators, D-chiro-inositol, glycogen synthetase kinase-3 inhibitors, glucagons-like peptide-1, glucagons-like peptide-1 analogues, glucagons-like peptide-1 agonists, amylin, amylin analogues, amylin agonists, glucocorticoid receptor antagonists, 11β-hydroxysteroid dehydrogenase inhibitors, aldose reductase inhibitors, protein kinase C inhibitors, γ-aminobutyric acid receptor antagonists, sodium channel antagonists, transcription factor NF-κB inhibitors, IKKβ inhibitors, lipid peroxidase inhibitors, N-acetylated-α-linked-acid-dipeptidase inhibitors, insulin-like growth factor-I, platelet-derived growth factors (PDGF), platelet-derived growth factor (PDGF) analogues, epidermal growth factors (EGF), nerve growth factors, carnitine derivatives, uridine, 5-hydroxy-1-methyl hydantoin, EGB-761, bimoclomol, sulodexide, Y-128 and TAR-428.

The "therapeutic drug for diabetes and diabetic complications" can be exemplified as follows.

Metoformine hydrochloride and fenformine are included as "biguanide drugs".

Among insulin secretion enhancers, examples of sulphonylurea drugs include glyburide (glibenclamide), glipizide, gliclazide, chlorpropamide and examples of non-sulphonylurea drugs include nateglinide, repaglinide and mitiglinide.

The "insulin formulations" include genetic recombinant human insulin and animal origin insulin. They are classified into three types by duration of action, and include immediate-acting type (human insulin, human neutral insulin), intermediate-acting type (insulin-human isofen insulin aqueous suspension, human neutral insulin-human isofen insulin aqueous suspension, human insulin zinc aqueous suspension, insulin zinc aqueous suspension) sustained-acting type (huma crystalline insulin zinc suspension).

The "glycosidase inhibitors" include acarbose, voglibose and miglitol.

Among "insulin sensitivity enhancing drugs", PPARγ agonists include troglitazone, pioglitazone, rosiglitazone, PPARα/γ dual agonists include MK-767 (KRP-297), tesaglitazar, LM4156, LY510929, DRF-4823, TY-51501, and PPARδ agonists include GW-501516.

The "tripeptidyl peptidase II inhibitors" include UCL-139.

The "dipeptidyl peptidase IV inhibitors" include NVP-DPP728A, LAF-237, MK-0431, P32/98 and TSL-225.

The "aldose reductase inhibitors" include ascorbyl gamolenate, tolrestat, epalrestat, fidarestat, sorbynyl, ponalrestat, risarestat and zenarestat.

The "γ-aminobutyric acid receptor antagonists" include topiramate.

The "sodium channel antagonists" include mexiletine hydrochloride.

The "transcription factor NF-κB inhibitors" include dexlipotam.

The "lipid peroxidase inhibitors" include tirilazad mesylate.

The "N-acetylated-α-linked-acid-dipeptidase inhibitors" include GPI-5693.

The "carnitine derivatives" include carnitine, levacecarnine hydrochloride.

The "antihyperlipemica drugs and antihypertensive drugs" which can be used together include, for example, hydroxymethylglutaryl coenzyme A reductase inhibitors, fibrate compounds, $\beta_3$-adrenaline receptor agonists, AMPK activators, acyl coenzyme A:cholesterol transacylase inhibitors, probcol, thyroid hormone receptor agonists, cholesterol absorption inhibitors, lipase inhibitors, microsome triglyceride transfer protein inhibitors, lipoxygenase inhibitors, carnitine palmitoyltransferase inhibitors, squalene synthetase inhibitors, low-density lipoprotein receptor enhancers, nicotine acid derivatives, bile acid adsorbing drugs, sodium conjugate bile acid transporter inhibitors, cholesterol ester transportation protein inhibitors, angiotensin converting enzyme inhibitors, angiotensin II receptor antagonists, endothelin converting enzyme inhibitors, endothelin receptor antagonists, diuretic drugs, calcium antagonists, vasodilatory hypotensive agents, sympatholytic drugs, central hypotensive agents, $\alpha_2$-adrenaline receptor agonists, antiplatelets, uric acid generation inhibitors, uric acid excretion enhancers, urine alkalizer, anorectic drugs, ACE inhibitors, adiponectin receptor agonists, GPR40 agonists, GPR40 antagonists.

The therapeutic drugs for hyperlipemia and antihypertensive drugs can be exemplified as follows.

The "hydroxymethylglutaryl coenzyme A reductase inhibitors" include fluvastatin, lovastatin, pravastatin, cerivastatin and pitavastatin.

The "fibrate compounds" include bezafibrate, beclobrate and binifibrate.

The "squalene synthetase inhibitors" include TAK-475, α-phosphonosulphonate derivatives (specification of US Patent No. 5712396).

The "acyl coenzyme A: cholesterol transacylase inhibitors" include CI-1011, NTE-122, FCE-27677, RP-73163, MCC-147 and DPU-129.

The "low-density lipoprotein receptor enhancers" include MD-700 and LY-295427.

The "microsome triglyceride transfer protein inhibitors" (MTP inhibitors) include compounds described in the specifications of US Patent No. 5739135, US Patent No. 5712279 and US Patent No. 5760246.

The "anorectic drugs" include adrenaline-noradrenalin agonists (mazindol, ephedrine, etc.), serotonin agonists (selective serotonin reuptake inhibitors, for example, fluvoxamine, etc.), adrenaline-serotonin agonists (sibutramine, etc.), melanocortin-4 receptor (MC4R) agonists, α-melanocyte stimulating hormones (α-MCH), leptin, cocaine and amphetamine-regulated transcript (CART).

The "thyroid hormone receptor agonists" include liothyronine sodium, repothyroxine sodium.

The "cholesterol absorption inhibitors" include ezetimibe.

The "lipase inhibitors" include orlistat.

The "carnitine palmitoyltransferase inhibitors" include etomoxir.

The "nicotine acid derivatives" include nicotinic acid, nicotinic acid amides, nicomol, nicorandils.

The "bile acid adsorbing drugs" include cholestyramine, cholestyirane and colesevelam hydrochloride.

The "angiotensin converting enzyme inhibitors" include captotil, enalapril maleate, alacepril and cilazapril.

The "angiotensin II receptor antagonists" include candesartan cilexetil, losartan potassium and eprosartan mesylate.

The "endothelin converting enzyme inhibitors" include CGS-31447, CGS-35066.

The "endothelin receptor antagonists" include L-749805, TBC-3214 and BMS-182874.

For example, it is considered to be preferable that the compounds of the present invention are used in combination with at least one kind of drugs selected from the group consisting of insulin sensitivity enhancing drugs (PPARγ agonists, PPARα/γ agonists, PPARδ agonists, PPARα/γ/δ agonists, etc.), glycosidase inhibitors, biguanide drugs, insulin secretion enhancers, insulin formulations and dipeptidyl peptidase IV inhibitors in the treatment of diabetes and the like.

Alternatively, it is considered to be preferable that the compounds of the present invention are used in combination with at least one kind of drugs selected from the group consisting of hydroxymethylglutaryl coenzyme A reductase inhibitors, fibrate compounds, squalene synthetase inhibitors, acyl coenzyme A:cholesterol transacylase inhibitors, low-density lipoprotein receptor enhancers, microsome triglyceride transfer protein inhibitors and anorectic drugs.

The pharmaceutical agents of the present invention can be systemically or topically administered orally or parenterally, for example, intrarectally, subcutaneously, intramuscularly, intravenously and percutaneously.

For the purpose of using a compound of the present invention as a pharmaceutical agent, it can be in a form of a solid composition, a liquid composition or any other form of composition, and the most suitable form may be selected as required. The pharmaceutical agent of the present invention can be produced by blending a pharmaceutically acceptable carrier with a compound of the present invention. Specifically, commonly used excipients, expanders, binding agents, disintegrating agents, coating agents, sugar-coating agents, pH regulators, resolvents or aqueous or a non-aqueous solvents may be added to prepare tablets, pills, capsules, granules, powders, powdered drugs, liquid drugs, emulsion, suspension, injection agents by conventional drug preparing techniques. Examples of excipients and expanders include lactose, magnesium stearate, starch, talc, gelatin, agar, pectin, Arabian gum, olive oil, sesame oil, cocoa butter, ethylene glycol and those commonly used.

In addition, the compounds of the present invention can be prepared into a drug by forming a clathrate compound with α-, β- or γ-cyclodextrin or methylated cyclodextrin.

The dose of the compounds of the present invention varies depending on disease, conditions, weight, age, sex, administration route, etc. but 0.1 to 1000 mg/kg weight/day for an adult is preferable and 0.1-200 mg/kg weight/day is more preferable, which can be administered once a day or divided into several times a day.

The compound of the present invention can be synthesized, for example, by a production process shown below.

Compound (II) of the present invention can be synthesized by a process shown in Scheme 1:

Scheme 1

[Formula 11]

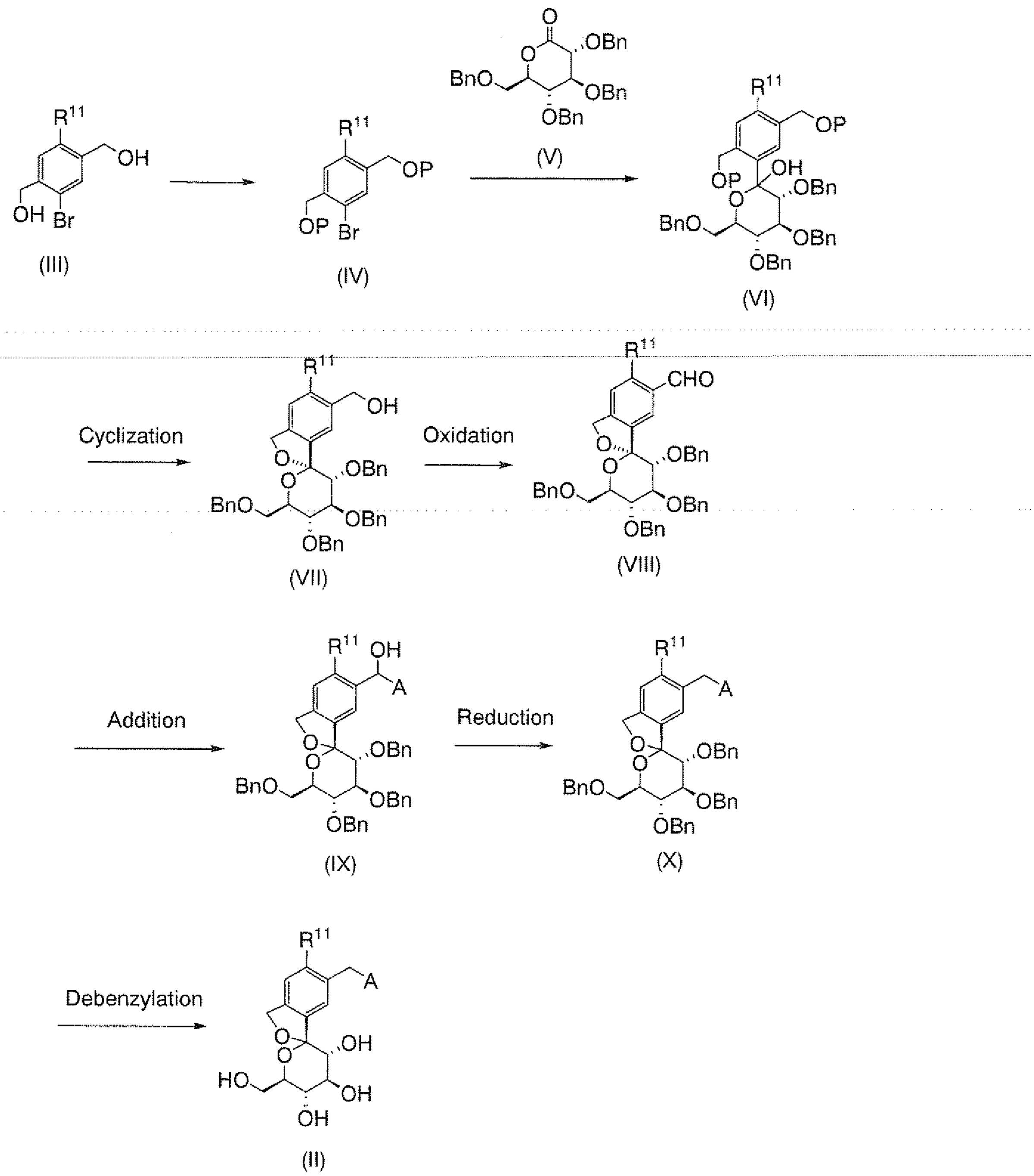


(III) (IV) (V) (VI) (VII) (VIII) (IX) (X) (II)

wherein $R^{11}$ means the same as defined above for $R^1$, P represents an appropriate protecting group, and A is a group represented by Formula (a), Formula (b), Formula (c) or Formula (d) defined above.

The reaction converting Compound (III) to Compound (IV) can be achieved by performing a reaction with a suitable protecting group introducing reagent in a suitable solvent. The suitable solvent includes THF, diethyl ether, N, N-dimethylformamide, dichloromethane, 1,2-dichloroethane, toluene and xylene. The suitable protecting group introducing reagent includes a reagent for introducing a protecting group, which can be removed in an acid conditions, such as trityl chloride, tert-butyldimethylsilyl chloride, methoxymethyl chloride, 3,4-dihydro-2H-pyran, 2-methoxypropene, and preferably 2-methoxypropene is used. It is necessary to carry out this reaction of introducing a protecting group in the presence of a suitable base or acid. Specifically, in the case of using 2-methoxypropene, it is preferable to allow a catalytic amount of p-toluenesulfonic acid to be present as an acid. The above reaction can be performed normally from about -20°C to about 50°C, preferably from about 0°C to about 25°C (room temperature) for about 10 minutes to about 5 hours, preferably for about 30 minutes to about 2 hours.

The reaction converting Compound (IV) to Compound (VI) can be achieved by performing a reaction with a

suitable alkyllithium reagent in a suitable solvent and then a reaction with Compound (V) ((3R,4S,5R,6R)-3,4,5-tris (benzyloxy)-6-(benzyloxymethyl)tetrahydropyran-2-one). The suitable solvent includes THF, diethyl ether, dimethoxyethane, diethoxyethane, dichloromethane and toluene and preferably THF and toluene. The suitable alkyllithium reagent includes n-butyllithium, sec-butylithium, tert-butylithium, methyllithium, and preferably n-butyllithium is used. The above reaction can be performed normally from about -78˚C to about 25˚C (room temperature) for about 10 minutes to about 2 hours, preferably for about 1 hour to about 2 hours. Compound (V) can be synthesized by a method described, for example, in a document (Carbohydr. Res., No. 260, page 243, 1994).

The reaction converting Compound (VI) to Compound (VII) can be achieved by performing a reaction with a suitable acid catalyst in a suitable solvent together with a deprotection step. The suitable solvent includes THF, dimethoxyethane, diethoxyethane, dichloromethane, toluene, methanol, ethanol, isopropanol, and preferably a mixture solvent of THF and methanol is used. The suitable acid catalyst includes p-toluenesulfonic acid, pyridinium-p-toluenesulfonic acid, methanesulfonic acid, trifluoromethanesulfonic acid, trifluoroacetic acid, camphorsulfonic acid, hydrochloric acid, sulfuric acid, acetic acid, and preferably p-toluenesulfonic acid is used. The above reaction can be performed normally from about -78˚C to about 100˚C, preferably from about 0˚C to about 60˚C for about 10 minutes to about 24 hours, preferably for about 2 hours to about 5 hours. In this step, isomerization of the spiro moiety occurs simultaneously with cyclization, and a compound with desired steric configuration can be obtained.

The reaction converting Compound (VII) to Compound (VIII) can be achieved by performing a reaction with a suitable oxidizing agent in a suitable solvent. The suitable solvent includes dichloromethane, 1,2-dichloroethane, toluene and xylene, and preferably dichloromethane is used. The suitable oxidizing agent includes Dess-Martin reagent, TPAP-NMO, DMSO-acetic anhydride, DMSO-oxalyl chloride, manganese dioxide, chromic acid-sulfuric acid, $SO_3$-pyridine, and preferably manganese dioxide is used. The above reaction can be performed normally from about -78˚C to about 40˚C, preferably from about 0˚C to about 25˚C (room temperature) for about 10 minutes to about 24 hours, preferably for about 1 hour.

The reaction converting Compound (VIII) to Compound (IX) can be achieved by performing a reaction with a suitable aryl metal reagent in a suitable solvent. The suitable solvent includes THF, diethyl ether, dimethoxyethane, diethoxyethane, dichloromethane and toluene, and preferably THF or diethyl ether is used. The suitable aryl metal reagent includes aryl magnesium halide and aryl lithium. The above reaction can be performed normally from about -78˚C to about 25˚C (room temperature) for about 10 minutes to about 2 hours, preferably for about 1 hour.

The reaction converting Compound (IX) to Compound (X) can be achieved by performing a reaction with a suitable reducing reagent in a suitable solvent. The suitable solvent includes dichloromethane, dichloroethane, acetonitrile and toluene, and preferably dichloromethane or acetonitrile is used. The suitable reducing reagent preferably includes trifluoroboron-diethyl ether complex and triethylsilane. The above reaction can be performed normally from about -78˚C to about 25˚C (room temperature), preferably from about -40˚C to about 25˚C (room temperature) for about 10 minutes to about 6 hours, preferably for about 1 hour to about 2 hours.

The reaction converting Compound (X) to Compound (II) of the present invention can be achieved by performing a reaction with a suitable debenzylation reagent in a suitable solvent. The suitable solvent includes THF, ethyl acetate, methanol, ethanol and dichloromethane. The suitable debenzylation reagent includes palladium-carbon and hydrogen gas, palladium hydroxide-carbon and hydrogen gas, boron trichloride, boron tribromide, boron trichloride-dimethyl sulfide complex, boron trifluoride-diethyl ether complex and ethane thiol, boron trifluoride-diethyl ether complex and dimethyl sulfide, boron trichloride-pentamethylbenzene, sodium cyanide, sodium methanethiol, and preferably palladium-carbon and hydrogen gas, or boron trichloride-pentamethylbenzene is used. The above reaction can be performed normally from about -78˚C to about 100˚C, preferably from about -78˚C to about 25˚C (room temperature) for about 1 hour to about 24 hours, preferably for about 2 hours. In the case of reaction using palladium-carbon and hydrogen gas, the reaction may proceed smoothly in the presence of a catalytic amount of an acid, specifically hydrochloric acid.

Compound (III) of Scheme 1 can be synthesized by a process shown in Scheme 2, Scheme 3 and Scheme 4:

Scheme 2

[Formula 12]

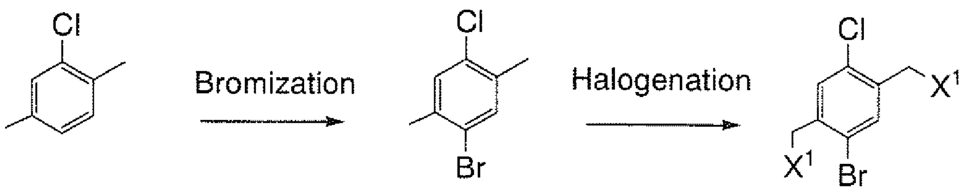


(XI) (XII) (XIII)

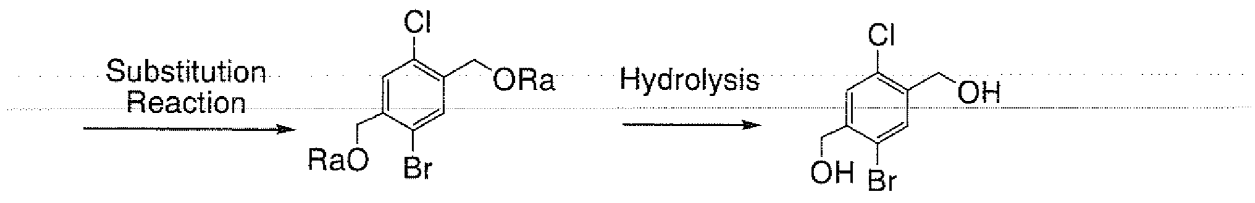


(XIV) (III: $R^{11}$=Cl)

wherein $X^1$ is a halogen atom such as a bromine atom and a chlorine atom, and Ra is an acyl group such as $C_{1-6}$ alkylcarbonyl and arylcarbonyl.

The reaction converting Compound (XI) to Compound (XII) can be achieved by performing a treatment with bromine in the presence of the iron powders. Specifically, it can be performed following a method described in a document (J. Prakt. Chem., 1889 <2>39, page 402).

The reaction converting Compound (XII) to Compound (XIII) can be achieved by performing a reaction with a suitable halogenation reagent in a suitable solvent. The suitable solvent includes ethyl acetate, ethyl acetate-water, and preferably ethyl acetate is used. The suitable halogenation reagent includes N-bromosuccinimide-2,2'-azobis(isobutyronitrile), N-bromosuccinimide-benzoyl peroxide, sodium bromate-sodium hydrogen sulfite, N-chlorosuccinimide-2,2'-azobis(isobutyronitrile), N-chlorosuccinimide-benzoyl peroxide, sulfuryl chloride-2,2'-azobis(isobutyronitrile), and preferably N-bromosuccinimide-2,2'-azobis(isobutyronitrile) is used. The above reaction can be performed normally from about 25°C (room temperature) to about 150°C, preferably from about 100°C to about 120°C for about 10 hours to about 24 hours, preferably for 15 minutes to about 1 hour.

The reaction converting Compound (XIII) to Compound (XIV) can be achieved by performing a reaction with a suitable carboxylate reagent in a suitable solvent. The suitable solvent includes dimethylformamide, acetonitrile, dimethoxyethane, ethyl acetate, and preferably dimethylformamide is used. The suitable carboxylate reagent includes sodium acetate, potassium acetate and sodium benzoate, and preferably sodium acetate is used. The above reaction can be performed normally from about 25°C (room temperature) to about 100°C, preferably at about 80°C for about 1 hour to about 24 hours, preferably for about 3 hours.

The reaction converting Compound (XIV) to Compound (III) can be achieved by performing a reaction with a suitable base reagent in a suitable solvent. The suitable solvent includes tetrahydrofuran-ethanol-water, tetrahydrofuran-methanol-water, ethanol-water, methanol-water, and preferably tetrahydrofuran-ethanol-water is used. The suitable base reagent includes sodium hydroxide, potassium hydroxide, lithium hydroxide, potassium carbonate, sodium carbonate, and preferably potassium hydroxide is used. The above reaction can be performed normally from about 0°C to about 100°C, preferably 25°C (room temperature) to about 80°C for about 15 minutes to about 24 hours, preferably for about 3 hours to about 5 hours.

Scheme 3

[Formula 13]

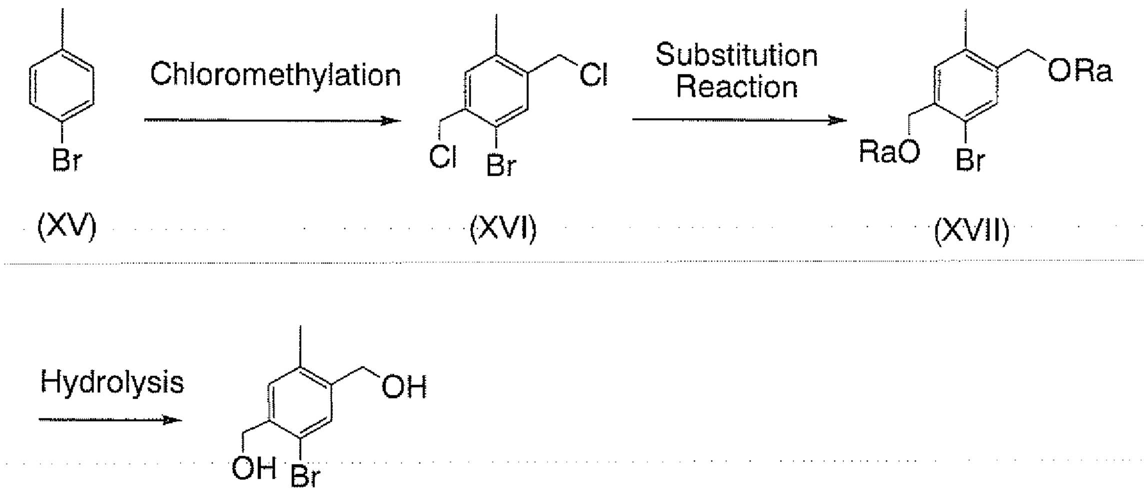


(XV) (XVI) (XVII)

(III: $R^{11}$=Me)

wherein Ra is an acyl group such as $C_{1-6}$ alkylcarbonyl and arylcarbonyl.

The reaction converting Compound (XV) to Compound (XVI) can be achieved following a method described in a document (J. Org. Chem., 1975, 40 (21), page 3101).

The reaction converting Compound (XVI) to Compound (XVII) can be achieved by a similar process as that for converting Compound (XIII) to Compound (XIV) in Scheme 2.

The reaction converting Compound (XVII) to Compound (III) can be achieved by a similar process as that for converting Compound (XIV) to Compound (III) in Scheme 2.

Scheme 4

[Formula 14]

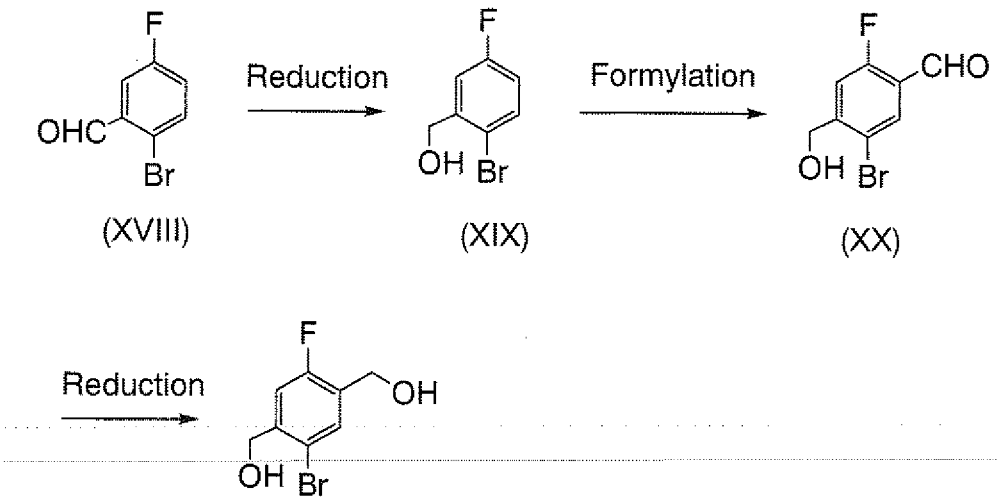


(XVIII) (XIX) (XX)

(III: $R^{11}$=F)

The reaction converting Compound (XVIII) to Compound (XIX) can be achieved by performing a reaction with a suitable reduction reagent in a suitable solvent. The suitable solvent includes methanol, ethanol and tetrahydrofuran. The suitable reduction reagent includes sodium borohydride, lithium borohydride, lithium aluminum hydride and diisobutyl aluminum hydride, and preferably sodium borohydride or diisobutyl aluminum hydride is used. The above reaction can be performed normally from about -20°C to about 50°C, preferably at about 0°C for about 10 minutes to about 5 hours, preferably about 20 minutes to about 3 hours. As a reference cited for this reaction, there is J. Org. Chem., No. 70, page 756, 2005.

The reaction converting Compound (XIX) to Compound (XX) can be achieved by performing a reaction with a suitable organic base reagent in a suitable solvent and then a reaction with a suitable formylation reagent. The suitable solvent includes THF, diethyl ether, dimethoxyethane, diethoxyethane, toluene, and preferably THF is used. The suitable organic base reagent includes n-butyllithium, sec-butylithium, tert-butylithium, methyllithium, n-butyllithium-2,2,6,6-tetramethylpiperidine, n-butyllithiumdiisopropylamine, and preferably n-butyllithium-2,2,6,6-tetramethylpiperidine is used. The suitable formylation reagent includes dimethylformamide, 1-formylpiperidine. The above reaction can be performed normally from about -78°C to about 25°C (room temperature), for about 10 minutes to about 5 hours, preferably about 1 hour to about 4 hours.

The reaction converting Compound (XX) to Compound (III) can be achieved by performing a reaction with a suitable reduction reagent in a suitable solvent. The suitable solvent includes methanol, ethanol, tetrahydrofuran. The suitable reduction reagent includes sodium borohydride, lithium borohydride, lithium aluminum hydride, and preferably sodium borohydride is used. The above reaction can be performed normally from about -20°C to about 50°C, preferably from about 0°C to about 25°C (room temperature), for about 5 minutes to about 24 hours, preferably about 10 minutes to about 1 hour.

The compound (X) of Scheme 1 can be also produced by a method of the following Scheme 5:

Scheme 5

[Formula 15]

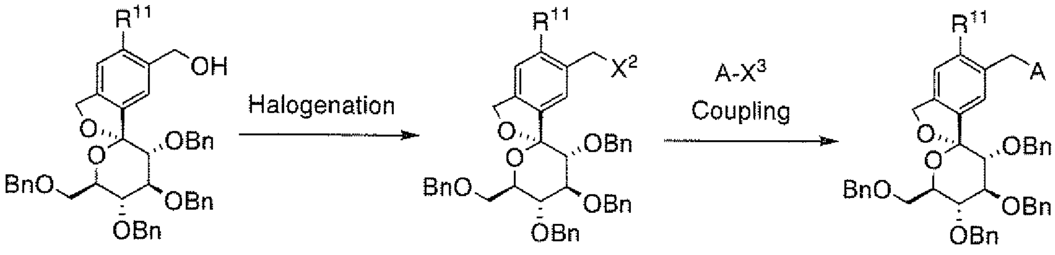


(VII) (XXI) (X)

wherein $R^{11}$ means the same as $R^1$ defined above, A means the same as defined above, $X^2$ represents a halogen atom, and $X^3$ represents a boron atom, a silyl atom, a magnesium atom, a zinc atom, a tin atom respectively having a substituent (s).

The reaction converting Compound (VII) to Compound (XXI) can be achieved by performing a reaction with a suitable halogenation reagent in a suitable solvent. The suitable solvent includes tetrahydrofuran, dichloromethane, dichloroethane, toluene, acetonitrile, and preferably dichloromethane is used. The suitable halogenation reagent includes carbon tetrachloride-triphenyl phosphine, carbon tetrabromide-triphenyl phosphine, thionyl chloride, thionyl bromide, and preferably carbon tetrachloride-triphenyl phosphine, or thionyl chloride is used. The above reaction can be performed normally from about -20˚C to about 60˚C, preferably from about 0˚C to about 25˚C (room temperature), for about 1 hour to about 24 hours, preferably about 1 hour to about 2 hours.

The reaction converting Compound (XXI) to Compound (X) can be achieved by performing a reaction with a suitable arylation agent ($A-X^3$) in a suitable solvent in the presence of a suitable transition metal catalyst, a suitable ligand, a suitable base and a suitable additive. The suitable solvent includes THF, dimethoxyethane, diethoxyethane, dioxane, N,N-dimethylformamide, N,N-dimethylacetamide, dimethylsulfoxide, 1,2-dichloroethane, toluene, xylene, ethanol, acetonitrile, water. The suitable transition metal catalyst includes palladium, nickel, cobalt, iron. The suitable ligand includes triphenyl phosphine, tri-tert-butyl phosphine, 2,2'-bis(diphenylphosphino)-1,1'-binaphthalene (BINAP), 1,2-bis (diphenylphosphino)ethane (dppe), 1,3-bis(diphenylphosphino)propane (dppp), 1,4-bis(diphenylphosphino)butane (dppb), 1,1'-bis(diphenylphosphino)ferrocene (dppf). The suitable base includes potassium acetate, sodium acetate, potassium phosphate, sodium phosphate, dipotassium hydrogen phosphate, sodium hydroxide, potassium hydroxide, sodium carbonate, potassium carbonate, cesium carbonate, triethylamine, diisopropylethylamine, 1,8-diazabicyclo [5,4,0]-7-undecene (DBU), 1,5-diazabicyclo[4,3,0]-5-nonene (DBN), sodium tert-butoxide, potassium tert-butoxide, tetramethylguanidine. The suitable additive includes tetra-n-butyl-ammonium bromide, tetra-n-butyl-ammonium iodide, sodium bromide, sodium iodide, potassium bromide, potassium iodide. The suitable arylation agent ($A-X^3$) includes arylboronic acid, arylboronic acid ester, aryl magnesium halide, aryl zinc, aryl lithium, aryl tin, aryl silane, and preferably arylboronic acid is used. The above reaction can be performed normally from about 0˚C to about 200˚C, preferably from about 80˚C to about 100˚C, for about 10 minutes to about 24 hours, preferably about 1 hour to about 16 hours. As for arylboronic acid preferable as an arylation agent ($A-X^3$), commercially available reagents can be used. When not commercially available, it can be synthesized following a method described in a reference (D. G. Hall, Boronic Acids: Preparation And Applications In Organic Synthesis And Medicines. (WILEY-VCH)).

The compounds of the present invention can be produced by a method of the following Scheme 6:

Scheme 6

[Formula 16]

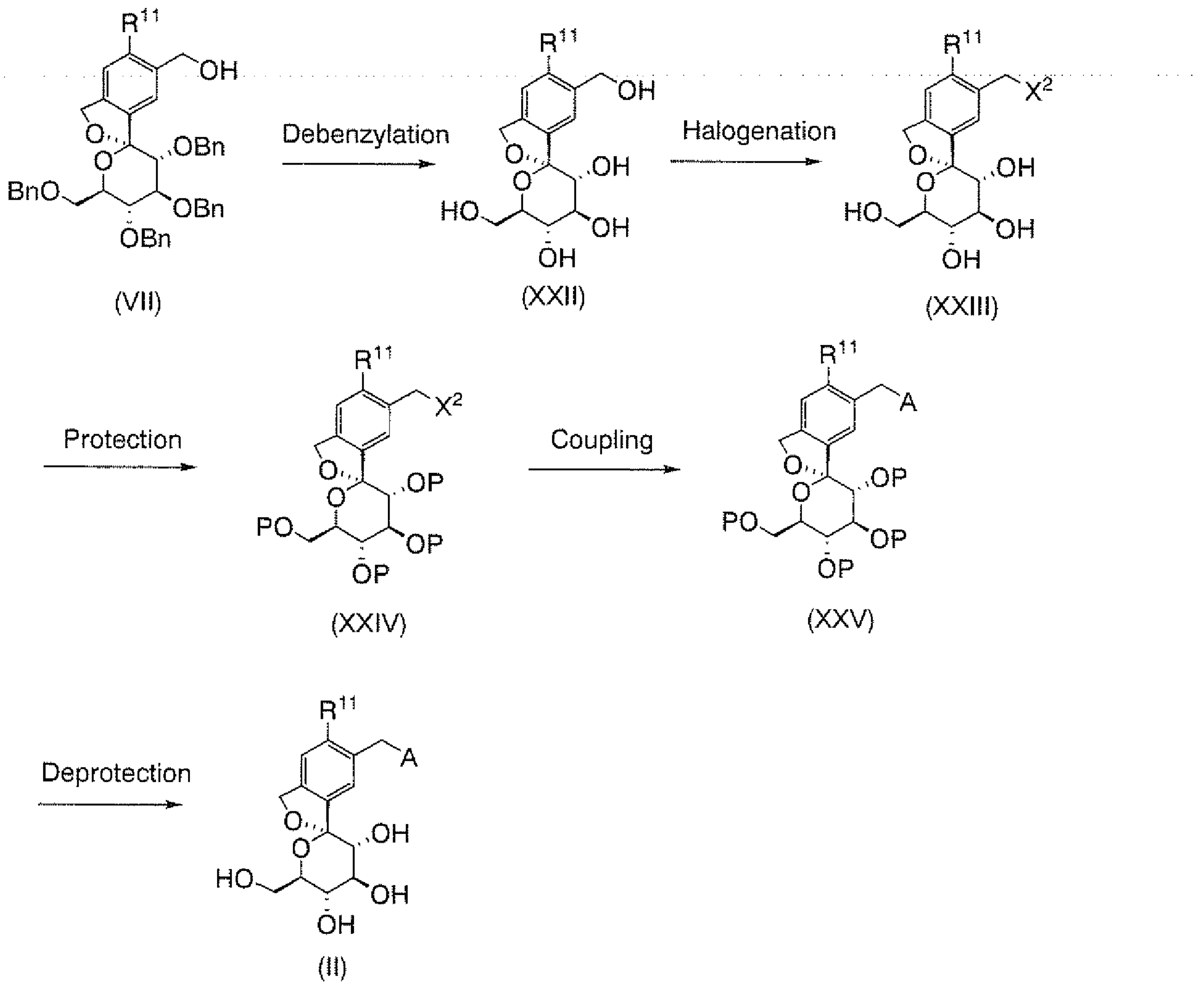


wherein $R^{11}$ means the same as $R^1$ defined above, A means the same as defined above, P represents a protecting group of a hydroxyl group such as $C_{1-6}$ alkylcarbonyl, $C_{1-6}$ alkoxycarbonyl, arylcarbonyl, and $X^2$ represents a halogen atom.

The reaction converting Compound (VII) to Compound (XXII) can be achieved by performing a reaction with a suitable debenzylation reagent in a suitable solvent. The suitable solvent includes dichloromethane, 1,2-dichloroethane, hexane, toluene, and preferably dichloromethane is used. The suitable debenzylation reagent includes boron trichloride, boron tribromide, boron trichloride-dimethyl sulfide complex, boron trifluoride-diethyl ether complex and ethane thiol, boron trifluoride-diethyl ether complex and dimethylsulfide, boron trichloride-pentamethylbenzene, sodium cyanide, sodium methanethiol. The above reaction can be performed normally from about -78˚C to about 100˚C, preferably from about -78˚C to about 25˚C (room temperature) for about 1 hour to about 24 hours.

The reaction converting Compound (XXII) to Compound (XXIII) can be achieved by performing a reaction with a suitable halogenation reagent in a suitable solvent. The suitable solvent includes dimethylsulfoxide, dimethylformamide, and preferably dimethylsulfoxide is used. The suitable halogenation reagent includes trimethylsilyl chloride, trimethylsilyl bromide, and preferably trimethylsilyl chloride is used. The above reaction can be performed normally from about -78˚C to about 50˚C, preferably at room temperature, for about 1 hour to about 5 hours.

The reaction converting Compound (XXIII) to Compound (XXIV) can be achieved by performing a reaction with a suitable protecting group introducing reagent in the presence of a suitable base in a suitable solvent. The suitable solvent includes tetrahydrofuran, dichloromethane, acetonitrile, ethyl acetate, dimethylformamide. The suitable base includes N-methylmorpholine, N,N-dimethylaminopyridine, triethylamine. The suitable protecting group introducing reagent includes acetic anhydride, acetyl chloride, methyl chlorocarbonate, ethyl chlorocarbonate, benzoyl chloride, and preferably acetic anhydride is used. The above reaction can be performed normally from about 0˚C to about 50˚C,

preferably at room temperature, for about 15 minutes to about 3 hours.

The reaction converting Compound (XXIV) to Compound (XXV) can be achieved by a similar process to the above reaction converting Compound (XXI) to Compound (X).

The reaction converting Compound (XXV) to Compound (II) can be achieved by performing a reaction with a suitable base reagent in a suitable solvent. The suitable solvent includes methanol, ethanol, ethanol-water, methanol-water, tetrahydrofuran-ethanol-water, tetrahydrofuran-methanol-water, and preferably methanol is used. The suitable base reagent includes sodium hydroxide, potassium hydroxide, lithium hydroxide, potassium carbonate, sodium carbonate, and preferably potassium carbonate is used. The above reaction can be performed normally from about 0˚C to about 100˚C, preferably at about 25˚C (room temperature), for about 15 minutes to about 24 hours, preferably for about 1 hour to about 2 hours.

Compound (XXII) of Schemes 6 can be produced by a method of the following Scheme 7:

Scheme 7

[Formula 17]

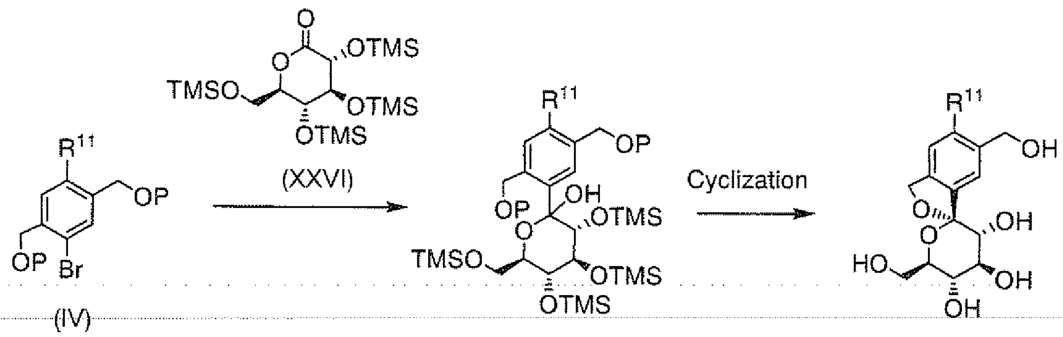


(IV) (XXVI) (XXVII) (XXII)

wherein $R^{11}$ means the same as $R^1$ defined above and P represents a protecting group of a hydroxyl group such as $C_{1-6}$ alkylcarbonyl, $C_{1-6}$ alkoxycarbonyl, arylcarbonyl.

The reaction converting Compound (IV) to Compound (XXVI) can be achieved by performing a reaction with a suitable alkyllithium reagent in a suitable solvent and then a reaction with Compound (XXVII) (2,3,4,6-tetrakis-O-(trimethylsilyl)-D-glucono-1,5-lactone). The suitable solvent includes THF, diethyl ether, dimethoxyethane, diethoxyethane, dichloromethane, toluene, and preferably THF or toluene is used. The suitable alkyllithium reagent includes n-butyllithium, sec-butylithium, tert-butylithium, methyllithium, and preferably n-butyllithium is used. The above reaction can be performed normally from about -78˚C to about 25˚C (room temperature), for about 10 minutes to about 2 hours, preferably for about 1 hour.

The reaction converting Compound (XXVII) to Compound (XXII) can be achieved by performing a reaction with a suitable acid catalyst in a suitable solvent while completing a deprotection step. The suitable solvent includes THF, dimethoxyethane, diethoxyethane, dichloromethane, toluene, methanol, ethanol, isopropanol, and preferably a mixture solvent of THF and methanol is used. The suitable acid catalyst includes p-toluenesulfonic acid, pyridinium-p-toluenesulfonic acid, methanesulfonic acid, trifluoromethanesulfonic acid, trifluoroacetic acid, camphorsulfonic acid, hydrochloric acid, sulfuric acid, acetic acid, and preferably p-toluenesulfonic acid is used. The above reaction can be performed normally from about -20˚C to about 100˚C, preferably from about 0˚C to about 60˚C, for about 10 minutes to about 24 hours, preferably for about 2 hours.

In this step, isomerization of the spiro moiety occurs simultaneously with cyclization, and a compound of desired steric arrangement can be obtained.

Compound (II) in which $R^1$ is an ethynyl group can be produced by a method of the following Scheme 8:

Scheme 8

[Formula 18]

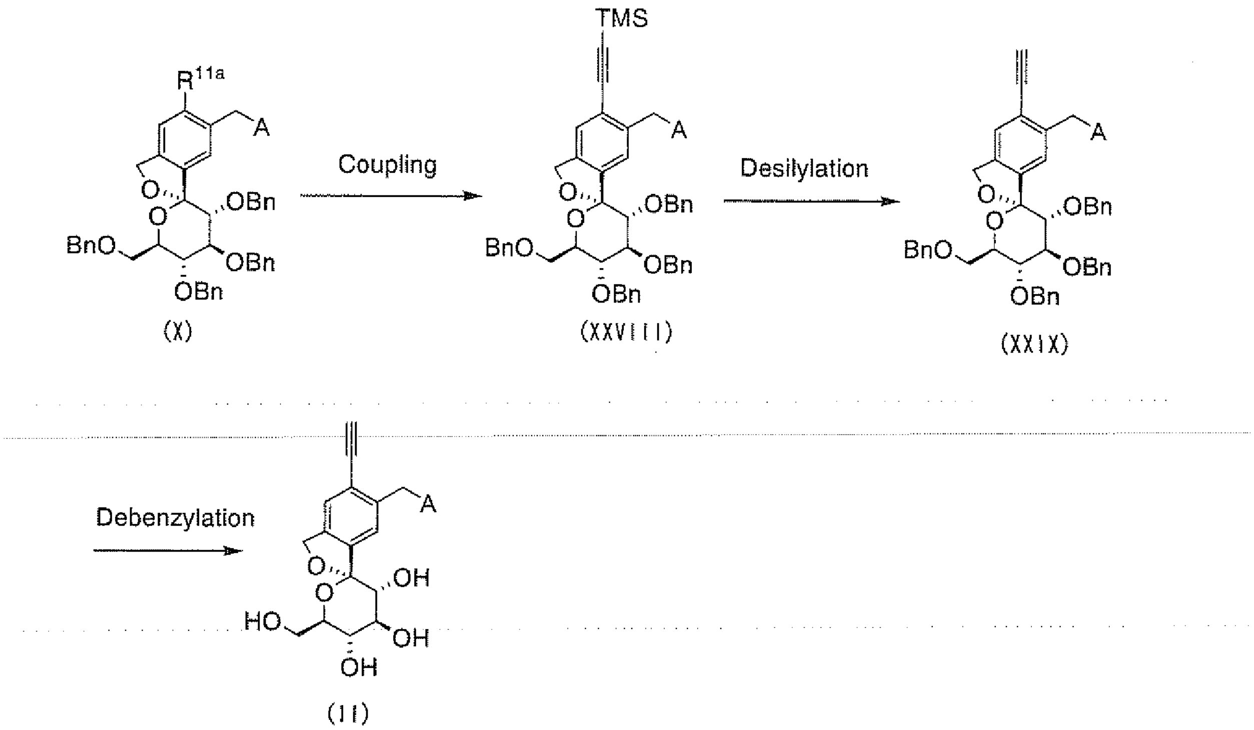

(X) (XXVIII) (XXIX) (II)

wherein $R^{11a}$ is a leaving group suitable for coupling reaction (for example, chlorine atom, bromine atom, trifluoromethanesulfonyloxy group, etc.) and A means the same as defined above.

The reaction converting Compound (X) to Compound (XXVIII) can be achieved by performing a reaction with ethynyltrimethylsilane in a suitable solvent in the presence of a suitable transition metal catalyst, a suitable ligand and a suitable base. The suitable solvent includes THF, dimethoxyethane, diethoxyethane, dioxane, N,N-dimethylformamide, N,N-dimethylacetamide, dimethylsulfoxide, 1,2-dichloroethane, toluene, xylene, ethanol, acetonitrile. The suitable transition metal catalyst includes palladium, nickel, cobalt, iron. The suitable ligand includes triphenylphosphine, 2-dicyclohexylphosphino-2',4',6'-triisopropylbiphenyl, tri-tert-butylphosphine, 2,2'-bis(diphenylphosphino)-1,1'-binaphthalene (BINAP), 1,2-bis(diphenylphosphino)ethane (dppe), 1,3-bis(diphenylphosphino)propane (dppp), 1,4-bis(diphenylphosphino)butane (dppb), 1,1'-bis(diphenylphosphino)ferrocene (dppf), acetonitrile. The suitable base includes potassium acetate, sodium acetate, potassium phosphate, sodium phosphate, dipotassium hydrogen phosphate, sodium hydroxide, potassium hydroxide, sodium carbonate, potassium carbonate, cesium carbonate, triethylamine, diisopropylethylamine, DBU, DBN, sodium tert-butoxide, potassium tert-butoxide, tetramethylguanidine. The above reaction can be performed normally from about 0°C to about 200°C, preferably from about 25°C (room temperature) to about 100°C, for about 10 minutes to about 24 hours, preferably for about 1 hour to about 4 hours.

The reaction converting Compound (XXVIII) to Compound (XXIX) can be achieved by performing a reaction with a suitable desilylation agent in a suitable solvent. The suitable solvent includes methanol, ethanol, water, tetrahydrofuran, and methanol is preferable. The suitable desilylation agent includes potassium carbonate, sodium carbonate, sodium hydroxide, potassium hydroxide, sodium methoxide, tetrabutylammonium fluoride, potassium fluoride. The above reaction can be performed normally from about 0°C to about 100°C, preferably at room temperature, for about 1 hour to about 24 hours.

The reaction converting Compound (XXIX) to Compound (II) can be achieved by performing a reaction with a suitable debenzylation reagent in a suitable solvent. The suitable solvent includes dichloromethane, 1,2-dichloroethane. The suitable debenzylation reagent includes boron trichloride, boron tribromide, boron trichloride-dimethyl sulfide complex, boron trifluoride-diethyl ether complex and ethanethiol, boron trifluoride-diethyl ether complex and dimethylsulfide, boron trichloride-pentamethylbenzene, sodium cyanide, sodium methanethiolate. The above reaction can be performed normally from about -78°C to about 100°C, preferably from about -78°C to about 25°C (room temperature) for about 1 hour to about 24 hours.

Compound (II) in which $R^1$ is an ethynyl group can be produced by a method of the following Scheme 9:

Scheme 9

[Formula 19]

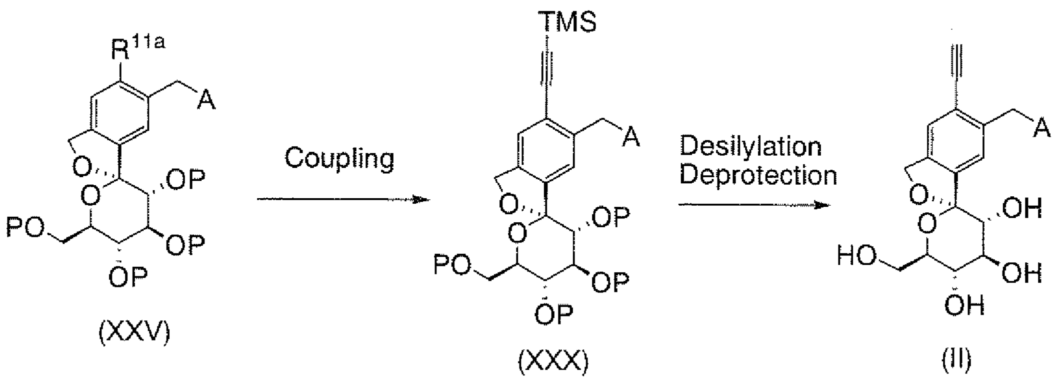


(XXV) (XXX) (II)

wherein $R^{11a}$ is a leaving group suitable for coupling reaction (for example, chlorine atom, bromine atom, trifluoromethanesulfonyloxy group, etc.), P represents an appropriate protecting group and A means the same as defined above.

The reaction converting Compound (XXV) to Compound (XXX) can be achieved by performing a reaction with ethynyltrimethylsilane in a suitable solvent in the presence of a suitable transition metal catalyst, a suitable ligand and a suitable base. The suitable solvent includes acetonitrile, tetrahydrofuran, dimethylformamide, dioxane, dimethylsulfoxide, toluene, dimethoxyethane, and preferably acetonitrile is used. The suitable transition metal catalyst includes palladium, nickel, cobalt, iron, and preferably palladium is used. The suitable ligand includes triphenylphosphine, 2-dlcyclohexylphosphino-2',4',6'-triisopropylbiphenyl, tri-tert-butylphosphine, 2,2'-bis(diphenylphosphino)-1,1'-binaphthalene (BINAP), 1,2-bis(diphenylphosphino)ethane (dppe), 1,3-bis(diphenylphosphino)propane (dppp), 1,4-bis(diphenylphosphino)butane (dppb), 1,1'-bis(diphenylphosphino)ferrocene (dppf), acetonitrile, and preferably 2-dicyclohexylphosphino-2',4',6'-triisopropylbiphenyl is used. The suitable base includes potassium carbonate, sodium carbonate, cesium carbonate, sodium acetate, potassium acetate, sodium hydroxide, potassium hydroxide, potassium phosphate, sodium phosphate, dipotassium hydrogen phosphate, triethylamine, and preferably sodium carbonate or cesium carbonate is used. The above reaction can be performed normally from about 0˚C to about 120˚C, preferably at about 25˚C (room temperature), for about 1 hour to about 24 hours, preferably for about 1 hour to about 4 hours.

The protecting group P of Compound (XXX) is preferably an acetyl group, a benzoyl group, a methoxycarbonyl group, an ethoxycarbonyl group. The reaction converting Compound (XXX) to Compound (II) can be achieved by performing a reaction with a suitable base in a suitable solvent. The suitable solvent includes methanol, ethanol, water, tetrahydrofuran, acetonitrile, and preferably methanol is used. The suitable base includes potassium carbonate, sodium carbonate, potassium hydroxide, sodium hydroxide, sodium methoxide, and preferably potassium carbonate is used. The above reaction can be performed normally from about 0˚C to about 100˚C, preferably at about 25˚C (room temperature), for about 1 hour to about 24 hours, preferably for about 1 hour to about 3 hours.

Compound (II) in which $R^2$ is an alkynyl group can be produced by a method of the following Scheme 10:

Scheme 10

[Formula 20]

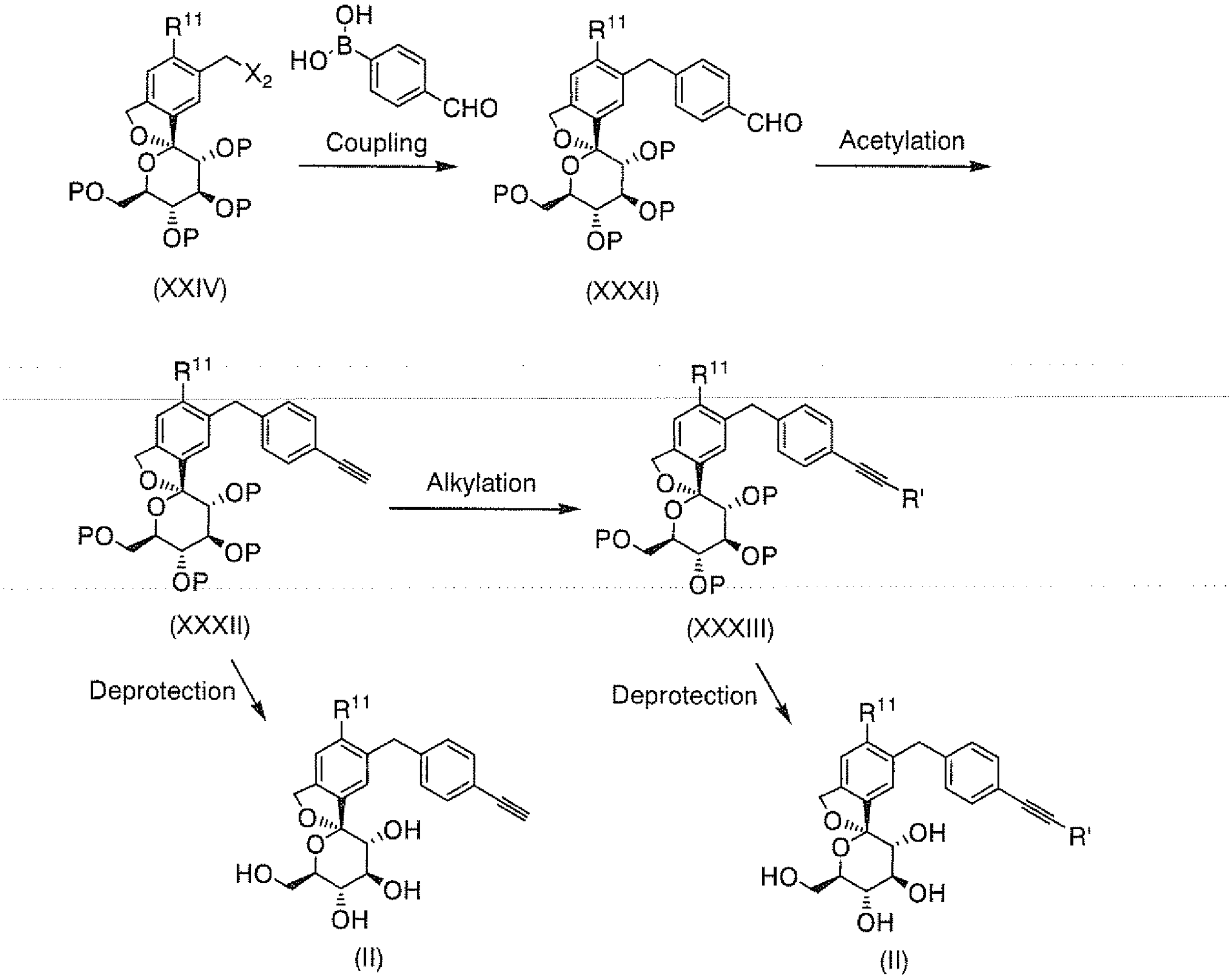


(XXIV) (XXXI)

(XXXII) (XXXIII)

(II) (II)

wherein $R^{11}$ means the same as $R^1$ defined above, $X^2$ represents a halogen atom, and P represents an appropriate protecting group of a hydroxyl group, and R' is a $C_{1-4}$ alkyl which may be substituted with $-OR^4$.

For protecting group P of Compound (XXIV), an ether-type protecting group such as a benzyl group, a p-methoxy benzyl group and an allyl group is preferable, and a benzyl group is particularly preferable.

The reaction converting Compound (XXIV) to Compound (XXXI) can be achieved by performing a reaction with a p-formylphenylation agent (preferably, p-formylphenylboronic acid) in a suitable solvent in the presence of a suitable transition metal catalyst, a suitable ligand, a suitable base and a suitable additive. The suitable solvent includes THF, dimethoxyethane, diethoxyethane, dioxane, N,N-dimethylformamide, N,N-dimethylacetamide, dimethylsulfoxide, 1,2-dichloroethane, toluene, xylene, ethanol, acetonitrile, water. The suitable transition metal catalyst includes palladium, nickel, cobalt, iron. The suitable ligand includes triphenylphosphine, tri-tert-butylphosphine, 2,2'-bis(diphenylphosphino)-1,1'-binaphthalene (BINAP), 1,2-bis(diphenylphosphino)ethane (dppe), 1,3-bis(diphenylphosphino)propane (dppp), 1,4-bis(diphenylphosphino)butane (dppb), and 1,1'-bis(diphenylphosphino)ferrocene (dppf). The suitable base includes potassium acetate, sodium acetate, potassium phosphate, sodium phosphate, dipotassium hydrogen phosphate, sodium hydroxide, potassium hydroxide, sodium carbonate, potassium carbonate, cesium carbonate, triethylamine, diisopropylethylamine, 1,8-diazabicyclo[5,4,0]-7-undecene (DBU), 1,5-diazabicyclo[4,3,0]-5-nonene (DBN), sodium tert-butoxide, potassium tert-butoxide, tetramethylguanidine. The suitable additive includes tetra-n-butylammonium bromide, tetra-n-butylammonium iodide, sodium bromide, sodium iodide, potassium bromide, potassium iodide. The above reaction can be performed normally from about 0°C to about 200°C, preferably from about 80°C to about 160°C, for about 10 minutes to about 24 hours, preferably about 15 minutes to about 16 hours.

The reaction converting Compound (XXXI) to Compound (XXXII) can be achieved by reacting a suitable base and a suitable ethynylation agent in a suitable solvent. The suitable solvent includes THF, dimethoxyethane, diethoxyethane, dioxane, dichloromethane, 1,2-dichloroethane, toluene, xylene, methanol, ethanol, and preferably a mixture solvent of THF and methanol is used. The suitable base includes potassium carbonate, sodium carbonate, sodium

hydroxide, potassium hydroxide. The suitable ethynylation agent includes dimethyl(1-diazo-2-oxopropyl)phosphonate. The above reaction can be performed normally from about 0˚C to about 120˚C, preferably from about 0˚C to about 25˚C (room temperature), for about 10 minutes to about 16 hours, preferably about 3 hours to about 5 hours. Dimethyl(1-diazo-2-oxopropyl) phosphonate usable as an ethynylation agent can be synthesized, for example, by following a method described in a document (Eur. J. Org. Chem., page 821, 2003).

The reaction converting Compound (XXXII) to Compound (XXXIII) can be achieved by performing a reaction with a suitable base in a suitable solvent and then a reaction with a suitable alkylating agent. The suitable solvent includes THF, diethyl ether, dimethoxyethane, diethoxyethane, toluene, and preferably THF is used. The suitable base includes n-butyllithium, sec-butylithium, tert-butylithium, methyllithium, and preferably n-butyllithium is used. The suitable alkylating agent includes alkyl halide, aldehyde, ketone, and preferably alkyl halide is used. The above reaction can be performed normally from about -78˚C to about 25˚C (room temperature), for about 1 hour to about 5 hours.

The reaction converting Compound (XXXII) or Compound (XXXIII) to Compound (II) is a deprotection reaction and removal of benzyl group, which is preferred as a protecting group, can be achieved by performing a reaction with a suitable debenzylation reagent in a suitable solvent. The suitable solvent includes dichloromethane, 1,2-dichloroethane. The suitable debenzylation reagent includes boron trichloride, boron tribromide, boron trichloride-dimethyl sulfide complex, boron trifluoride diethyl ether complex and ethanethiol, boron trifluoride-diethyl ether complex and dimethylsulfide, boron trichloride-pentamethylbenzene, sodium cyanide, sodium methanethiolate, and preferably boron trichloride-pentamethylbenzene is used. The above reaction can be performed normally from about -78˚C to about 25˚C (room temperature), preferably from about -78˚C to about 0˚C, for about 1 hour to about 7 hours, preferably for about 2 hours to about 3 hours.

The compound (II) in which $R^2$ is an alkynyl group can be produced by a process of the following Scheme 11:

Scheme 11

[Formula 21]

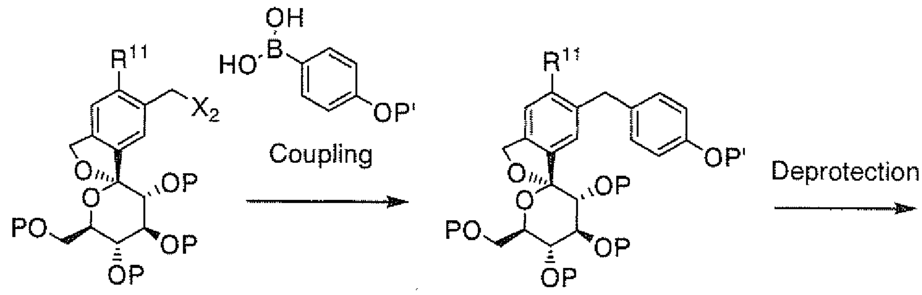


(XXIV) (XXXIV)

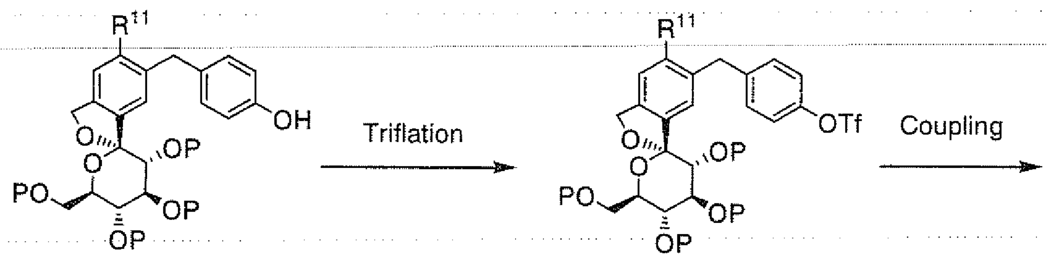


(XXXV) (XXXVI)

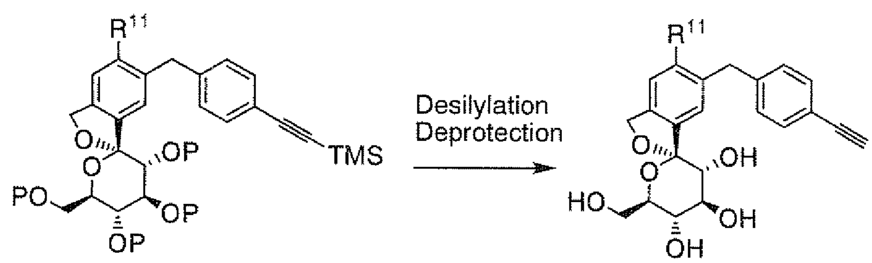


(XXXVII) (II)

wherein $R^{11}$ means the same as $R^1$ defined above, $X^2$ represents a halogen atom, and P and P' respectively represent an appropriate protecting group of a hydroxyl group.

The reaction converting Compound (XXIV) to Compound (XXXIV) can be achieved by performing a reaction with a suitable aryl boronic acid in a suitable solvent in the presence of a suitable transition metal catalyst, a suitable ligand, a suitable base and a suitable additive. The suitable solvent includes THF, dimethoxyethane, diethoxyethane, dioxane, N,N-dimethylformamide, N,N-dimethylacetamide, dimethylsulfoxide, 1,2-dichloroethane, toluene, xylene, ethanol, acetonitrile, water. The suitable transition metal catalyst includes palladium, nickel, cobalt, iron. The suitable ligand includes triphenylphosphine, tri-tert-butyl phosphine, 2,2'-bis(diphenylphosphino)-1,1'-binaphthalene (BINAP), 1,2-bis (diphenylphosphino)ethane (dppe), 1,3-bis(diphenylphosphino)propane (dppp), 1,4-bis(diphenylphosphino)butane (dppb), 1,1'-bis(diphenylphosphino)ferrocene (dppf). The suitable base includes potassium acetate, sodium acetate, potassium phosphate, sodium phosphate, dipotassium hydrogen phosphate, sodium hydroxide, potassium hydroxide, sodium carbonate, potassium carbonate, cesium carbonate, triethylamine, diisopropylethylamine, 1,8-diazabicyclo [5,4,0]-7-undecene (DBU), 1,5-diazabicyclo[4,3,0]-5-nonene (DBN), sodium tert-butoxide, potassium tert-butoxide, tetramethylguanidine. The suitable additive includes tetra-n-butylammonium bromide, tetra-n-butylammonium iodide, sodium bromide, sodium iodide, potassium bromide, potassium iodide. The suitable O-protecting group of a suitable aryl boronic acid includes silane protecting groups such as trimethylsilyl group, tert-butyldimethylsilyl group, triisopropylsilyl group, triethylsilyl group, tert-butyldiphenylsilyl group; and ether protecting groups such as methoxymethyl group, methoxyethoxymethyl group, tetrahydropyranyl group, trityl group, benzyl group, p-methoxy benzyl group, and preferably silane protecting groups are used. The above reaction can be performed normally from about 0°C to about 200°C, preferably from about 80°C to about 100°C, for about 10 minutes to about 24 hours, preferably about 1 hour to about 16 hours. The aryl boronic acid can be obtained by protecting a phenolic hydroxyl group of commercially available 4-hydroxyphenyl boronic acid with a suitable protecting group.

The reaction converting Compound (XXXIV) to Compound (XXXV) can be achieved by performing a reaction with a suitable desilylation agent in a suitable solvent in the case that a silane protecting group, which is preferred as a protecting group of a phenol hydroxyl group, is used. The suitable solvent includes tetrahydrofuran, diethyl ether, dimethoxyethane, diethoxyethane, dioxane, N,N-dimethylformamide, N,N-dimethylacetamide, dimethylsulfoxide, dichloromethane, 1,2-dichloroethane, toluene, xylene, ethanol, acetonitrile, water, and preferably tetrahydrofuran is used. The suitable desilylation agent includes tetrabutylammonium fluoride, potassium fluoride, cesium fluoride, hydrogen fluoride, acetic acid, hydrochloric acid, sulfuric acid, trifluoroacetic acid, p-toluenesulfonic acid, triethylamine-hydrogen fluoride, pyridine-hydrogen fluoride, and preferably tetrabutylammonium fluoride is used. The above reaction can be performed normally from about -20˚C to about 100˚C, preferably from about 0˚C to about 25˚C (room temperature), for about 10 minutes to about 24 hours, preferably about 15 minutes to about 5 hours.

The reaction converting Compound (XXXV) to Compound (XXXVI) can be achieved by performing a reaction with a suitable triflation agent in a suitable solvent in the presence of a suitable base. The suitable solvent includes tetrahydrofuran, diethyl ether, dimethoxyethane, diethoxyethane, dioxane, N,N-dimethylformamide, N,N-dimethyl acetamide, dichloromethane, 1,2-dichloroethane, toluene, xylene, acetonitrile, and preferably dichloromethane is used. The suitable base includes pyridine, triethylamine, diisopropylethylamine, N,N-dimethylaminopyridine, and preferably pyridine is used. The suitable triflation agent includes trifluoromethanesulfonic anhydride. The above reaction can be performed normally from about -78˚C to about 25˚C (room temperature), preferably from about -20˚C to about 25˚C (room temperature), for about 10 minutes to about 24 hours, preferably about 1 hour to about 6 hours.

The reaction converting Compound (XXXVI) to Compound (XXXVII) can be achieved by performing a reaction with trimethylsilyl acetylene in a suitable solvent in the presence of a suitable transition metal catalyst, a suitable ligand, a suitable base and a suitable additive. The suitable solvent includes THF, dimethoxyethane, diethoxyethane, dioxane, N,N-dimethylformamide, N,N-dimethylacetamide, dimethylsulfoxide, 1,2-dichloroethane, toluene, xylene, ethanol, acetonitrile, water, and preferably N,N-dimethylformamide is used. The suitable transition metal catalyst includes palladium, nickel, cobalt, iron, and preferably palladium is used. The suitable ligand includes triphenylphosphine, tri-tert-butyl phosphine, 2,2'-bis(diphenylphosphino)-1,1'-binaphthalene (BINAP), 1,2-bis(diphenylphosphino)ethane (dppe), 1,3-bis (diphenylphosphino)propane (dppp), 1,4-bis(diphenylphosphino)butane (dppb), 1,1'-bis(diphenylphosphino)ferrocene (dppf). The suitable base includes potassium acetate, sodium acetate, potassium phosphate, sodium phosphate, dipotassium hydrogen phosphate, sodium hydroxide, potassium hydroxide, sodium carbonate, potassium carbonate, cesium carbonate, triethylamine, diisopropylethylamine, 1,8-diazabicyclo[5,4,0]-7-undecene (DBU), 1,5-diazabicyclo[4,3,0]-5-nonene (DBN), sodium tert-butoxide, potassium tert-butoxide, tetramethylguanidine. The suitable additive includes copper (I) iodide. The above reaction can be performed normally from about 0˚C to about 200˚C, preferably from about 80˚C to about 100˚C, for about 10 minutes to about 24 hours, preferably about 1 hour to about 6 hours.

The reaction converting Compound (XXXVII) to Compound (II) can be achieved by performing a deprotection reaction simultaneously with desilylation or performing a deprotection reaction after performing desilylation. When the protecting group is an acyl group such as an acetyl group, a benzoyl group, a methoxycarbonyl group, an ethoxycarbonyl group, deprotection and desilylation can be performed simultaneously by performing a reaction with a suitable base in a suitable solvent. The suitable solvent includes methanol, ethanol, water, tetrahydrofuran, acetonitrile, and preferably methanol is used. The suitable base includes potassium carbonate, sodium carbonate, potassium hydroxide, sodium hydroxide, sodium methoxide, and preferably potassium carbonate is used. The above reaction can be performed normally from about 0˚C to about 100˚C, preferably at about 25˚C (room temperature), for about 1 hour to about 24 hours, preferably about 1 hour to about 3 hours. When the protecting group is a benzyl group, this step can be achieved by performing a reaction with a suitable debenzylation reagent in a suitable solvent after conducting desilylation reaction by the above-mentioned method. The suitable solvent includes dichloromethane, 1,2-dichloroethane. The suitable debenzylation reagent includes boron trichloride, boron tribromide, boron trichloride-dimethyl sulfide complex, boron trifluoride-diethyl ether complex and ethanethiol, boron trifluoride-diethyl ether complex and dimethylsulfide, boron trichloride-pentamethylbenzene, sodium cyanide, sodium methanethiolate, and preferably boron trichloride-pentamethylbenzene is used. The above reaction can be performed normally from about -78˚C to about 25˚C (room temperature), preferably from about -78˚C to about 0˚C, for about 1 hour to about 7 hours, preferably for about 2 hours to about 3 hours.

Compound (XXXVII), Compound (XXXII) and Compounds (XXXIII) of Schemes 10 and 11 can also be produced by the following Scheme 12:

Scheme 12

[Formula 22]

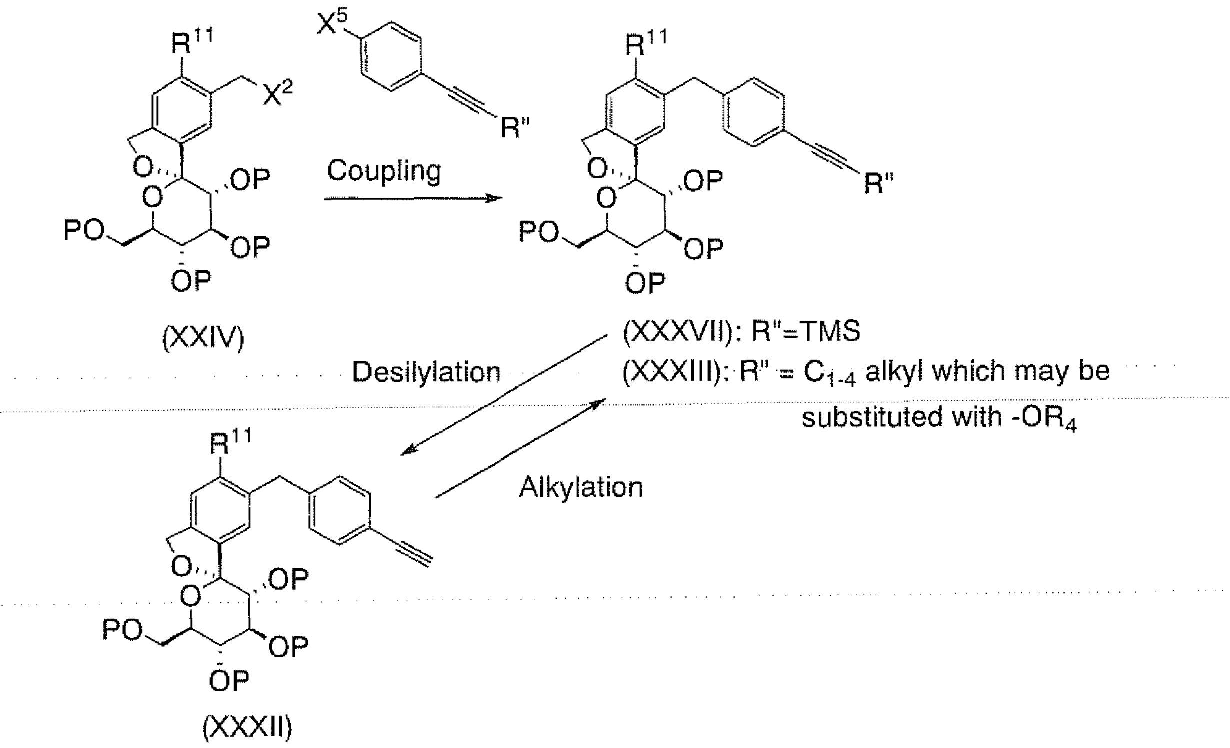


wherein $R^{11}$ means the same as $R^1$ defined above, $X^2$ represents a halogen atom, $X^5$ represents a boron atom, a silyl atom, a magnesium atom, a zinc atom, a tin atom respectively having a substituent(s), P represents an appropriate protecting group of a hydroxyl group and R" is a $C_{1-4}$ alkyl which may be substituted with $-OR^4$ or trimethylsilyl.

Compound (XXXVII) and Compound (XXXIII) can be synthesized by reacting Compound (XXIV) with a suitable p-alkynyl substituted phenylation agent in a suitable solvent in the presence of a suitable transition metal catalyst, a suitable ligand, a suitable base and a suitable additive. The suitable solvent includes THF, dimethoxyethane, diethoxyethane, dioxane, N,N-dimethylformamide, N,N-dimethylacetamide, dimethylsulfoxide, 1,2-dichloroethane, toluene, xylene, ethanol, acetonitrile, water. The suitable transition metal catalyst includes palladium, nickel, cobalt, iron. The suitable ligand includes triphenylphosphine, tri-tert-butyl phosphine, 2,2'-bis(diphenylphosphino)-1,1'-binaphthalene (BINAP), 1,2-bis(diphenylphosphino)ethane (dppe), 1,3-bis(diphenylphosphino)propane (dppp), 1,4-bis(diphenylphosphino)butane (dppb), 1,1'-bis(diphenylphosphino)ferrocene (dppf). The suitable base includes potassium acetate, sodium acetate, potassium phosphate, sodium phosphate, dipotassium hydrogen phosphate, sodium hydroxide, potassium hydroxide, sodium carbonate, potassium carbonate, cesium carbonate, triethylamine, diisopropylethylamine, 1,8-diazabicyclo[5,4,0]-7-undecene (DBU), 1,5-diazabicyclo[4,3,0]-5-nonene (DBN), sodium tert-butoxide, potassium tert-butoxide, tetramethylguanidine. The suitable additive includes tetra-n-butylammonium bromide, tetra-n-butylammonium iodide, sodium bromide, sodium iodide, potassium bromide, potassium iodide. The suitable p-alkynyl substituted phenylation agent includes compounds in which $X^5$ is boronic acid, boronic acid ester, magnesium halide, zinc, lithium, tin, silane, and preferably boronic acid compound is used. The above reaction can be performed normally from about 25°C (room temperature) to about 200°C, preferably from about 80°C to about 120°C, for about 10 minutes to about 24 hours, preferably about 1 hour to about 16 hours.

Compound (XXXII) can be synthesized by reacting Compound (XXXVII) with a suitable base in a suitable solvent. As for this case, however, the protecting group is suitably a group such as a benzyl group which can stand against a basic condition. The suitable solvent includes methanol, ethanol, water, tetrahydrofuran, acetonitrile, and preferably methanol is used. The suitable base includes potassium carbonate, sodium carbonate, potassium hydroxide, sodium hydroxide, sodium methoxide, and preferably potassium carbonate is used. The above reaction can be performed normally from about 0°C to about 100°C, preferably at about 25°C (room temperature), for about 1 hour to about 24 hours, preferably about 1 hour to about 3 hours.

Compounds (II) in which $R^2$ is an alkynyl group can be produced by a process shown in the following Scheme 13:

Scheme 13

[Formula 23]

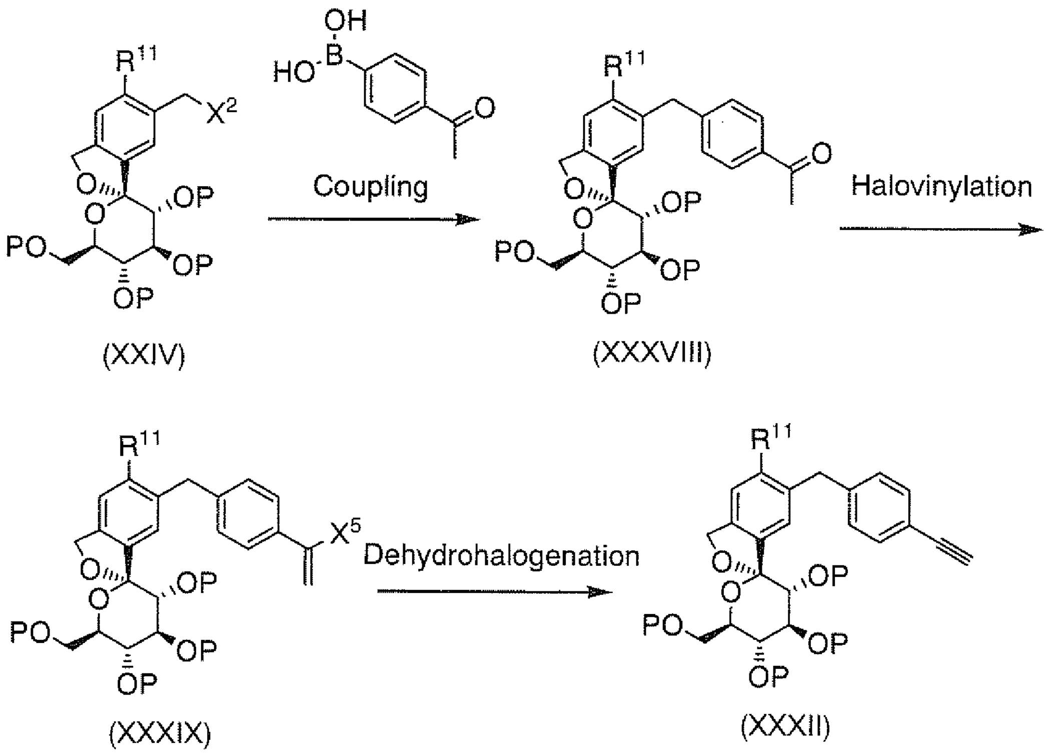


(XXIV) (XXXVIII) (XXXIX) (XXXII)

wherein $R^{11}$ means the same as $R^1$ defined above, $X^2$ and $X^5$ respectively represent a halogen atom, and P represents an appropriate protecting group of a hydroxyl group.

The reaction converting Compound (XXIV) to Compound (XXXVIII) can be achieved by performing a reaction with 4-acetylphenyl boronic acid in a suitable solvent in the presence of a suitable transition metal catalyst, a suitable ligand, a suitable base and a suitable additive. The suitable solvent includes THF, dimethoxyethane, diethoxyethane, dioxane, N,N-dimethylformamide, N,N-dimethylacetamide, dimethylsulfoxide, 1,2-dichloroethane, toluene, xylene, ethanol, acetonitrile, water. The suitable transition metal catalyst includes palladium, nickel, cobalt, iron. The suitable ligand includes triphenylphosphine, tri-tert-butyl phosphine, 2,2'-bis(diphenylphosphino)-1,1'-binaphthalene (BINAP), 1,2-bis (diphenylphosphino)ethane (dppe), 1,3-bis(diphenylphosphino)propane (dppp), 1,4-bis(diphenylphosphino)butane (dppb), 1,1'-bis(diphenylphosphino)ferrocene (dppf). The suitable base includes potassium acetate, sodium acetate, potassium phosphate, sodium phosphate, dipotassium hydrogen phosphate, sodium hydroxide, potassium hydroxide, sodium carbonate, potassium carbonate, cesium carbonate, triethylamine, diisopropylethylamine, 1,8-diazabicyclo [5,4,0]-7-undecene (DBU), 1,5-diazabicyclo[4,3,0]-5-nonene (DBN), sodium tert-butoxide, potassium tert-butoxide, tetramethylguanidine. The suitable additive includes tetra-n-butylammonium bromide, tetra-n-butylammonium iodide, sodium bromide, sodium iodide, potassium bromide, potassium iodide. The above reaction can be performed normally from about 25°C (room temperature) to about 200°C, preferably from about 80°C to about 120°C, for about 10 minutes to about 24 hours, preferably about 1 hour to about 16 hours.

The reaction converting Compound (XXXVIII) to Compound (XXXIX) can be achieved by performing a reaction with phosphorus oxychloride in a suitable solvent. The suitable solvent includes dichloromethane, 1,2-dichloroethane, toluene, xylene. The above reaction can be performed normally from about 25°C (room temperature) to about 200°C, preferably from about 60°C to about 120°C, for about 10 minutes to about 24 hours.

The reaction converting Compound (XXXIX) to Compound (XXXII) can be achieved by performing a reaction with a suitable base in a suitable solvent. The suitable solvent includes tetrahydrofuran, dimethoxyethane, diethoxyethane, methanol, ethanol, tert-butanol. The suitable base includes potassium hydroxide, sodium hydroxide, potassium tert-butoxide, sodium amide. The above reaction can be performed normally from about 25°C (room temperature) to about 200°C, preferably from about 25°C (room temperature) to about 80°C, for about 10 minutes to about 24 hours. Here, the protecting group is suitably a group such as a benzyl group which can stand against a basic condition. When

the protecting group is an acyl group such as an acetyl group, a benzoyl group, a methoxycarbonyl group, an ethoxycarbonyl group, deprotection reaction proceeds at the same time and Compound (II) is obtained.

Compound (XXXII) can be converted into a desired Compound (II) by appropriately performing a combination of the steps described in Schemes 10-13.

The production process of the compounds of the present invention is not limited to the methods described above. The compounds of the invention can be synthesized, for example, by combining steps included in Schemes 1-13 appropriately.

[EXAMPLES]

The subject matter of the present invention will now be described in more detail with reference to the following examples and test examples. However, the present invention shall not be limited to such subject matter.

In the following examples, the respective symbols have the following meaning:

NMR: Nuclear magnetic resonance spectrum (TMS internal standard); MS: mass spectrometry value; HPLC: high performance liquid chromatography.

The NMR, MS and HPLC were carried out using the following equipment.

NMR: JEOL JNM-EX-270 (270 MHz), Brucker ARX300 (300 MHz), Varian Mercury 300 (300 MHz), or JEOL JNM-ECP400 (400 MHz).

MS: LCQ manufactured by Thermo Finnigan, Micromass ZQ manufactured by Waters or a Q-micro Triple Quadrupole Mass Spectrometer

HPLC: 2690/2996 (detector) manufactured by Waters

Example 1

(1S,3'R,4'S,5'S,6'R)-5-chloro-6-[(4-ethylphenyl)methyl]-3',4',5',6'-tetrahydro-6'-(hydroxymethyl)-spiro[isobenzofuran-1(3H),2'-[2H]pyran]-3',4',5'-triol

[Formula 24]

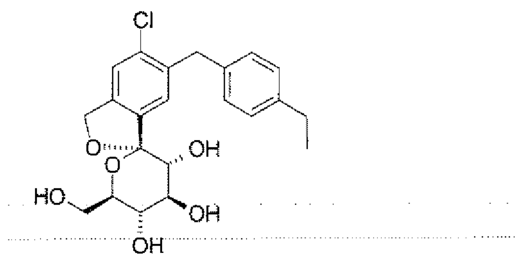

1) Synthesis of (4-acetoxymethyl-2-bromo-5-chloro)benzyl acetate

To a solution (45 mL) of 1-bromo-4-chloro-2,5-dimethylbenzene (10.0 g, 45.5 mmol) in ethyl acetate was added N-bromosuccinimide (21.0 g, 118.4 mmol) and 2,2'-azobis(isobutyronitrile) (300 mg), and the resultant mixture was stirred for 20 minutes at 100 to 120°C. The reaction mixture was cooled to room temperature and then ethyl acetate was added thereto. The resultant mixture was then successively washed with water and saturated brine. The organic layer was dried over anhydrous magnesium sulfate, and the solvent was then removed by distillation under reduced pressure. The obtained crude product (20.8 g) was dissolved in DMF (100 mL), and sodium acetate (11.2 g, 136.5 mmol) was added thereto. The resultant mixture was stirred for 3 hours at 80°C. The reaction mixture was cooled to room temperature and then dichloromethane was added thereto. The mixture was then successively washed with water and saturated brine. The organic layer was dried over anhydrous magnesium sulfate, and the solvent was then removed by distillation under reduced pressure. To the resulting residue was added ethyl acetate (100 mL), and this solution was stirred for 15 hours at room temperature. Undissolved material was collected by filtration, to thereby obtain the titled compound (5.9 g, 38.6%).

$^1$H-NMR ($CDCl_3$) δ: 2.15 (3H, s), 2.16 (3H, s), 5.14 (pH, s), 5.16 (2H, s), 7.42 (1H, s), 7.61 (1H, s).

2) Synthesis of (2-bromo-5-chloro-4-hydroxymethylphenyl)methanol

To a solution of (4-acetoxymethyl-2-bromo-5-chloro)benzyl acetate (28.6 g, 85.2 mmol) in a mixture of THF (250 mL), ethanol (250 mL) and water (125 mL) was added potassium hydroxide (14.3 g, 256 mmol), and the resultant mixture was stirred for 3 hours at 80˚C. The reaction mixture was cooled to room temperature and then solvent was removed by distillation under reduced pressure. To the resulting residue were added water (200 mL) and ethyl acetate (100 mL), and this mixture was stirred for 1 hour at room temperature.
Undissolved material was collected by filtration and dried, to thereby obtain the titled compound (20.7 g, 96.6%).
$^1$H-NMR ($CD_3OD$) δ: 4.61 (2H, s), 4.66 (2H, s), 7.52 (1H, s), 7.70 (1H, s).

3) Synthesis of 1-bromo-4-chloro-2,5-bis[(1-methoxy-1-methyl)ethoxymethyl]benzene

Under a nitrogen atmosphere, to a solution (500 mL) of (2-bromo-5-chloro-4-hydroxymethylphenyl)methanol (20.7 g, 82.3 mmol) in anhydrous THF were added 2-methoxypropene (78.8 mL, 823.1 mmol) and pyridinium p-toluenesulfonate (207 mg, 0.823 mmol) at 0˚C, and the resultant mixture was stirred for 2 hours. Aqueous potassium carbonate (1 M, 200 mL) was added thereto, and then the resultant mixture was extracted with ethyl acetate (800 mL) containing triethylamine (2.5 mL). The organic layer was successively washed with water (500 mL) and saturated brine (500 mL), and then dried over anhydrous magnesium sulfate. The solvent was then removed by distillation under reduced pressure, to thereby obtain the titled compound (33.2 g, 100%).
$^1$H-NMR ($CDCl_3$) δ: 1.45 (12H, s), 3.22 (6H, s), 4.48 (2H, s), 4.53 (2H, s), 7.51 (1H, s), 7.68 (1H, s).

4) Synthesis of (1S,3'R,4'S,5'S,6'R)-5-chloro-3',4',5',6'-tetrahydro-6,6'-bis(hydroxymethyl)-spiro[isobenzofuran-1(3H), 2'-[2H]pyran]-3',4',5'-triol

N-butyllithium (1.6 M n-hexane solution, 39.1 mL, 62.53 mmol) was added dropwise over 5 minutes at -78˚C under a nitrogen atmosphere to a solution (500 mL) of 1-bromo-4-chloro-2,5-bis[(1-methoxy-1-methyl)ethoxymethyl] benzene (24.7 g, 62.53 mmol) in anhydrous THF, and the resultant mixture was stirred under the same condition for 20 minutes. A solution of 2,3,4,6-tetrakis-O-(trimethylsilyl)-D-glucono-1,5-lactone (26.54 g, 56.85 mmol) in THF (40 mL) was then added dropwise over 5 minutes to the resultant mixture. The reaction mixture was stirred for 1 hour, and then water was added thereto. The resultant mixture was extracted with diethyl ether. The resultant organic layer was then successively washed with water and saturated brine, and then dried over anhydrous magnesium sulfate. The solvent was then removed by distillation under reduced pressure. The resulting residue (46.97 g) was dissolved in a mixed solvent of THF (94 mL) and MeOH (47 mL), and p-toluenesulfonic acid (2.16 g) was added thereto. The mixture was stirred at room temperature for 15 hours, and then cooled with ice. MTBE (188 mL) was added thereto, and the precipitate was collected by filtration. The obtained solid was dried under reduced pressure, to thereby obtain the titled compound (12.7 g, 67.1%).
$^1$H-NMR ($CD_3OD$) δ: 3.44-3.50 (1H, m), 3.63-3.84 (5H, m), 4.71 (2H, s), 5.11 (2H, dd, 12.3, 19.1 Hz), 7.33 (1H, s), 7.55 (1H, s).

5) Synthesis of (1S,3'R,4'S,5'S,6'R)-3',4',5'-tri(acetoxy)-6'-acetoxymethyl-5-chloro-6-chloromethyl-3',4',5',6'-tetrahydro-spiro[isobenzofuran-1(3H),2'-[2H]pyran]

To a solution of (1S,3'R,4'S,5'S,6'R)-5-chloro-3',4',5',6'-tetrahydro-6,6'-bis(hydroxymethyl)-spiro[isobenzofuran-1(3H),2'-[2H]pyran]-3',4',5'-triol (1.0 g, 3.0 mmol) in DMSO (1.7 mL, 24.0 mmol) was added chlorotrimethylsilane (1.1 mL, 8.4 mmol) at room temperature, and the resultant mixture was stirred under this consdition for 4 hours. The reaction mixture was concentrated under reduced pressure, and the resulting residue was dried under reduced pressure for 15 hours. To a solution (20 mL) of the resulting residue in THF were added N-methylmorpholine (3.7 mL, 30 mmol) and 4-(dimethylamino)pyridine (385 mg, 3.15 mmol) at 0˚C, and acetic anhydride (1.7 mL, 18 mmol) was then added dropwise to the resultant mixture. The reaction mixture was stirred under this condition for 30 minutes, and then stirred for another 30 minutes at room temperature. Excess aqueous phosphoric acid was added thereto, and the resultant mixture was extracted with ethyl acetate. The organic layer was successively washed with water and saturated brine, and then dried over anhydrous magnesium sulfate. The solvent was then removed by distillation under reduced pressure. The resulting residue was purified by silica gel column chromatography (developing solvent = ethyl acetate:n-hexane (1:1)), to thereby obtain the titled compound (840 mg, 53.9%).
$^1$H-NMR ($CDCl_3$) δ: 1.77 (3H, s), 2.01 (3H, s), 2.05 (3H, s), 2.08 (3H, s), 4.01-4.06 (1H m), 4.25-4.33 (2H, m), 4.70 (2H, d, J=2.3 Hz), 5.17 (2H, dd, J=21.0, 13.4 Hz), 5.25-5.31 (1H, m), 5.55-5.64 (2H, m), 7.32 (1H, s), 7.51 (1H, s).

6) Synthesis of (1S,3'R,4'S,5'S,6'R)-3',4',5'-tri(acetoxy)-6'-acetoxymethyl-5-chloro-6-[(4-ethylphenyl)methyl]-3',4',5',6'-tetrahydrospiro[isobenzofuran-1(3H),2'-[2H]pyran]

To a solution of (1S,3'R,4'S,5'S,6'R)-3',4',5'-tri(acetoxy)-6'-acetoxymethyl-5-chloro-6-chloromethyl-3',4',5',6'-tetrahydro-spiro[isobenzofuran-1(3H),2'-[2H]pyran] (300 mg, 0.58 mmol) in DMF (2.85 mL) and water (0.15 mL) were added 4-ethylphenylboronic acid (174 mg, 1.16 mmol), tetrakis(triphenylphosphine)palladium (34 mg, 0.029 mmol), sodium carbonate (184 mg, 1.74 mmol) and tetrabutylammonium bromide (39 mg, 0.116 mmol), and the resultant mixture was stirred for 15 hours at 85°C. The reaction mixture was cooled to room temperature, and then water (10 mL) was added thereto. The resultant mixture was extracted with ethyl acetate (100 mL). The organic layer was successively washed with water and saturated brine, and then dried over anhydrous magnesium sulfate. The solvent was then removed by distillation under reduced pressure. The resulting residue was purified by silica gel column chromatography (developing solvent = ethyl acetate:n-hexane (1:3)), to thereby obtain the titled compound (236 mg, 69.1%).
$^1$H-NMR ($CDCl_3$) δ: 1.22 (3H, t, J=7.6 Hz), 1.75 (3H, s), 2.00 (3H, s), 2.04 (3H, s), 2.06 (3H, s), 2.62 (2H, q, J=7.6 Hz), 4.01-4.13 (3H, m), 4.22-4.33 (2H, m), 5.13 (2H, dd, J=12.2, 13.0 Hz), 5.26 (1H, dd, J=8.8, 9.5 Hz), 5.52-5.63 (2H, m), 7.10 (4H, dd, J=8.4, 13.7 Hz), 7.26 (2H, s).

7) Synthesis of (1S,3'R,4'S,5'S,6'R)-5-chloro-6-[(4-ethylphenyl)methyl]-3',4',5',6'-tetrahydro-6'-(hydroxymethyl)-spiro [isobenzofuran-1(3H),2'-[2H]pyran]-3',4',5'-triol

To a solution of (1S,3'R,4'S,5'S,6'R)-3',4',5'-tri(acetoxy)-6'-acetoxymethyl-5-chloro-6-[(4-ethylphenyl)methyl]-3',4',5',6'-tetrahydrospiro[isobenzofuran-1(3H),2'-[2H]pyran] (236 mg, 0.40 mmol) in methanol (16 mL) was added potassium carbonate (166 mg, 1.20 mmol), and the resultant mixture was stirred for 1 hour at room temperature. Water (5 mL) was added thereto, and the resultant mixture was extracted with ethyl acetate (50 mL). The organic layer was washed with saturated brine, and then dried over anhydrous magnesium sulfate. The solvent was then removed by distillation under reduced pressure. The resulting residue was purified by silica gel column chromatography (developing solvent = dichloromethane:methanol (15:1)), to thereby obtain the titled compound (110 mg, 65.3%).
$^1$H-NMR ($CD_3OD$) δ: 1.20 (3H, t, J=7.6 Hz), 2.59 (2H, q, J=7.6 Hz), 3.39-3.45 (1H, m), 3.62-3.82 (5H, m), 4.09 (2H, d, J=3.4 Hz), 5.10 (2H, dd, J=13.0, 20.6 Hz), 7.10 (4H, s), 7.25 (1H, s), 7.35 (1H, s).
MS (ESI$^+$): 443 $[M+Na]^+$, 863 $[2M+Na]^+$.

Reference Example 1

Synthesis of 4-(2-fluoroethyl)phenylboronic acid

[Formula 25]

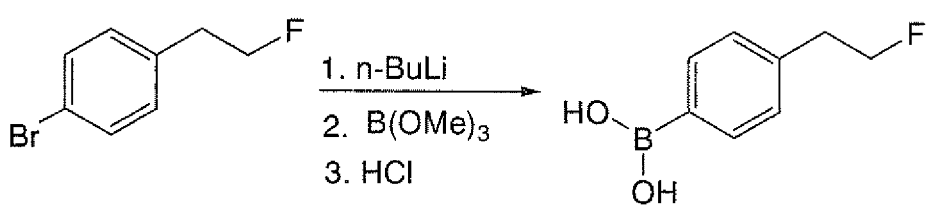


Under a nitrogen stream, to a solution of 1-bromo-4-(2-fluoroethyl)-benzene (Tetrahedron: Asymmetry, 1993, 4(10), page 2183) (412 mg, 2.03 mmol) in THF (9 mL) was added a solution of n-butyllithium in n-hexane (2.71 M, 0.87 mL, 2.36 mmol) at -78°C, and the resultant mixture was stirred at the same temperature for 0.5 hours. Trimethoxyborane (0.36 mL, 3.21 mmol) was added thereto, and the resultant mixture was stirred at room temperature for 4.5 hours. Then, to the solution was added 20% hydrochloric acid, and the resultant mixture was extracted 3 times with methylene chloride. The organic layer was concentrated under reduced pressure. The resulting residue was purified by silica gel flash column chromatography (developing solvent = ethyl acetate:n-hexane (1:1)), to thereby obtain the titled compound (216 mg, 63%).
$^1$H-NMR ($CDCl_3$) δ: 2.99-3.18 (2H, m), 4.55-4.80 (2H, m), 7.28-8.19 (4H, m).
The compounds listed in Tables 1-1 to 1-3 can be easily produced in the same manner as described in Example

1 or in the production processes, or by making slight modifications to such processes that would be obvious to a person skilled in the art.

[Table 1-1]

Table 1-1

| | | |
|---|---|---|
| Example 2 | 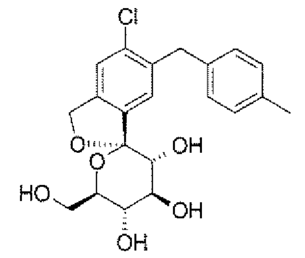 | $^1$H-NMR ($CD_3OD$) δ: 2.28 (3H, s), 3.42 (1H, dd, J=8.4, 9.9 Hz), 3.61-3.82 (5H, m), 4.07 (2H, d, J=3.1 Hz), 5.09 (2H, dd, J=13.0, 20.2 Hz), 7.07 (4H, s), 7.23 (1H, s), 7.35 (1H, s)<br>MS (ESI$^+$): 429 [M+Na]$^+$ |
| Example 3 | 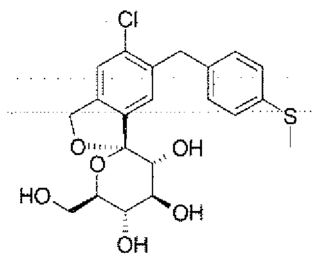 | $^1$H-NMR ($CD_3OD$) δ: 2.44 (3H, s), 3.40 (1H, m), 3.61-3.82 (5H, m), 4.10 (2H, s), 5.10 (2H, dd, J=11.8, 20.6 Hz), 7.15 (4H, dd, 8.8, 14.9 Hz), 7.26 (1H, s), 7.36 (1H, s)<br>MS (ESI$^+$): 461 [M+Na]$^+$ |
| Example 4 | 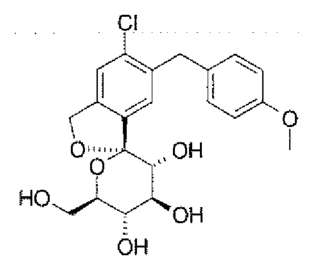 | $^1$H-NMR ($CD_3OD$) δ: 3.38-3.48 (1H, m), 3.60-3.85 (8H, m), 3.98-4.13 (2H, m), 5.02-5.17 (2H, m), 6.79-6.87 (2H, m), 7.06-7.16 (2H, m), 7.23 (1H, s), 7.35 (1H, s)<br>MS (ESI$^+$): 445 [M+Na]$^+$ |
| Example 5 | 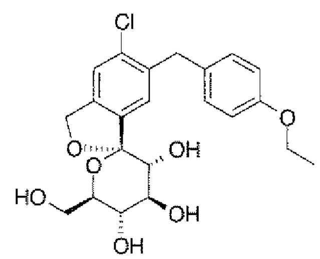 | $^1$H-NMR ($CD_3OD$) δ: 1.36 (3H, t, J=7.1 Hz), 3.38-3.47 (1H, m), 3.58-3.81 (5H, m), 3.94-4.07 (4H, m), 6.76-6.84 (2H, m), 7.05-7.14 (2H, m), 7.23 (1H, s), 7.35 (1H, s)<br>MS (ESI$^+$): 436 [M+Na]$^+$ |
| Example 6 | 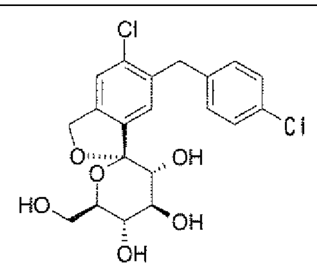 | $^1$H-NMR ($CD_3OD$) δ: 3.39-3.47 (1H, m), 3.61-3.82 (5H, m), 4.12 (2H, s), 5.11 (2H, dd, J=13.0, 7.0 Hz), 7.15-7.30 (5H, m), 7.37 (1H, s)<br>MS (ESI$^+$): 449 [M+Na]$^+$ |

[Table 1-2]

Table 1-2

| | | |
|---|---|---|
| Example 7 | 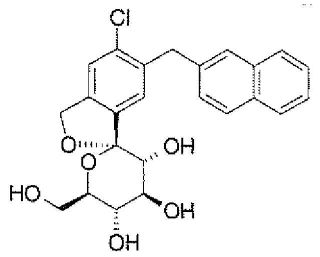 | $^1$H-NMR ($CD_3OD$) δ: 3.41 (1H, dd, J=8.0, 10.3 Hz), 3.61-3.82 (5H, m), 4.31 (2H, s), 5.12 (2H, dd, J=13.0, 19.8 Hz), 7.32 (1H, s), 7.36-7.45 (4H, m), 7.62 (1H, s), 7.74-7.81 (3H, m)<br>MS (ESI$^+$): 465 $[M+Na]^+$ |
| Example 8 | 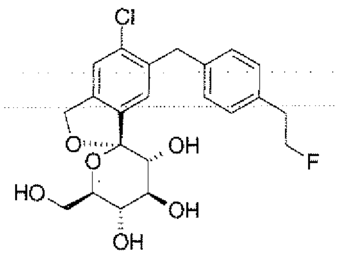 | $^1$H-NMR ($CD_3OD$) δ: 2.88-3.00 (2H, m), 3.40-3.46 (1H, m), 3.62-3.82 (5H, m), 4.05-4.16 (2H, m), 4.46-4.66 (2H, m), 5.04-5.15 (2H, m), 7.11-7.14 (4H, m), 7.26 (1H, s), 7.35 (1H, s)<br>MS (ESI$^+$): 461 $[M+23]^+$ |
| Example 9 | 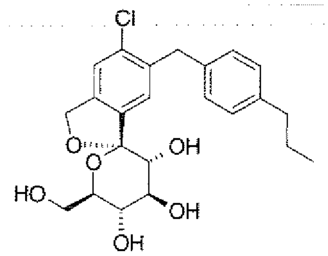 | $^1$H-NMR ($CD_3OD$) δ: 0.92 (3H, t, J=7.2 Hz), 1.60 (2H, dt, J=7.2 Hz, 7.6 Hz), 2.53 (2H, t, J=7.6 Hz), 3.37-3.48 (1H, m), 3.57-3.88 (5H, m), 3.99-4.17 (2H, m), 5.00-5.18 (2H, m), 7.03-7.15 (4H, m), 7.25 (1H, s), 7.35 (1H, s)<br>MS (ESI$^+$): 435 $[M+1]^+$ |
| Example 10 | 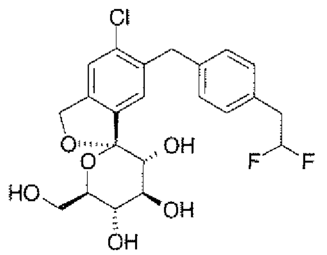 | $^1$H-NMR ($CD_3OD$) δ: 3.09 (2H, td, J=4.6 Hz, J=17.9 Hz), 3.38-3.49 (1H, m), 3.61-3.84 (5H, m), 4.03-4.19 (2H, m), 5.04-5.16 (2H, m), 5.96 (1H, tt, J=4.6 Hz, J=56.9 Hz), 7.18 (4H, s), 7.29 (1H, s), 7.36 (1H, s)<br>MS (ESI$^+$): 457 $[M+1]^+$ |
| Example 11 | 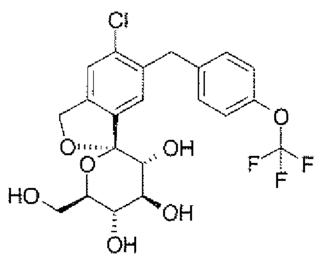 | $^1$H-NMR ($CD_3OD$) δ: 3.39-3.50 (1H, m), 3.60-3.86 (5H, m), 4.17 (2H, s), 5.08 (1H, d, J=13.0 Hz), 5.14 (1H, d, J=13.0 Hz), 7.12-7.20 (2H, m), 7.25-7.41 (4H, m)<br>MS (ESI$^+$): 499 $[M+Na]^+$ |

[Table 1-3]

Table 1-3

| | | |
|---|---|---|
| Example 12 | 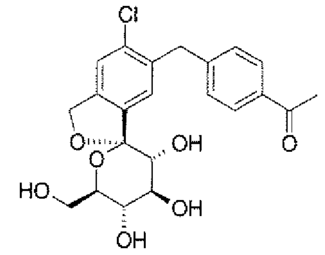 | $^1$H-NMR ($CD_3OD$) δ: 2.57 (3H, s), 3.40-3.47 (1H, m), 3.59-3.83 (5H, m), 4.16-4.27 (2H, m), 5.11 (2H, dd, J=13.0 Hz, 6.9 Hz), 7.30-7.40 (4H, m), 7.89-7.93 (2H, m)<br>MS (ESI$^+$): 457 [M+Na]$^+$ |
| Example 13 | 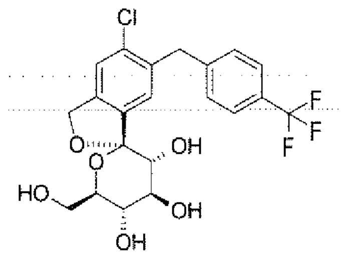 | $^1$H-NMR ($CD_3OD$) δ: 3.39-3.49 (1H, m), 3.61-3.85 (5H, m), 4,23 (2H, s), 5.08 (1H, d, J=13.0 Hz), 5.15 (1H, d, J=13.0 Hz), 7.31-7.44 (4H, m), 7.52-7.60 (2H, m)<br>MS (ESI$^+$): 483 [M+Na]$^+$ |
| Example 14 | 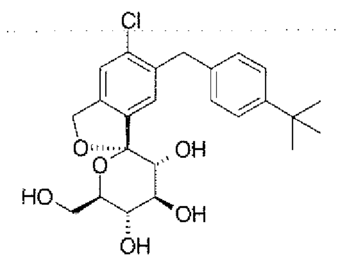 | $^1$H-NMR ($CD_3OD$) δ: 1.29 (9H, s), 3.39-3.46 (1H, m), 3.61-3.83 (5H, m), 4.09 (2H, d, J=3.8 Hz), 5.10 (2H, dd, J=13.1, 7.2 Hz), 7.10-7.14 (2H, m), 7.27-7.37 (4H, m)<br>MS (ESI$^+$): 471 [M+Na] |
| Example 15 | 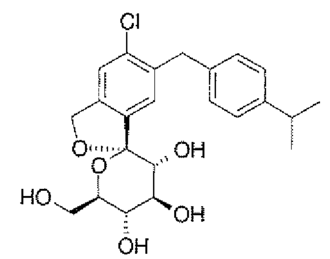 | $^1$H-NMR ($CD_3OD$) δ: 1.21 (3H, s), 1.23 (3H, s), 2.80-2.89 (1H, m), 3.39-3.45 (1H, m), 3.61-3.83 (5H, m), 4.09 (2H, d, J=3.4 Hz), 5.10 (2H, dd, J=13.4, 7.0 Hz), 7.12 (4H, s), 7.27 (1H, s), 7.36 (1H, s)<br>MS (ESI$^+$): 457 [M+Na]$^+$ |

Example 16

(1S,3'R,4'S,5'S,6'R)-6-[(4-ethylphenyl)methyl]-5-fluoro-3',4',5',6'-tetrahydro-6'-(hydroxymethyl)-spiro[isobenzofuran-1(3H),2'-[2H]pyran]-3',4',5'-triol

[Formula 26]

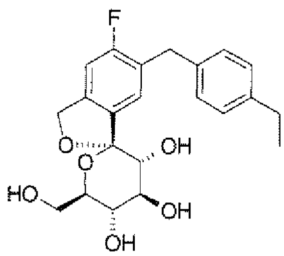

1) Synthesis of 5-bromo-2-fluoro-4-(hydroxymethyl)benzaldehyde

Tetramethylpiperidine (0.68 g, 4.87 mmol) was dissolved in tetrahydofuran (4.5 mL). To the resultant solution was added n-butyllithium (1.0 M n-hexane solution, 4.88 mL) at 0˚C, and this solution was stirred for 15 minutes. The resultant mixture was cooled to -78˚C and a solution of (2-promo-5-fluorophenyl)methanol (0.50 g, 2.43 mmol) in tetrahydrofuran (2.5 mL) was added dropwise thereto. The temperature of the solution was raised over 2 hours to -40˚C. The solution was again cooled to -78˚C, and then dimethylformamide (0.47 mL, 6.07 mmol) was added thereto. The temperature of the solution was raised to room temperature, and the solution was stirred for 30 minutes. Saturated aqueous ammonium chloride was then added thereto, and the resultant mixture was extracted with ethyl acetate. The organic layer was dried over anhydrous sodium sulfate, and then concentrated under reduced pressure, to thereby obtain the titled compound (604.3 mg, quantitative).
$^1$H-NMR ($CDCl_3$) δ: 4.78 (2H, s), 7.46 (1H, d, J=10.6 Hz), 8.01 (1H, d, J=6.2 Hz), 10.29 (1H, s).

2) Synthesis of [2-bromo-5-fluoro-4-(hydroxymethyl)phenyl]methanol

5-Bromo-2-fluoro-4-(hydroxymethyl)benzaldehyde (604.3 mg, 2.59 mmol) was dissolved in methanol (5 mL). To the resultant solution was added sodium borohydride (98.1 mg, 2.59 mmol) at 0˚C. After stirring for 10 minutes, about 3 mL of methanol was removed by distillation. Water was added thereto, and the resultant mixture was extracted with ethyl acetate. The organic layer was concentrated under reduced pressure. The resulting residue was purified by silica gel flash column chromatography (developing solvent = methanol:dichloromethane (3:100)), to thereby obtain the titled compound (247.3 mg, 43%).
$^1$H-NMR ($CD_3OD$) δ: 4.61 (2H, s), 4.64 (2H, s), 7.28 (1H, d, J=11.0 Hz), 7.64 (1H, d, J=7.0 Hz).

3) Synthesis of 1-bromo-4-fluoro-2,5-bis[(1-methoxy-1-methylethoxy)methyl]benzene

[2-Bromo-5-fluoro-4-(hydroxymethyl)phenyl]methanol (71.3 mg, 0.303 mmol) was dissolved in tetrahydrofuran (1 mL). To the resultant solution was added 2-methoxypropene (214.7 mg, 2.97 mmol). The resultant mixture was cooled to 0˚C, and then p-toluenesulfonic acid (1.0 mg, 0.0029 mmol) was added thereto. This solution was stirred for 40 minutes, and then saturated aqueous sodium hydrogen carbonate was added thereto. The resultant mixture was extracted with ethyl acetate. The organic layer was dried over anhydrous sodium sulfate, and then concentrated under reduced pressure, to thereby obtain the titled compound (111.7 mg, 97%).
$^1$H-NMR ($CDCl_3$) δ: 1.43 (6H, s), 1.45 (6H, s), 3.22 (3H, s), 3.24 (3H, s), 4.48 (2H, s), 4.50 (2H, s), 7.26-7.28 (1H, m), 7.59 (1H, d, J=6.6 Hz).

4) Synthesis of (1S,3'R,4'S,5'S,6'R)-3',4',5'-tris(benzyloxy)-6'-(benzyloxymethyl)-5-fluoro-3',4',5',5'-tetrahydro-6-(hydroxymethyl)-spiro[isobenzofuran-1(3H),2'-[2H]pyran]

1-Bromo-4-fluoro-2,5-bis[(1-methoxy-1-methylethoxy)methyl]benzene (340.5 mg, 0.850 mmol) was dissolved in tetrahydrofuran (2.5 mL), and the resultant solution was cooled to -78˚C. N-butyllithium (1.0 M n-hexane solution, 1.02 mL) was added dropwise to the solution, which was then stirred for 30 minutes. (3R,4S,5R,6R)-3,4,5-tris(benzyloxy)-6-(benzyloxymethyl)-tetrahydropyran-2-one (0.642 g, 1.191 mmol) dissolved in tetrahydrofuran (1.0 mL) was added dropwise to the solution, which was then stirred for 50 minutes. Saturated aqueous ammonium chloride was added thereto at -78˚C, and the resultant mixture was extracted with ethyl acetate. The organic layer was dried over anhydrous sodium sulfate, and then concentrated under reduced pressure, to thereby obtain a crude product (0.968 g).
The obtained crude product (0.968 g) was dissolved in a mixed solvent of methanol (1.0 mL) and tetrahydrofuran (1.5 mL). To the resultant solution was added p-toluenesulfonic acid hydrate (29.3 mg, 0.170 mmol). This solution was stirred for 3 hours at room temperature, and then saturated aqueous sodium hydrogen carbonate was added thereto.

The resultant mixture was extracted with ethyl acetate. The organic layer was dried over anhydrous sodium sulfate, and then concentrated under reduced pressure. The resulting residue was purified by silica gel flash column chromatography (developing solvent = ethyl acetate:n-hexane (15:100 to 1:4)), to thereby obtain a stereoisomeric mixture of the titled compound (0.29 g).

The obtained stereoisomeric mixture (0.29 g) was again dissolved in a mixed solvent of methanol (0.59 mL) and tetrahydrofuran (0.86 mL), and p-toluenesulfonic acid hydrate (14.7 mg, 0.013 mmol) was added thereto. The resultant mixture was stirred under reflux for 2.5 hours, and then saturated aqueous sodium hydrogen carbonate was added thereto. The resultant mixture was extracted with ethyl acetate. The organic layer was dried over anhydrous sodium sulfate, and then concentrated under reduced pressure, to thereby obtain the titled compound (0.29 g, 50%).

$^1$H-NMR ($CDCl_3$) δ: 3.64 (1H, d, J=9.5 Hz), 3.78 (1H, d, J=11.0, 3.0 Hz), 3.81-3.88 (2H, m), 4.06 (1H, d, J=8.1 Hz), 4.11-4.17 (1H, m), 4.25 (1H, d, J=11.7 Hz), 4.45 (1H, d, J=12.1 Hz), 4.57 (1H, d, J=12.1 Hz), 4.61-4.68 (4H, m), 4.88 (1H, d, J=11.0 Hz), 4.90-4.96 (2H, m), 5.16 (2H, s), 6.81 (2H, d, J=7.0 Hz), 6.93 (1H, d, J=9.5 Hz), 7.11-7.20 (6H, d, J=6.6 Hz), 7.26-7.34 (13H, m).

5) Synthesis of (1S,3'R,4'S,5'S,6'R)-3',4',5'-tris(benzyloxy)-6'-(benzyloxymethyl)-6-(chloromethyl)-5-fluoro-3',4',5',6'-tetrahydro-spiro[isobenzofuran-1(3H),2'-[2H]pyran]

(1S,3'R,4'S,5'S,6'R)-3',4',5'-tris(benzyloxy)-6'-(benzyloxymethyl)-5-fluoro-3',4',5',6'-tetrahydro-6-(hydroxymethyl)-spiro[isobenzofuran-1(3H),2'-[2H]pyran] (0.29 g, 0.43 mmol) was dissolved in dichloromethane (4.0 mL), and to the resultant solution were added carbon tetrachloride (0.33 g, 2.15 mmol) and triphenylphosphine (0.56 g, 2.15 mmol). The resultant mixture was then stirred for 1.5 hours at room temperature. Dichloromethane was removed by distillation (about 1.5 mL), and then the resulting residue was purified by silica gel flash column chromatography (developing solvent = ethyl acetate:n-hexane (17:100)), to thereby obtain the titled compound (218.1 mg, 70%).

$^1$H-NMR ($CDCl_3$) δ: 3.64 (1H, dd, J=11.2, 1.6 Hz), 3.77-3.86 (3H, m), 4.06 (1H, dd, J=10.3, 1.1 Hz), 4.10-4.15 (1H, m), 4.20 (1H, d, J=11.4 Hz), 4.45-4.64 (6H, m), 4.87-4.96 (3H, m), 5.14 (2H, s), 6.82 (2H, d, J=6.6 Hz), 6.96 (1H, d, J=9.2 Hz), 7.11-7.34 (19H, m).

6) Synthesis of (1S,3'R,4'S,5'S,6'R)-3',4',5'-tris(benzyloxy)-6'-(benzyloxymethyl)-6-[(4-ethylphenyl)methyl]-5-fluoro-3',4',5',6'-tetrahydro-spiro[isobenzofuran-1(3H),2'-[2H]pyran]

(1S,3'R,4'S,5'S,6'R)-3',4',5'-tris(benzyloxy)-6'-(benzyloxymethyl)-6-(chloromethyl)-5-fluoro-3',4',5',6'-tetrahydro-spiro[isobenzofuran-1(3H),2'-[2H]pyran] (0.19 g, 0.273 mmol), sodium carbonate (86.9 mg, 0.819 mmol), tetrakis triphenylphosphine palladium (15.7 mg, 0.013 mmol), tetrabutylammonium bromide (17.6 mg, 0.054 mmol) and 4-ethylphenylboronic acid (81.9 mg, 0.546 mmol) were dissolved in a mixed solvent of dimethylformamide (1.3 mL) and water (0.07 mL). Using a microwave radiation reaction apparatus, the mixture was heated to 140˚C and then stirred for 20 minutes. Water was added thereto, and the resultant mixture was extracted with ethyl acetate. The organic layer was dried over anhydrous sodium sulfate, and then concentrated under reduced pressure. The resulting residue was purified by silica gel flash column chromatography (developing solvent = ethyl acetate:n-hexane (15:100)), to thereby obtain the titled compound (143.8 mg, 68%).

$^1$H-NMR ($CDCl_3$) δ: 1.15 (3H, t, J=7.7 Hz), 2.54 (2H, q, J=7.7 Hz), 3.64 (1H, d, J=9.5 Hz), 3.78-4.15 (8H, m), 4.44-4.51 (2H, m), 4.55-4.63 (2H, m), 4.86-4.92 (3H, m), 5.10 (1H, d, J=13.0 Hz), 5.15 (1H, d, J=13.0 Hz), 6.75 (2H, s), 6.94 (1H, d, J=8.8 Hz), 6.98-7.20 (10H, m), 7.26-7.30 (13H, m).

7) Synthesis of (1S,3'R,4'S,5'S,6'R)-6-[(4-ethylphenyl)methyl]-5-fluoro-3',4',5',6'-tetrahydro-6'-(hydroxymethyl)-spiro[isobenzofuran-1(3H),2'-[2H]pyran]-3',4',5'-triol

(1S,3'R,4'S,5'S,6'R)-3',4',5'-tris(benzyloxy)-6'-(benzyloxymethyl)-6-[(4-ethylphenyl)methyl]-5-fluoro-3',4',5',6'-tetrahydro-spiro[isobenzofuran-1(3H),2'-[2H]pyran] (143.8 mg, 0.187 mmol) was dissolved in a mixed solvent of ethyl acetate (4.5 mL) and methanol (4.5 mL). To the resultant solution were added 10% palladium-carbon (30.0 mg) and 1 N hydrochloric acid (1 drop), and then the mixture was stirred for 2 hours under hydrogen. The resultant mixture was filtered and then the filtrate was concentrated under reduced pressure. The resulting residue was purified by silica gel flash column chromatography (developing solvent = methanol:dichloromethane (1:10)), to thereby obtain the titled compound (56.6 mg, 74%).

$^1$H-NMR ($CD_3OD$) δ: 1.20 (3H, t, J=7.6 Hz), 2.59 (2H, q, J=7.6 Hz), 3.43 (1H, t, J=9.3 Hz), 3.64-3.78 (5H, m), 3.93 (1H, d, J=14.8 Hz), 3.99 (1H, d, J=14.8 Hz), 5.06 (1H, d, J=12.8 Hz), 5.12 (1H, d, J=12.8 Hz), 7.02 (1H, d, J=9.5 Hz), 7.09-7.14 (4H, m), 7.22 (1H, d, J=6.6 Hz).

MS ($ESI^+$): 405 $[M+1]^+$.

The compounds listed in Table 1-4 can be easily produced in the same manner as described in Example 16

or in the production processes, or by making slight modifications to such processes that would be obvious to a person skilled in the art.

[Table 1-4]

Table 1-4

| | | |
|---|---|---|
| Example 17 | 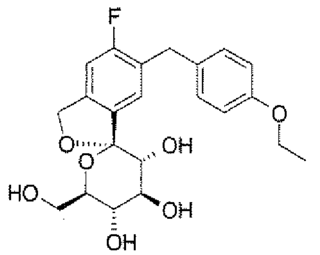 | $^1$H-NMR ($CDCl_3$) δ: 1.37 (3H, t, J=7.0 Hz), 3.73-3.99 (10H, m), 5.00 (1H, d, J=13.0 Hz), 5.10 (1H, d, J=13.0 Hz), 6.79 (2H, d, J=8.1 Hz), 6.90 (1H, d, J=9.2 Hz), 7.08-7.10 (3H, m)<br>MS ($ESI^+$): 421 $[M+1]^+$ |
| Example 18 | 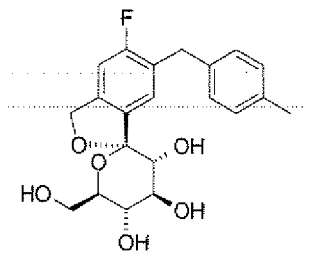 | $^1$H-NMR ($CDCl_3$) δ: 2.32 (3H, s), 3.69-3.91 (6H, m), 3.97 (2H, s), 5.06 (1H, d, J=13.2 Hz), 5.17 (1H, d, J=13.2 Hz), 6.96 (1H, d, J=9.2 Hz), 7.09-7.11 (5H, m)<br>MS ($ESI^+$): 390 $[M]^+$ |
| Example 19 | 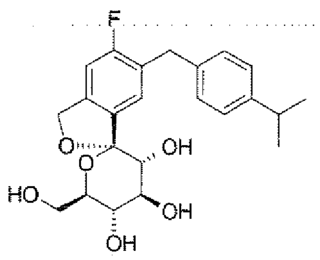 | $^1$H-NMR ($CD_3OD$) δ: 1.21 (6H, d, J=7.0 Hz), 2.81-2.88 (1H, m), 3.41-3.46 (1H, m), 3.63-3.67 (1H, m), 3.70-3.81 (4H, m), 3.96 (2H, dd, J=14.6, 23.0 Hz), 5.06 (1H, d, J=12.4 Hz), 5.12 (1H, d, J=12.4 Hz), 7.02 (1H, d, J=9.5 Hz), 7.13 (4H, s), 7.23 (1H, d, J=6.6 Hz)<br>MS ($ESI^+$): 418 $[M]^+$ |
| Example 20 | 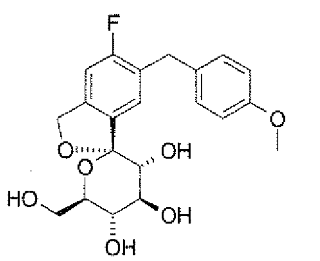 | $^1$H-NMR ($CDCl_3$) δ: 3.76 (3H, s), 3.79-3.91 (8H, m), 5.02 (1H, d, J=12.8 Hz), 5.13 (1H, d, J=12.8 Hz), 6.81 (2H, d, J=8.4 Hz), 6.92 (1H, d, J=9.2 Hz), 7.10-7.12 (3H, m)<br>MS ($ESI^+$): 407 $[M+1]^+$ |

Example 21

(1S,3'R,4'S,5'S,6'R)-3',4',5',6'-tetrahydro-6'-(hydroxymethyl)-6-[(4-isopropylphenyl)methyl]-5-methyl-spiro[isobenzofuran-1(3H),2'-[2H]pyran]-3',4',5'-triol

[Formula 27]

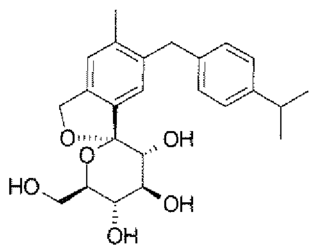

1) Synthesis of (4-acetoxymethyl-2-bromo-5-methyl)benzyl acetate

To a solution of 1-bromo-2,5-bis(chloromethyl)-4-methylbenzene (29.2 g, 108.96 mmol) in DMF (150 mL) was added sodium acetate (26.8 g, 326.89 mmol), and the resultant mixture was stirred for 3 hours at 80˚C. The reaction mixture was cooled to room temperature and then water was added thereto. The resultant mixture was extracted with dichloromethane. The organic layer was successively washed with water and saturated brine, and then dried over anhydrous magnesium sulfate. The solvent was then removed by distillation under reduced pressure. To the resulting residue were added ethyl acetate (70 mL) and n-hexane (100 mL). This solution was stirred for 15 hours at room temperature. Undissolved material was collected by filtration, to thereby obtain the titled compound (23.4 g, 67.9%).
$^1$H-NMR ($CDCl_3$) δ: 2.12 (3H, s), 2.14 (3H, s), 2.30 (3H, s), 5.07 (2H, s), 5.15 (2H, s), 7.22 (1H, s), 7.54 (1H, s).

2) Synthesis of (2-bromo-5-methyl-4-hydroxymethylphenyl)methanol

To a solution of (4-acetoxymethyl-2-bromo-5-methyl)benzyl acetate (23.4 g, 74.0 mmol) in a mixture of THF (200 mL), ethanol (200 mL) and water (100 mL) was added potassium hydroxide (12.5 g, 222.0 mmol), and the resultant mixture was stirred for 5 hours at 80˚C. The reaction mixture was cooled to room temperature and then the solvent was removed by distillation under reduced pressure. To the resulting residue were added water (200 mL) and ethyl acetate (100 mL), and the mixture was stirred for 1 hour at room temperature. Undissolved material was collected by filtration and dried, to thereby obtain the titled compound (15.1 g, 88.3%).
$^1$H-NMR ($CD_3OD$) δ: 2.28 (3H, s), 4.58 (2H, s), 4.61 (2H, s), 7.31 (1H, s), 7.53 (1H, s).

3) Synthesis of 1-bromo-4-methyl-2,5-bis[(1-methoxy-1-methyl)ethoxymethyl]benzene

Under a nitrogen atmosphere, to a solution (500 mL) of (2-bromo-5-methyl-4-hydroxymethylphenyl)methanol (18.0 g, 77.9 mmol) in anhydrous THF were added 2-methoxypropene (74.6 mL, 779.0 mmol) and pyridinium p-toluenesulfonate (196 mg, 0.78 mmol) at 0˚C, and the resultant mixture was stirred for 30 minutes. Aqueous potassium carbonate (1 M, 200 mL) was added thereto, and then the resultant mixture was extracted with ethyl acetate (800 mL) containing triethylamine (2.5 mL). The organic layer was successively washed with water (500 mL) and saturated brine (500 mL), and then dried over anhydrous magnesium sulfate. The solvent was then removed by distillation under reduced pressure, to thereby obtain the titled compound (29.2 g, 100%).
$^1$H-NMR ($CDCl_3$) δ: 1.44 (6H, s), 1.45 (6H, s), 2.27 (3H, s), 3.23 (3H, s), 3.25 (3H, s), 4.42 (2H, s), 4.50 (pH, s), 7.29 (1H, s), 7.57 (1H, s).

4) Synthesis of (1S,3'R,4'S,5'S,6'R)-3',4',5',6'-tetrahydro-6,6'-bis(hydroxymethyl)-5-methyl-spiro[isobenzofuran-1(3H), 2'-[2H]pyran]-3',4',5'-triol

N-butyllithium (2.5 M n-hexane solution, 31.4 mL, 78.43 mmol) was added dropwise over 5 minutes at -78˚C under a nitrogen atmosphere to a solution (500 mL) of 1-bromo-4-methyl-2,5-bis[(1-methoxy-1-methyl)ethoxymethyl] benzene (26.8 g, 71.3 mmol) in anhydrous THF, and the resultant mixture was stirred under the same condition for 30 minutes. A solution of 2,3,4,6-O-tetrabenxyl-D-glucono-1,5-lactone (38.4 g, 71.3 mmol) in THF (40 mL) was then added dropwise over 5 minutes to the mixture. The reaction mixture was stirred for 1 hour, and then saturated aqueous ammonium chloride was added thereto. The resultant mixture was extracted with ethyl acetate. The resultant organic layer was then successively washed with water and saturated brine, and then dried over anhydrous magnesium sulfate. The solvent was then removed by distillation under reduced pressure. The resulting residue was dissolved in a mixed solvent of THF (130 mL) and MeOH (65 mL), and p-toluenesulfonic acid (2.71 g) was added thereto. The reaction mixture was stirred at room temperature for 2 hours, and then ethyl acetate was added thereto. The resultant organic layer was then successively washed with water and saturated brine. The organic layer was dried over anhydrous magnesium

sulfate, and then the solvent was removed by distillation under reduced pressure. The resulting residue was purified by silica gel column chromatography (developing solvent = ethyl acetate:n-hexane (1:4)), to thereby obtain the titled compound (8.9 g, 18.9%).

$^1$H-NMR ($CDCl_3$) δ: 1.34 (1H-OH, brs), 2.36 (3H, s), 3.63-3.92 (4H, m), 4.06-4.24 (2H, m), 4.42-4.65 (7H, m), 4.87-4.96 (3H, m), 5.17 (2H, s), 6.78 (2H, d, J=6.9 Hz), 7.07-7.37 (20H, m).

5) Synthesis of (1S,3'R,4'S,5'S,6'R)-3',4',5'-tris(benzyloxy)-6'-benzyloxymethyl-6-(chloromethyl)-3',4',5',6'-tetrahydro-5-methyl-spiro[isobenzofuran-1(3H),2'-[2H]pyran]

To a solution of (1S,3'R,4'S,5'S,6'R)-3',4',5'-tris(benzyloxy)-6'-benzyloxymethyl-3',4',5',6'-tetrahydro-6-(hydroxymethyl)-5-methyl-spiro[isobenzofuran-1(3H),2'-[2H]pyran] (8.9 g, 13.2 mmol) in dichloromethane (300 mL) was added thionyl chloride (2.2 mL, 30.4 mmol) at 0˚C, and the resultant mixture was stirred at the same temperature for 1 hour. 10% Aqueous sodium hydrogen carbonate was added thereto followed by conducting liquid-liquid separation. The organic layer was washed with saturated brine, dried over anhydrous sodium sulfate and concentrated under reduced pressure. The resulting residue was purified by silica gel flash column chromatography (developing solvent = ethyl acetate:n-hexane (1:4)), to thereby obtain the titled compound (5.88 g, 64.4%).

$^1$H-NMR ($CDCl_3$) δ: 2.46 (3H, s), 3.64 (1H, dd, J=1.9, 11.1 Hz), 3.77-3.90 (3H, m), 4.04-4.19 (2H, m), 4.44-4.65 (7H, m), 4.87-4.97 (3H, m), 5.15 (2H, s), 6.77-6.82 (2H, m), 7.07-7.36 (20H, m).

6) Synthesis of (1S,3'R,4'S,5'S,6'R)-3',4',5'-tris(benzyloxy)-6'-(benzyloxymethyl)-3',4',5',6'-tetrahydro-6-[(4-isopropylphenyl)methyl]-5-methyl-spiro[isobenzofuran-1(3H),2'-[2H]pyran]

To a solution of (1S,3'R,4'S,5'S,6'R)-3',4',5'-tris(benzyloxy)-6'-(benzyloxymethyl)-6-(chloromethyl)-3',4',5',6'-tetrahydro-5-methyl-spiro[isobenzofuran-1(3H),2'-[2H]pyran] (300 mg, 0.43 mmol) in a mixture of DMF (2.85 mL) and water (0.15 mL) were added 4-isopropylphenylboronic acid (142 mg, 0.87 mmol), tetrakis triphenylphosphine palladium (25 mg, 0.022 mmol), sodium carbonate (138 mg, 1.3 mmol) and tetrabutylammonium bromide (29 mg, 0.087 mmol), and the resultant mixture was stirred for 15 hours at 85˚C. The reaction mixture was cooled to room temperature, and then water (10 mL) was added thereto. The resultant mixture was extracted with ethyl acetate (100 mL). The organic layer was washed with saturated brine, dried over anhydrous sodium sulfate and concentrated under reduced pressure. The resulting residue was purified by silica gel flash column chromatography (developing solvent = ethyl acetate:n-hexane (1:8)), to thereby obtain the titled compound (160 mg, 47.6%).

$^1$H-NMR ($CDCl_3$) δ: 1.15 (3H, s), 1.17 (3H, s), 2.28 (3H, s), 2.74-2.89 (1H, m), 3.62-3.68 (1H, m), 3.77-3.96 (5H, m), 4.06-4.15 (3H, m), 4.43-4.65 (4H, m), 4.84-4.94 (3H, m), 5.11-5.21 (2H, m), 6.72-7.31 (26H, m).

7) Synthesis of (1S,3'R,4'S,5'S,6'R)-3',4',5',6'-tetrahydro-6'-(hydroxymethyl)-6-[(4-isopropylphenyl)methyl]-5-methyl-spiro[isobenzofuran-1(3H),2'-[2H]pyran]-3',4',5'-triol

To a solution of (1S,3'R,4'S,5'S,6'R)-3',4',5'-tris(benzyloxy)-6'-(benzyloxymethyl)-3',4',5',6'-tetrahydro-6-[(4-isopropylphenyl)methyl]-5-methyl-spiro[isobenzofuran-1(3H),2'-[2H]pyran] (160 mg, 0.21 mmol) in a mixture of ethyl acetate (0.5 mL) and methanol (2 mL) were added 10% palladium-carbon (100 mg) and 1 N hydrochloric acid (1 drop), and then the mixture was stirred for 13 hours under hydrogen. The resultant mixture was filtered and then the filtrate was concentrated under reduced pressure. The resulting residue was purified by silica gel flash column chromatography (developing solvent = methanol:dichloromethane (1:10)), to thereby obtain the titled compound (10.3 mg, 12.1%).

$^1$H-NMR ($CD_3OD$) δ: 1.18-1.24 (6H, m), 2.23 (3H, s), 2.79-2.88 (1H, m), 3.40-3.47 (1H, m), 3.63-3.85 (5H, m), 3.98 (2H, s), 5.09 (2H, dd, J=12.2 Hz, 10.0 Hz), 7.02-7.15 (6H, m).

MS ($ESI^+$): 437 $[M+Na]^+$.

The compounds listed in Table 1-5 can be easily produced in the same manner as described in Example 21 or in the production processes, or by making slight modifications to such processes that would be obvious to a person skilled in the art.

[Table 1-5]

Table 1-5

| | | |
|---|---|---|
| Example 22 | 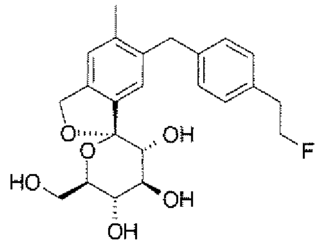 | $^1$H-NMR ($CD_3OD$) δ: 2.22 (3H, s), 2.87-2.99 (2H, m), 3.40-3.47 (1H, m), 3.66 (1H, dd, J=5.76, 12.08 Hz), 3.72-3.83 (4H, m), 4.00 (2H, s), 4.47 (1H, t, J=6.59), 4.63 (1H, t, J=6.59), 5.09 (2H, dd, J=12.35, 22.23 Hz), 7.04-7.14 (6H, m)<br>MS ($ESI^+$): 419 $[M+1]^+$ |
| Example 23 | 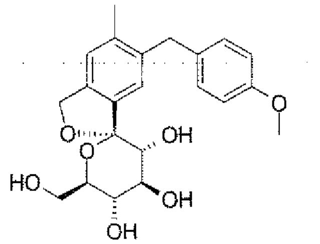 | $^1$H-NMR ($CD_3OD$) δ: 2.23 (3H, s), 3.39-3.47 (1H, m), 3.62-3.84 (8H, m), 3.95 (2H, s), 5.09 (2H, dd, J=12.7 Hz, 9.5 Hz), 6.80 (2H, d, J=8.3 Hz), 7.00-7.12 (4H, m)<br>MS ($ESI^+$); 425 $[M+Na]^+$ |
| Example 24 | 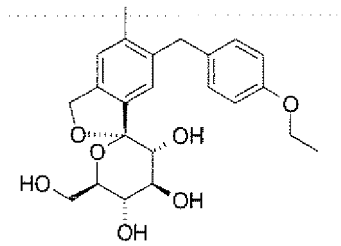 | $^1$H-NMR ($CD_3OD$) δ: 1.35 (3H, t, J=6.9 Hz), 2.23 (3H, s), 3.38-3.49 (1H, m), 3.61-3.85 (5H, m), 3.91-4.04 (4H, m), 5.02-5.18 (2H, m), 6.74-6.83 (2H, m), 6.96-7.15 (4H, m)<br>MS ($ESI^+$): 439 $[M+Na]^+$ |
| Example 25 | 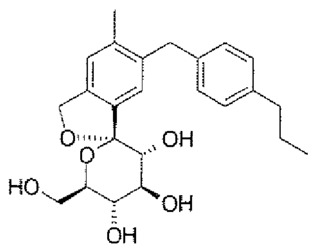 | $^1$H-NMR ($CD_3OD$) δ: 0.91 (3H, t, J=7.3 Hz), 1.56-1.64 (2H, m), 2.23 (3H, s), 2.53 (2H, t, J=7.74 Hz), 3.39-3.47 (1H, m), 3.62-3.85 (5H, m), 3.98 (2H, s), 5.09 (2H, dd, J=12.5 Hz, 9.9 Hz), 7.00-7.14 (6H, m)<br>MS ($ESI^+$): 415 $[M+1]^+$ |
| Example 26 | 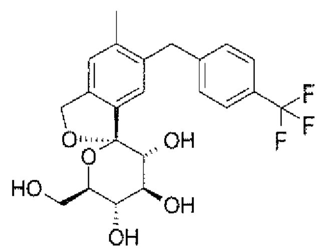 | $^1$H-NMR ($CD_3OD$) δ: 2.23 (3H, s), 3.39-3.47 (1H, m), 3.62-3.85 (5H, m), 4.13 (2H, s), 5.11 (2H, dd, J=12.7 Hz, 8.7 Hz), 7.13-7.19 (2H, m), 7.30-7.35 (2H, m), 7.53-7.57 (2H, m)<br>MS ($ESI^+$): 463 $[M+Na]^+$ |

The compounds listed in Table 1-6 can be easily produced in the same manner as described in Example 1 or 21, or in the production processes, or by making slight modifications to such processes that would be obvious to a person skilled in the art.

[Table 1-6]

Table 1-6

| | | |
|---|---|---|
| Example 27 | 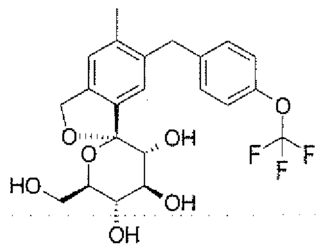 | $^1$H-NMR ($CD_3OD$) δ: 2.23 (3H, s), 3.42-3.48 (1H, m), 3.64-3.85 (5H, m), 4.07 (2H, s), 5.11 (2H, dd, J=12.2 Hz, 9.2 Hz), 7.12-7.24 (6H, m)<br>MS (ESI$^+$): 479 [M+Na]$^+$ |
| Example 28 | 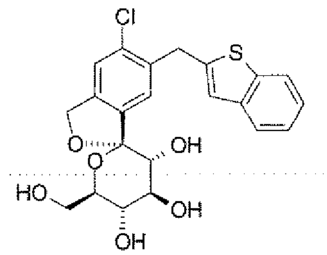 | $^1$H-NMR ($CD_3OD$) δ: 3.37-3.49 (1H, m), 3.60-3.86 (5H, m), 4.31-4.46 (2H, m), 5.04-5.20 (2H, m), 7.04 (1H, s), 7.18-7.34 (2H, m), 7.37-7.47 (2H, m), 7.60-7.79 (2H, m)<br>MS (ESI$^+$): 449 [M+1]$^+$ |
| Example 29 | 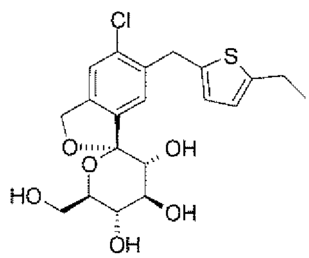 | $^1$H-NMR ($CD_3OD$) δ: 1.24 (3H, t, J=7.6 Hz), 2.75 (2H, q, J=7.6 Hz), 3.39-3.47 (1H, m), 3.62-3.83 (5H, m), 4.20-4.25 (2H, m), 5.10 (2H, dd, J=12.3, 7.9 Hz), 6.56-6.61 (2H, m), 7.31-7.38 (2H, m)<br>MS (ESI$^+$): 449 [M+Na]$^+$ |
| Example 30 | 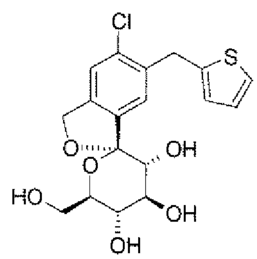 | $^1$H-NMR ($CD_3OD$) δ: 3.38-3.49 (1H, m), 3.61-3.84 (5H, m), 4.22-4.38 (2H, m), 5.03-5.17 (2H, m), 6.80-6.86 (1H, m), 6.87-6.93 (1H, m), 7.16-7.23 (1H, m), 7.33 (1H, s), 7.37 (1H, s,<br>MS (ESI$^+$): 421 [M+Na]$^+$ |

Example 31

(1S,3'R,4-S,5'S,6'R)-6-[(4-ethylphenyl)methyl]-5-ethynyl-3',4',5',6'-tetrahydro-6'-(hydroxymethyl)-spiro[isobenzofuran-1(3H),2'-[2H]pyran]-3',4',5'-triol

[Formula 28]

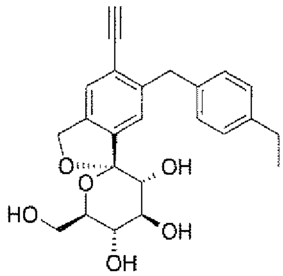

1) Synthesis of (1S,3'R,4'S,5'S,6'R)-3',4',5'-triacetoxy-6'-(acetoxymethyl)-6-[(4-ethylphenyl)methyl]-3',4',5',6'-tetrahydro-5-trimethylsilylethynyl-spiro[isobenzofuran-1(3H),2'-[2H]pyran]

Under a nitrogen stream, a solution of (1S,3'R,4'S,5'S,6'R)-3',4',5'-tri(acetoxy)-6'-acetoxymethyl-5-chloro-6-[(4-ethylphenyl)methyl]-3',4',5',6'-tetrahydro-spiro[isobenzofuran-1(3H),2'-[2H]pyran] (97 mg, 0.165 mmol), dichlorobis(acetonitrile)palladium(II) (9 mg, 0.035 mmol), 2-dicyclohexylphosphino-2',4',6'-triisopropylbiphenyl (53 mg, 0.111 mmol) and cesium carbonate (141 mg, 0.433 mmol) in acetonitrile (3.35 mL) was stirred for 0.5 hours at room temperature. To the mixture was then added trimethylsilylacetylene (0.26 mL, 1.84 mmol), and the resultant mixture was stirred for 2 hours at 90°C. The reaction mixture was concentrated under reduced pressure, and the resulting residue was purified by silica gel flash column chromatography (developing solvent = ethyl acetate:n-hexane (1:2)), to thereby obtain the titled compound (96 mg, 81%).

$^1$H-NMR ($CDCl_3$) δ: 0.24 (9H, s), 1.22 (3H, t, J=7.5 Hz), 1.71 (3H, s), 2.00 (3H, s), 2.05 (3H, s), 2.06 (3H, s), 2.58-2.66 (2H, m), 4.02-4.33 (5H, m), 5.05-5.30 (3H, m), 5.53-5.62 (2H, m), 7.08-7.16 (4H, m), 7.23 (1H, s), 7.34 (1H, s).

2) Synthesis of (1S,3'R,4'S,5'S,6'R)-6-[(4-ethylphenyl)methyl]-5-ethynyl-3',4',5',6'-tetrahydro-6'-(hydroxymethyl)-spiro[isobenzofuran-1(3H),2'-[2H]pyran]-3',4',5'-triol

To a solution of (1S,3'R,4'S,5'S,6'R)-3',4',5'-triacetoxy-6'-(acetoxymethyl)-6-[(4-ethylphenyl)methyl]-3',4',5',6'-tetrahydro-5-trimethylsilylethynyl-spiro[isobenzofuran-1(3H),2'-[2H]pyran] (101 mg, 0.155 mmol) in methanol (1 mL) was added potassium carbonate (14 mg), and the resultant mixture was stirred for 1.5 hours at room temperature. The solvent was then removed by distillation under reduced pressure. The resulting residue was purified by silica gel column chromatography (developing solvent = methylene chloride:methanol (85:15)), to thereby obtain the titled compound (23 mg, 36%).

$^1$H-NMR ($CD_3OD$) δ: 1.19 (3H, t, J=7.69 Hz), 2.58 (2H, q, J=7.41, 7.69 Hz), 3.38-3.45 (1H, m), 3.60-3.82 (6H, m), 4.15 (2H, dd, J=14.82 Hz, 23.06 Hz), 5.03-5.14 (2H, m), 7.1 (4H, dd, J=8.23 Hz, 20.58 Hz), 7.19 (1H, s), 7.41 (1H, s).

MS (ESI$^+$): 411 $[M+1]^+$.

Example 32

(1S,3'R,4'S,5'S,6'R)-5-chloro-6-[(4-(ethynylphenyl)methyl]-3',4',5',6'-tetrahydro-6'-(hydroxymethyl)-spiro[isobenzofuran-1(3H),2'-[2H]pyran]-3',4',5'-triol

[Formula 29]

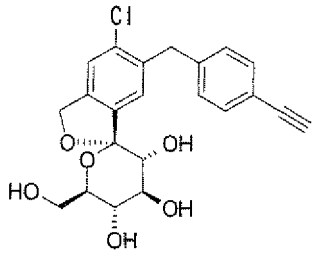

1) Synthesis of 4-(tert-butyldimethylsilyloxy)phenylboronic acid

A solution of 4-hydroxyphenylboronic acid (1 g, 7.25 mmol), tert-butyldimethylsilyl chloride (3.28 g, 21.76 mmol) and imidazole (2.47 g, 36.3 mmol) in DMF was stirred overnight at room temperature. The reaction mixture was diluted with ethyl acetate, washed with saturated sodium hydrogen carbonate solution, then dried over anhydrous sodium sulfate, and concentrated under reduced pressure. The resulting residue was purified by silica gel flash column chromatography (developing solvent = ethyl acetate:n-hexane (1:1)), to thereby obtain the titled compound (1.1 g, 60.2%).

$^1$H-NMR ($CD_3OD$) δ: 0.21 (6H, s), 0.99 (9H, s), 6.83 (2H, d, J=8.4 Hz), 7.54 (2H, d, J=8.4 Hz).

2) Synthesis of (1S,3'R,4'S,5'S,6'R)-3',4',5'-triacetoxy-6'-(acetoxymethyl)-6-[(4-(tert-butyldimethylsilyloxy)phenyl)methyl]-5-chloro-3',4',5',6'-tetrahydro-spiro[isobenzofuran-1(3H),2'-[2H]pyran]

A reaction between (1S,3'R,4'S,5'S,6'R)-3',4',5'-tri(acetoxy)-6'-acetoxymethyl-5-chloro-6-chloromethyl-3',4',5',6'-tetrahydro-spiro[isobenzofuran-1(3H),2'-[2H]pyran] and 4-(tert-butyldimethylsilyloxy)phenylboronic acid was conducted in the same manner as that in step 6 of Example 1, to thereby obtain the titled compound with a yield of 15.5%.

$^1$H-NMR ($CDCl_3$) δ: 0.18 (6H, s), 0.97 (9H, s), 1.74 (3H, s), 2.00 (3H, s), 2.05 (3H, s), 2.06 (3H, s), 3.92-4.16 (3H, m), 4.19-4.36 (2H, m), 5.10 (1H, d, J=12.6 Hz), 5.18 (1H, d, J=12.6 Hz), 5.22-5.32 (1H, m), 5.48-5.65 (2H, m), 6.73-6.82 (2H, m), 6.98-7.09 (2H, m), 7.21-7.30 (2H, m).

3) Synthesis of (1S,3'R,4'S,5'S.6'R)-3',4',5'-triacetoxy-6'-(acetoxymethyl)-5-chloro-3',4',5',6'-tetrahydro-6-[(4-hydroxyphenyl)methyl]-spiro[isobenzofuran-1(3H),2'-[2H]pyran]

A solution of 1.0 M tetrabutylammoium fluoride in THF (0.12 mL, 0.12 mmol) was added dropwise under a nitrogen atmosphere and under ice cooling to a solution of (1S,3'R,4'S,5'S,6'R)-3',4',5'-triacetoxy-6'-(acetoxymethyl)-6-[(4-(tert-butyldimethylsilyloxy)phenyl)methyl]-5-chloro-3',4',5',6'-tetrahydro-spiro[isobenzofuran-1(3H),2'-[2H]pyran] (62 mg, 0.090 mmol) in anhydrous THF (2 mL). The reaction mixture was stirred under the same condition for 15 minutes, and then diluted with water. The resultant mixture was extracted with ethyl acetate. The organic layer was dried over anhydrous sodium sulfate, and then concentrated under reduced pressure. The resulting residue was purified by silica gel flash column chromatography (developing solvent = ethyl acetate:n-hexane (1:1)), to thereby obtain the titled compound (44 mg, 84.9%).

$^1$H-NMR ($CDCl_3$) δ: 1.75 (3H, s), 2.00 (3H, s), 2.05 (3H, s), 2.06 (3H, s), 3.92-4.16 (3H, m), 4.19-4.35 (2H, m), 4.71 (1H, s), 5.10 (1H, d, J=13.0 Hz), 5.18 (1H, d, J=13.0 Hz), 5.21-5.31 (1H, m), 5.48-5.65 (2H, m), 6.73-6.82 (2H, m), 7.01-7.09 (2H, m), 7.19-7.30 (2H, m).

4) Synthesis of (1S,3'R,4'S,5'S,6'R)-3',4',5'-triacetoxy-6'-(acetoxymethyl)-5-chloro-3',4',5',6'-tetrahydro-6-[(4-(trifluoromethanesulfonyloxy)phenyl)methyl]-spiro[isobenzofuran-1(3H),2'-[2H]pyran]

A solution of (1S,3'R,4'S,5'S,6'R)-3',4',5'-triacetoxy-6'-(acetoxymethyl)-5-chloro-3',4',5',6'-tetrahydro-6-[(4-hydroxyphenyl)methyl]-spiro[isobenzofuran-1(3H),2'-[2H]pyran] (42.4 mg, 0.073 mmol) in anhydrous dichloromethane (2 mL) was cooled with ice, and pyridine (17.8 μL, 0.220 mmol) was added dropwise under a nitrogen atmosphere to the solution. Then, trifluoromethanesulfonic anhydride (18.5 μL, 0.110 mmol) was added dropwise to the solution. The reaction mixture was stirred under the same condition for 2 hours, and the resultant mixture was then purified by silica gel flash column chromatography (developing solvent = ethyl acetate:n-hexane (1:1)), to thereby obtain the titled compound (49 mg, 94.0%).

$^1$H-NMR ($CDCl_3$) δ: 1.74 (3H, s), 2.01 (3H, s), 2.05 (3H, s), 2.06 (3H, s), 3.97-4.37 (5H, m), 5.11 (1H, d, J=13.0 Hz), 5.19 (1H, d, J=13.0 Hz), 5.22-5.32 (1H, m), 5.50-5.66 (2H, m), 7.14-7.33 (6H, m).

5) Synthesis of (1S,3'R,4'S,5'S,5'R)-3',4',5'-triacetoxy-6'-(acetoxymethyl)-5-chloro-3',4',5',6'-tetrahydro-6-[(4-(2-trimethylsilylethynyl)phenyl)methyl]-spiro[isobenzofuran-1(3H),2'-[2H]pyran]

A suspension of (1S,3'R,4'S,5'S,6'R)-3',4',5'-triacetoxy-6'-(acetoxymethyl)-5-chloro-3',4',5',6'-tetrahydro-6-[(4-(trifluoromethanesulfonyloxy)phenyl)methyl]-spiro[isobenzofuran-1(3H),2'-[2H]pyran] (49 mg, 0.069 mmol), trimethylsilylacetylene (19.5 μL, 0.141 mmol), copper(I) iodide (2.63 mg, 0.014 mmol), triethylamine (0.05 mL, 0.358 μmol) and dichlorobis(triphenylphosphine)palladium (3.99 mg, 3.45 μmol) in DMF (1 mL) was stirred under a nitrogen atmosphere for 2 hours at 90°C. The reaction mixture was then diluted with water, and the resultant mixture was extracted with ethyl acetate. The organic layer was dried over anhydrous sodium sulfate, and then concentrated under reduced pressure. The resulting residue was purified by silica gel flash column chromatography (developing solvent = ethyl acetate:n-hexane (1:2)), to thereby obtain the titled compound (13 mg, 28.6%).

$^1$H-NMR ($CDCl_3$) δ: 0.23 (9H, s), 1.75 (3H, s), 2.00 (3H, s), 2.05 (3H, s), 2.06 (3H, s), 3.97-4.17 (3H, m), 4.19-4.35 (2H, m), 5.10 (1H, d, J=13.0 Hz), 5.18 (1H, d, J=13.0 Hz), 5.21-5.31 (1H, m), 5.47-5.66 (2H, m), 7.06-7.16 (2H, m), 7.19-7.31 (2H, m), 7.36-7.45 (2H, m).

6) Synthesis of (1S,3'R,4'S,5'S,6'R)-5-chloro-6-[(4-(ethynylphenyl)methyl]-3',4',5',6'-tetrahydro-6'-(hydroxymethyl)-spiro[isobenzofuran-1(3H),2'-[2H]pyran]-3',4',5'-triol

The titled compound was produced in the same manner as that in the final step of Example 1 by using (1S,3'R,4'S,5'S,6'R)-3',4',5'-triacetoxy-6'-(acetoxymethyl)-5-chloro-3',4',5',6'-tetrahydro-6-[(4-(2-trimethylsilylethynyl)phenyl)methyl]-spiro[isobenzofuran-1(3H),2'-[2H]pyran] as a starting material . The yield was 54.4%.

$^1$H-NMR ($CD_3OD$) δ: 3.39-3.50 (2H, m), 3.59-3.85 (5H, m), 4.10-4.20 (2H, m), 5.07 (1H, d, J=13.0 Hz), 5.14 (1H, d, J=13.0 Hz), 7.14-7.46 (6H, m).

MS ($ESI^+$): 439 $[M+Na]^+$.

Example 33

(1S,3'R,4'S,5'S,6'R)-3',4',5',6'-tetrahydro-6'-(hydroxymethyl)-5-methyl-6-[(4-(propyn-1-yl)phenyl)methyl]-spiro[isobenzofuran-1(3H),2'-[2H]pyran]-3',4',5'-triol

[Formula 30]

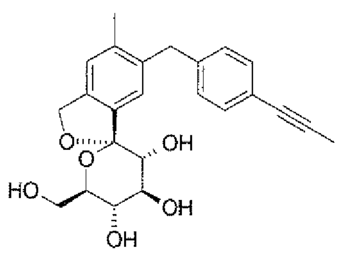

1) Synthesis of (1S,3'R,4'S,5'S,6'R)-3',4',5'-tris(benzyloxy)-6'-(benzyloxymethyl)-6-[(4-formylphenyl)methyl]-3',4',5',6'-tetrahydro-5-methyl-spiro[isobenzofuran-1(3H),2'-[2H]pyran]

A mixture of (1S,3'R,4'S,5'S,6'R)-3',4',5'-tris(benzyloxy)-6'-(benzyloxymethyl)-6-(chloromethyl)-3',4',5',6'-tetrahydro-5-methyl-spiro[isobenzofuran-1(3H),2'-[2H]pyran] (568 mg, 0.822 mmol), 4-formylphenylboronic acid (246 mg, 1.641 mmol), sodium carbonate (261 mg, 2.46 mmol), tetrabutylammonium bromide (53 mg, 0.164 mmol), tetrakis [triphenylphosphine]palladium (47 mg, 0.041 mmol), toluene (2.14 mL) and distilled water (0.21 mL) was stirred under a nitrogen stream for 20 minutes at 150°C (microwave irradiation). The reaction mixture was cooled to room temperature, and then purified by silica gel flash column chromatography (developing solvent = ethyl acetate:n-hexane (1:3)), to thereby obtain the titled compound (255 mg, 41%).

$^1$H-NMR ($CDCl_3$) δ: 2.27 (3H, s), 3.66 (1H, dd, J=1.92, 10.98 Hz), 3.79-3.87 (3H, m), 4.05-4.17 (4H, m), 4.44-4.67 (5H, m), 4.86-4.94 (3H, m), 5.16-5.20 (2H, m), 6.75-6.78 (2H, m), 7.05-7.34 (22H, m), 7.62 (2H, dd, J=1.65, 6.59 Hz), 9.88 (1H, s).

2) Synthesis of (1S,3'R,4'S,5'S,6'R)-3',4',5'-tris(benzyloxy)-6'-(benzyloxymethyl)-6-[(4-ethynylphenyl)methyl]-3',4',5',6'-tetrahydro-5-methyl-spiro[isobenzofuran-1(3H),2'-[2H]pyran]

A mixture of (1S,3'R,4'S,5'S,6'R)-3',4',5'-tris(benzyloxy)-6'-(benzyloxymethyl)-6-[(4-formylphenyl)methyl]-3',4',5',6'-tetrahydro-5-methyl-spiro[isobenzofuran-1(3H),2'-[2H]pyran] (255 mg, 0.335 mmol), potassium carbonate (93 mg, 0.673 mmol), dimethyl(1-diazo-2-oxopropyl)phosphonate (116 mg, 0.604 mmol), methanol (4.2 mL) and THF (0.8 mL) was stirred under a nitrogen stream for 4.5 hours at room temperature. The reaction mixture was concentrated under reduced pressure, and the resulting residue was purified by silica gel flash column chromatography (developing solvent = ethyl acetate:n-hexane (1:3)), to thereby obtain the titled compound (187 mg, 74%).
$^1$H-NMR ($CDCl_3$) δ: 2.25 (3H, s), 3.02 (1H, s), 3.64-3.68 (1H, m), 3.78-4.16 (9H, m), 4.44-4.65 (4H, m), 4.85-4.94 (3H, m), 5.13-5.22 (2H, m), 6.76-6.79 (2H, m), 6.95-7.32 (24H, m).

3) Synthesis of (1S,3'R,4'S,5'S,6'R)-3',4',5'-tris(benzyloxy)-6'-(benzyloxymethyl)-3',4',5',6'-tetrahydro-5-methyl-6-[(4-(propyn-1-yl)phenyl)methyl]-spiro[isobenzofuran-1(3H),2'-[2H]pyran]

Under a nitrogen stream, to a solution of (1S,3'R,4'S,5'S,6'R)-3',4',5'-tris(benzyloxy)-6'-(benzyloxymethyl)-6-[(4-ethylphenyl)methyl]-3',4',5',6'-tetrahydro-5-methyl-spiro[isobenzofuran-1(3H),2'-[2H]pyran] (144 mg, 0.19 mmol) in THF (1.9 mL) was added a solution of n-butyllithium in n-hexane (2.67 M, 78 μL, 0.208 mmol) at -78°C, and the resultant mixture was stirred at the same temperature for 2 hours. Methyl iodide (59 μL, 0.948 mmol) was added thereto, and the resultant mixture was stirred at the same temperature for 0.5 hours. The solution was then further stirred for 0.5 hours at room temperature. Distilled water was added thereto, and the resultant mixture was twice extracted with methylene chloride. The organic layer was concentrated under reduced pressure, and the resulting residue was purified by silica gel flash column chromatography (developing solvent = ethyl acetate:n-hexane (1:4)), to thereby obtain the titled compound (133 mg, 90%).
$^1$H-NMR ($CDCl_3$) δ: 2.03 (3H, s), 2.25 (3H, s), 3.66 (1H, dd, J=1.65, 11.25 Hz), 3.78-4.16 (9H, m), 4.44-4.65 (4H, m), 4.86-4.94 (3H, m), 5.17 (2H, dd, J=1.65, 11.25 Hz), 6.76-6.80 (2H, m), 6.91-7.32 (24H, m).
4) Synthesis of (1S,3'R,4'S,5'S,6'R)-3',4',5',6'-tetrahydro--6'-(hydroxymethyl)-5-methyl-6-[(4-(propyn-1-yl) phenyl)methyl]-spiro[isobenzofuran-1(3H),2'-[2H]pyran]-3',4',5'-triol
Under a nitrogen stream, to a solution (9.4 mL) of (1S,3'R,4'S,5'S,6'R)-3',4',5'-tris(benzyloxy)-6'-(benzyloxymethyl)-3',4',5',6'-tetrahydro-5-methyl-6-[(4-(propyn-1-yl)phenyl)methyl]-spiro[isobenzofuran-1(3H),2'-[2H]pyran] (133 mg, 0.173 mmol) and pentamethylbenzene (258 mg, 1.74 mmol) in methylene chloride was added a solution of boron trichloride in methylene chloride (1.0 M, 1.7 mL, 1.7 mmol) at -78°C, and the resultant mixture was stirred at the same temperature for 2 hours. To the reaction mixture was then added methanol (9.4 mL). The temperature of the solution was raised to room temperature, and then saturated aqueous sodium hydrogen carbonate was added thereto. The resultant mixture was extracted with ethyl acetate. The organic layer was dried over anhydrous potassium carbonate, and then the solvent was removed by distillation under reduced pressure. The resulting residue was purified by silica gel column chromatography (developing solvent = methylene chloride:methanol (9:1)), to thereby obtain the titled compound (14 mg, 20%).
$^1$H-NMR ($CD_3OD$) δ: 1.99 (3H, s), 2.22 (3H, s), 3.41-3.47 (1H, m), 3.66 (1H, dd, J=5.76, 12.08 Hz), 3.71-3.84 (4H, m), 4.00 (2H, s), 5.03-5.15 (2H, m), 7.03-7.23 (6H, m).
MS ($ESI^+$): 411 $[M+1]^+$.

Test Example 1

Evaluation of inhibitory activity against methyl-α-D-glucopyranoside uptake of human $Na^+$-glucose cotransporter (SGLT1 and SGLT2)

1) Construction of human SGLT1 expression vector

Human SGLT1 cDNA was amplified by PCR with a cDNA library derived from human small intestine (Clontech) as a template, synthetic DNA primers and KOD+ DNA Polymerase (Toyobo Co., Ltd., Japan). The amplified cDNA was inserted into pcRII-Topo vector by using a Topo TA Cloning Dual Promoter kit (Invitrogen). *E. coli* competent cells (Invitrogen, TOP10) were transformed with the plasmid vector, cultured in LB medium containing ampicillin (50 mg/L) to grow ampicillin-resistant clones. The plasmid vector containing human SGLT1 cDNA was purified from the clone in a standard manner (see Maniatis et al., Molecular Cloning). Human SGLT1 cDNA added restriction enzyme recognition sites (*Eco RI* at 5' -end, *Hind III* at 3'-end) was amplified by PCR with the plasmid vector as a template, synthetic DNA primers containing an additional restriction enzyme recognition site, and KOD+ DNA Polymerase. This amplified cDNA was digested with Eco RI and Hind III and ligated into expression vector pcDNA3.1(-) (Invitrogen) digested with *Eco RI* and *Hind III* by a Rapid DNA Ligation kit (Roche Diagonostics). *E. coli* competent cells (Invitrogen, DH5α) were transformed with the ligated expression vector and grown in ampicillin-containing LB medium. Human SGLT1 expression vector was purified from the ampicillin-resistant clone in a standard manner.

2) Construction of human SGLT2 expression vector

Human SGLT2 cDNA was amplified by PCR with a cDNA library derived from human kidney (Clontech) as a template, synthetic DNA primers and KOD+ DNA Polymerase. The amplified cDNA was inserted into pcRII-Topo vector by using a Topo TA Cloning Dual Promoter kit. *E. coli* competent cells (TPO10) were transformed with the plasmid vector, cultured in LB medium containing ampicillin (50 mg/L) to grow ampicillin-resistant clones. The plasmid vector containing human SGLT2 cDNA was purified from the clone in a standard manner. Human SGLT2 cDNA added restriction enzyme recognition sites (*Xho I* at 5'-end, *Hind III* at 3'-end) was amplified by PCR with the plasmid vector as a template, synthetic DNA primers containing an additional restriction enzyme recognition site and KOD+ DNA Polymerase. This amplified cDNA was digested with *Xho I* and *Hind III*, and ligated into expression vector pcDNA3.1(-) digested with *Xho I* and *Hind III* by using a Rapid DNA Ligation kit. *E. coli* competent cells (DH5α) were transformed with the ligated expression vector and grown in ampicillin-containing LB medium. Human SGLT2 expression vector was purified from the ampicillin-resistant clone in a standard manner.

3) Establishment of cell lines stably expressing human SGLT1 or human SGLT2

The human SGLT1 expression vector or the human SGLT2 expression vector was digested with the restriction enzyme Pvu I and transfected into CHO-K1 cells with FuGENE (Roche Diagonostics). After the transfection, the cells were cultured at 37˚C in the presence of 5% $CO_2$ for about 3 weeks in DMEM medium (Gibco) containing penicillin (50 U/mL, SIGMA), streptomycin (50 mg/L, SIGMA), geneticin (200 mg/L, Nacalai Tesque, Inc., Japan) and 20% fetal bovine serum to obtain geneticin-resistant clones. Among these clones, clones stably expressing human SGLT1 or human SGLT2 were selected by the evaluating the sodium-dependent uptake activity of sugar (methyl-α-D-glucopyranoside).

4) Evaluation of inhibitory activity against methyl-α-D-glucopyranoside uptake

Cell lines stably expressing human SGLT1 or human SGLT2 CHO were seeded in 96-well culture plates at a density of 30000 to 40000 cells/well and cultured for 4 to 6 days. The medium in these plates was removed and replaced by 150 μL/well pretreatment buffer (i.e., a buffer containing 140 mM choline chloride, 2 mM potassium chloride, 1 mM calcium chloride, 1 mM magnesium chloride, 10 mM 2-[4-(2-hydroxyethyl)-1-piperazinyl]ethanesulfonic acid and tris (hydroxymethyl)aminomethane, pH 7.4), and the plates were incubated at 37˚C for 20 minutes. The pretreatment buffer in the plates was removed, replaced by 50 μL/well fresh pretreatment buffer, and the plates were incubated at 37˚C for 20 minutes. Methyl-α-D-(U-$^{14}$C)glucopyranoside (6.3 mL, Amersham Pharmacia Biotech, 200 mCi/L) was added to and mixed with 100 mL buffer (i.e., a buffer containing 140 mM sodium chloride, 2 mM potassium chloride, 1 mM calcium chloride, 1 mM magnesium chloride, 1 mM methyl-α-D-glucopyranoside, 10 mM [4-(2-hydroxyethyl)-1-piperazinyl] ethanesulfonic acid and tris(hydroxymethyl)aminomethane, pH 7.4), which was used as uptake buffer. Test compounds were dissolved into uptake buffer and these test compound solutions were used for evaluating inhibitory activity. Uptake buffer without a test compound was used as a control solution. Moreover, for use in measuring baseline uptake in the absence of sodium, sodium-free solution was prepared in the same manner to contain 140 mM choline chloride instead of sodium chloride. The pretreatment buffer was removed from each well of the plates and replaced by 35 μL/well test compound solutions, control solution or sodium-free solution, and the plates were incubated at 37˚C for 45 minutes. The solutions were removed and replaced by 300 μL/well washing buffer (i.e., a buffer containing 140 mM choline chloride, 2 mM potassium chloride, 1 mM calcium chloride, 1 mM magnesium chloride, 10 mM methyl-α-D-glucopyranoside, 10 mM 2-[4-(2-hydroxyethyl)-1-piperazinyl]ethanesulfonic acid and tris(hydroxymethyl)aminomethane, pH 7.4). The washing buffer was removed immediately. This washing procedure was repeated once again, and a cell lysis solution (1 M sodium hydroxide, 0.1% sodium lauryl sulfate) was added in a volume of 30 μL per well to solubilize the cells. 2 M hydrochloric acid (15 μL) was added to the cell lysate in each well, and 40 μL of the resulting solution was transferred to a LumaPlate (Packard). The LumaPlate were left overnight at room temperature to evaporate the solvent. The samples on the plate were measured for their radioactivity with a TopCount NXT (Packard). Assuming that the value obtained by subtracting the baseline uptake level from the uptake level of the control sample was set to 100%, the concentration required for a test compounds to cause 50% inhibition of the uptake level ($IC_{50}$ value) were calculated from the concentration-dependent inhibition curve using ELfit ver.3. As a result, the compounds of the present invention were found to show a remarkable inhibitory effect on SGLT2. The $IC_{50}$ values for the inhibition of SGLT2 of the representative compounds of the present invention are shown in Tables 2-1 and 2-2.

Among the test compounds, the compound of Example 43 (comparative compound 1), 1,1-anhydro-1-C-[5-(4-ethylphenyl)methyl-2-(hydroxymethyl)-4-methylphenyl]-β-glucopyranose (also referred to as (1S,3'R,4'S,5'S,6'R)-5-methyl-6-[(4-(ethylphenyl)methyl]-3'.4',5',6'-tetrahydro-6'-(hydroxymethyl)-spiro[isobenzofuran-1(3H),2'-[2H]pyran]-3',4',5'-triol), described in the specification of PCT/JP2006/30128, which is invented in advance by the present inventors, was used as a comparative compound.

[Table 2-1]

Table 2-1

| Test compound | $IC_{50}$ value (nM) |
|---|---|
| Example 1 | 1.3 |
| Example 2 | 1.2 |
| Examples 3 | 1.3 |
| Example 4 | 1.0 |
| Example 5 | 1.5 |
| Example 6 | 1.3 |
| Example 7 | 1.7 |
| Example 8 | 1.1 |
| Example 8 | 2.3 |
| Example 10 | 1.2 |
| Example 11 | 1.9 |
| Example 12 | 1.5 |
| Example 13 | 1.7 |
| Example 14 | 3.4 |
| Example 15 | 1.7 |
| Example 16 | 1.5 |
| Example 17 | 2.6 |
| Example 18 | 1.7 |
| Example 19 | 2.0 |
| Example 20 | 2.2 |
| Example 21 | 2.1 |
| Example 22 | 1.1 |
| Example 23 | 1.5 |
| Example 24 | 1.8 |
| Example 25 | 2.3 |
| Example 26 | 2.8 |
| Example 27 | 2.6 |
| Example 28 | 1.4 |
| Example 29 | 1.5 |
| Example 30 | 1.3 |
| Example 31 | 2.1 |

[Table 2-2]

Table 2-2

| Test compound | hhSGLT2 $IC_{50}$ value (nM) | hSGLT1 $IC_{50}$ value (nM) | hSGLT2 selectivity |
|---|---|---|---|
| Comparative Compound 1 | 1.6 | 132 | 83 |
| Example 1 | 1.3 | 201 | 155 |
| Example 5 | 1.5 | 425 | 283 |
| Example 7 | 1.7 | 381 | 224 |
| Example 8 | 1.1 | 355 | 323 |
| Example 9 | 2.3 | 1358 | 590 |
| Example 15 | 1.7 | 1013 | 596 |
| Example 21 | 2.0 | 682 | 341 |

Test Example 2

Study for glucose lowering effect in db/db Mouse A study for glucose lowering effect was conducted using db/db mice (purchased from Clea Japan, Inc., male, 9 to 11 weeks old), type 2 diabetic model mice. On the day of the study, body weights and blood glucose levels were measured prior to the start of testing. Measurement of the blood glucose levels was performed using a simple blood glucose meter (manufactured by Bayer HealthCare, Dexter-Z II). The mice were allocated to some groups consisting of 5 mice each so that homogeneity of body weights or blood glucose levels among groups would be ensured. Blood collection was carried out by making an incision on the tip of the tail and taking the blood therefrom. On the day of the study, the test compounds were suspended in 0.5% carboxymethylcellulose solution (CMC solution), and the dosing solutions of which concentration was adjusted to either 0.3 mg/10 mL or 3 mg/10 mL were prepared. The dosing solutions were orally administered by gavage for mice in 10 mL/kg. To the control group, only a 0.5% CMC solution was orally administered in 10 mL/kg. Blood was collected prior to or sequentially after administration of test compounds by above method. The blood glucose levels were determined using a hexokinase method (AutoSera S GLU, manufactured by Daiichi Pure Chemicals Co., Ltd.). A percent reduction of blood glucose relative to control group at each time point after administration of test compounds was calculated by the following equation.

Percent reduction of blood glucose (%) = {blood glucose level in the control group (mg/dL)-blood glucose level in the test compound group (mg/dL)}/blood glucose level in the control group (mg/dL)×100

Among the test compounds, the compound of Example 4 (comparative compound 2), 1,1-anhydro-1-C-[5-(4-isopropylphenyl)methyl-2-(hydroxymethyl)phenyl]-β-glucopyranose (also referred to as (1S,3'R,4'S,5'S,6'R)-3',4',5',6'-tetrahydro-6'-(hydroxymethyl)-6-[(4-isopropylphenyl)methyl]-spiro[isobenzofuran-1(3H),2'-[2H]pyran]-3',4',5'-triol), described in the specification of PCT/JP2006/301284, which is a prior application by the present inventors, was used as a comparative compound. The results are shown in Tables 3-1 and 3-2.

[Table 3-1]

Table 3-1

| Test compound | Dose (mg/kg) | Percent reduction of blood glucose (%) 6 hours after administration |
|---|---|---|
| Comparative Compound 2 | 0.3 | 16 |
| Example 11 | 0.3 | 45 |
| Example 12 | 0.3 | 27 |
| Example 17 | 0.3 | 33 |

[Table 3-2]

Table 3-2

| Test compound | Dose (mg/kg) | Percent reduction of blood glucose (%) 6 hours after administration | Percent reduction of blood glucose (%) 24 hours after administration |
|---|---|---|---|
| Comparative Compound 2 | 0.3 | 16 | 4.9 |
| Comparative Compound 2 | 3.0 | 53 | 13 |
| Example 1 | 0.3 | 42 | 32 |
| Example 5 | 0.3 | 52 | 25 |
| Example 7 | 0.3 | 54 | 42 |
| Example 8 | 0.3 | 48 | 27 |
| Example 9 | 0.3 | 48 | 28 |
| Example 15 | 0.3 | 50 | 27 |
| Example 21 | 0.3 | 51 | 33 |

It is clear from these results that the compounds of the present invention exhibit 25% or more reduction of blood glucose 6 hours after administration, and thus have a strong hypoglycemic effect. Further, the compounds of the present invention exhibit 25% or more reduction of blood glucose 24 hours after administration, thereby it is revealed that they have a continuous hypoglycemic effect.

Further, as with the compounds of Examples 11, 12 and 17, the compounds of Examples 6, 10, 13, 14, 16, 18 to 20, 22 to 27, 30 and 31 also showed 25% or more reduction of blood glucose 6 hours after administration by 0.3 mg/kg administration.

In addition, as with the compounds in Table 3-2, the compounds of Examples 2 to 4, 28 and 29 also showed 25% or more reduction of blood glucose 24 hours after administration by 0.3 mg/kg administration.

[INDUSTRIAL APPLICABILITY]

The present invention provides spiroketal compounds exhibiting excellent inhibition effect on SGLT2 activity or prodrugs or pharmacologically acceptable salts thereof. The compounds of the present invention are useful as preventive or therapeutic drugs for diabetes, diabetes-related diseases or diabetic complications.

## Claims

**1.** A compound represented by Formula (II):

[Formula 1]

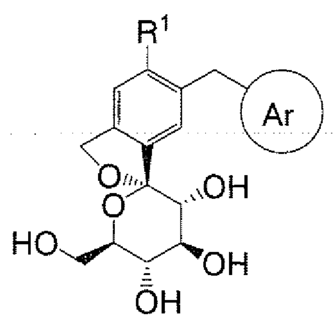

(II)

wherein $R^1$ is a chlorine atom, a fluorine atom, a methyl group or an ethynyl group;

Ar is a group represented by the following Formula (a), Formula (b), Formula (c) or Formula (d):

[Formula 2]

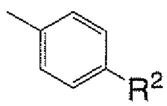

(a)

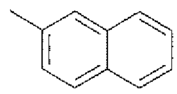

(b)

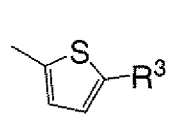

(c)

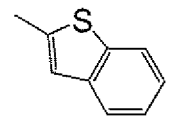

(d)

wherein $R^2$ is a $C_{1-6}$ alkyl group which may be substituted with one or more halogen atoms, a $C_{1-6}$ alkoxy group which may be substituted with one or more halogen atoms, a $C_{1-3}$ alkylthio group, a halogen atom, a $C_{1-3}$ alkylcarbonyl group or a $C_{2-5}$ alkynyl group which may be substituted with $-OR^4$;
$R^3$ is a hydrogen atom or a $C_{1-3}$ alkyl group;
$R^4$ is a hydrogen atom or a $C_{1-3}$ alkyl group;
provided that Ar is a group represented by Formula (a), when $R^1$ is a fluorine atom, a methyl group or an ethynyl group, and
that $R^2$ is a methoxy group, an ethoxy group, an isopropyl group, a propyl group, a trifluoromethyl group, a trifluoromethoxy group, a 2-fluoroethyl group or a 1-propynyl group, when $R^1$ is a methyl group or a pharmaceutically acceptable salt or a solvate thereof.

**2.** The compound according to claim 1 represented by Formula (III):

[Formula 3]

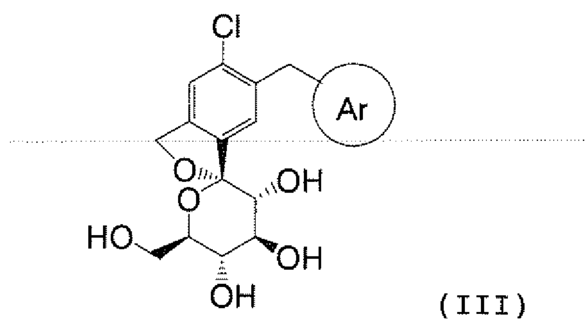

(III)

wherein Ar is a group represented by the following Formula (a), Formula (b), Formula (c) or Formula (d):

[Formula 4]

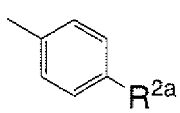

(a)

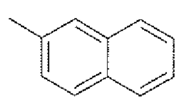

(b)

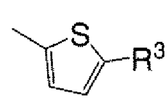

(c)

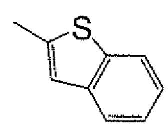

(d)

wherein $R^{2a}$ is a methyl group, an ethyl group, a propyl group, an isopropyl group, a tert-butyl group, a methoxy group, an ethoxy group, a trifluoromethoxy group, a trifluoromethyl group, a 2-fluoroethyl group, a 2,2-difluoroethyl group, a methylthio group, a chloro group, an acetyl group or an ethynyl group,
$R^3$ is a hydrogen atom or an ethyl group or a pharmaceutically acceptable salt or a solvate thereof.

**3.** The compound according to claim 1 represented by Formula (IV):

[Formula 5]

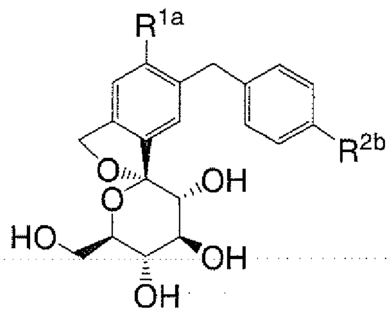

(IV)

wherein $R^{1a}$ is a fluorine atom or an ethynyl group;
$R^{2b}$ is a methyl group, an ethyl group, an isopropyl group, a methoxy group or an ethoxy group
or a pharmaceutically acceptable salt or a solvate thereof.

**4.** The compound according to claim 1 represented by Formula (V):

[Formula 6]

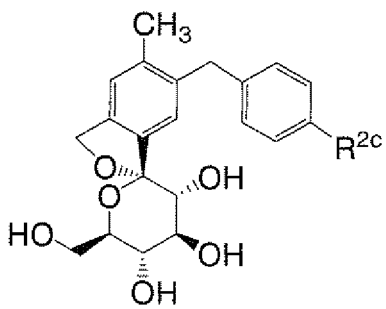

(V)

wherein $R^{2c}$ is a methoxy group, an ethoxy group, a propyl group, an isopropyl group, a trifluoromethyl group, a trifluoromethoxy group, a 2-fluoroethyl group or a 1-propynyl group
or a pharmaceutically acceptable salt or a solvate thereof.

**5.** The compound according to claim 2 represented by Formula (VI):

[Formula 7]

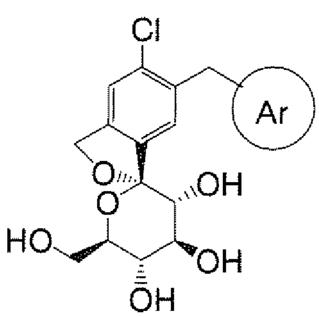

(VI)

wherein Ar is a group represented by the following Formula (a) or Formula (b):

[Formula 4]

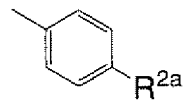

(a)

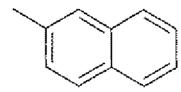

(b)

wherein $R^{2a}$ is an ethyl group, a propyl group, an isopropyl group, an ethoxy group or a 2-fluoroethyl group or a pharmaceutically acceptable salt or a solvate thereof.

**6.** The compound according to claim 4 represented by Formula (VII):

[Formula 8]

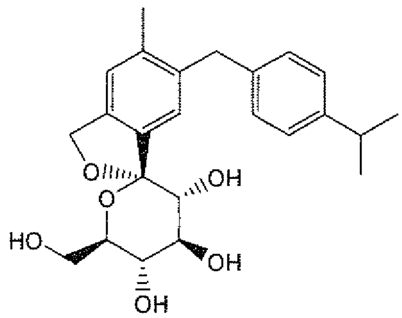

or a pharmaceutically acceptable salt or a solvate thereof.

**7.** The compound according to claim 1 selected from
(1S,3'R,4'S,5'S,6'R)-5-chloro-6-[(4-ethylphenyl)methyl]-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-[(4-methylphenyl)methyl]-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-[(4-(methylthio)phenyl)methyl]-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-[(4-methoxyphenyl)methyl]-spiro[isobenzofuran-1(3H),2'-[2H]pyran]-3',4',5'-triol;
(1S,3'R,4'S,5'S,6'R)-5-chloro-6-[(4-ethoxyphenyl)methyl]-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-6-[(4-chlorophenyl)methyl]-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-(2-naphthylmethyl)-spiro[isobenzofuran-1(3H),2'-[2H]pyran]-3',4',5'-triol;
(1S,3'R,4'S,5'S,6'R)-5-chloro-6-[(4-(2-fluoroethyl)phenyl)methyl]-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-[(4-propylphenyl)methyl]-spiro[isobenzofuran-1(3H),2'-[2H]pyran]-3',4',5'-triol;
(1S,3'R,4'S,5'S,6'R)-5-chloro-6-[(4-(2,2-difluoroethyl)phenyl)methyl]-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-6-[(4-trifluoromethoxyphenyl)methyl]-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-6-[(4-acetylphenyl)methyl]-5-chloro-3',4',5',6'-tetrahydro-6'-(hydroxymethyl)-spiro[isobenzofuran-1(3H),2'-[2H]pyran]-3',4',5'-triol;

(1S,3'R,4'S,5'S,6'R)-5-chloro-6-[(4-trifluoromethylphenyl)methyl]-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-6-[(4-tert-butylphenyl)methyl]-5-chloro-3',4',5',6'-tetrahydro-6'-(hydroxymethyl)-spiro[isobenzofuran-1(3H),2'-[2H]pyran]-3',4',5'-triol;
(1S,3'R,4'S,5'5,6'R)-5-chloro-3',4',5',6'-tetrahydro-6'-(hydroxymethyl)-6-[(4-isopropylphenyl)methyl]-spiro[isobenzofuran-1(3H),2'-[2H]pyran]-3',4',5'-triol;
(1S,3'R,4'S,5'S,6'R)-6-[(4-ethylphenyl)methyl]-5-fluoro-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-6-[(4-ethoxyphenyl)methyl]-5-fluoro-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-fluoro-3',4',5',6'-tetrahydro-6'-(hydroxymethyl)-6-[(4-methylphenyl)methyl]-spiro[isobenzofuran-1(3H),2'-[2H]pyran]-3',4',5'-triol;
(1S,3'R,4'S,5'S,6'R)-5-fluoro-3',4',5',6'-tetrahydro-6'-(hydroxymethyl)-6-[(4-isopropylphenyl)methyl]-spiro[isobenzofuran-1(3H),2'-[2H]pyran]-3',4',5'-triol;
(1S,3'R,4'S,5'S,6'R)-5-fluoro-3',4',5',6'-tetrahydro-6'-(hydroxymethyl)-6-[(4-methoxyphenyl)methyl]-spiro[isobenzofuran-1(3H),2'-[2H]pyran]-3',4',5'-triol;
(1S,3'R,4'S,5'S,6'R)-3',4',5',6'-tetrahydro-6'-(hydroxymethyl)-6-[(4-isopropylphenyl)methyl]-5-methyl-spiro[isobenzofuran-1(3H),2'-[2H]pyran]-3',4',5'-triol;
(1S,3'R,4'S,5'S,6'R)-6-[(4-(2-fluoroethyl)phenyl)methyl]-5-methyl-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-3',4',5',6'-tetrahydro-6'-(hydroxymethyl)-6-[(4-methoxyphenyl)methyl]-5-methyl-spiro[isobenzofuran-1(3H),2'-[2H]pyran]-3',4',5'-triol;
(1S,3'R,4'S,5'S,6'R)-6-[(4-ethoxyphenyl)methyl]-3',4',5',6'-tetrahydro-6'-(hydroxymethyl)-5-methyl-spiro[isobenzofuran-1(3H),2'-[2H]pyran]-3',4',5'-triol;
(1S,3'R,4'S,5'S,6'R)-3',4',5',6'-tetrahydro-6'-(hydroxymethyl)-5-methyl-6-[(4-propylphenyl)methyl]-spiro[isobenzofuran-1(3H),2'-[2H]pyran]-3',4',5'-triol;
(1S,3'R,4'S,5'S,6'R)-6-[(4-trifluoromethylphenyl)methyl]-3',4',5',6'-tetrahydro-6'-(hydroxymethyl)-5-methyl-spiro[isobenzofuran-1(3H),2'-[2H]pyran]-3',4',5'-triol;
(1S,3'R,4'S,5'S,6'R)-6-[(4-trifluoromethoxyphenyl)methyl]-3',4',5',6'-tetrahydro-6'-(hydroxymethyl)-5-methyl-spiro[isobenzofuran-1(3H),2'-[2H]pyran]-3',4',5'-triol;
(1S,3'R,4'S,5'S,6'R)-6-[(benzothiophen-2-yl)methyl]-5-chloro-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-6-[(5-ethylthiophen-2-yl)methyl]-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-[(thiophen-2-yl)methyl]-spiro[isobenzofuran-1(3H),2'-[2H]pyran]-3',4',5'-triol;
(1S,3'R,4'S,5'S,6'R)-6-[(4-ethylphenyl)methyl]-5-ethynyl-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-6-[(4-ethynylphenyl)methyl]-3',4',5',6'-tetrahydro-6'-(hydroxymethyl)-spiro[isobenzofuran-1(3H),2'-[2H]pyran]-3',4',5'-triol; and
(1S,3'R,4'S,5'S,6'R)-3',4',5',6'-tetrahydro-6'-(hydroxymethyl)-5-methyl-6-[(4-(propyn-1-yl)phenyl)methyl]-spiro[isobenzofuran-1(3H),2'-[2H]pyran]-3',4',5'-triol;
or a pharmaceutically acceptable salt or a solvate thereof.

**8.** The compound according to claim 1 selected from (1S,3'R,4'S,5'S,6'R)-5-chloro-6-[(4-ethylphenyl)methyl]-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-[(4-methylphenyl)methyl]-spiro[isobenzofuran-1(3H),2'-[2H]pyran]-3',4',5'-triol;
(1S,3'R,4'S,5'S,6'R)-5-chloro-3'.4',5',6'-tetrahydro-6'-(hydroxymethyl)-6-[(4-(methylthio)phenyl)methyl]-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-[(4-methoxyphenyl)methyl]-spiro[isobenzofuran-1(3H),2'-[2H]pyran]-3',4',5'-triol;
(1S,3'R,4'S,5'S,6'R)-5-chloro-6-[(4-ethoxyphenyl)methyl]-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-(2-naphthylmethyl)-spiro[isobenzofuran-1(3H),2'-[2H]pyran]-3',4',5'-triol;
(1S,3'R,4'S,5'S,6'R)-5-chloro-6-[(4-(2-fluoroethyl)phenyl)methyl]-3',4',5',6'-tetrahydro-6'-(hydroxymethyl)-spiro[isobenzofuran-1(3H),2'-[2H]pyran]-3',4',5'-triol;

(1S,3'R,4'S,5'S,6'R)-5-chloro-3',4',5',6'-tetrahydro-6'-(hydroxymethyl)-6-[(4-propylphenyl)methyl]-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-[(4-isopropylphenyl)methyl]-spiro[isobenzofuran-1(3H),2'-[2H]pyran]-3',4',5'-triol;
(1S,3'R,4'S,5'S,6'R)-3',4',5',6'-tetrahydro-6'-(hydroxymethyl)-6-[(4-isopropylphenyl)methyl]-5-methyl-spiro[isobenzofuran-1(3H),2'-[2H]pyran]-3',4',5'-triol;
(1S,3'R,4'S,5'S,6'R)-6-[(benzothiophen-2-yl)methyl]-5-chloro-3',4',5',6'-tetrahydro-6'-(hydroxymethyl)-spiro[isobenzofuran-1(3H),2'-[2H]pyran]-3',4',5'-trlol; and
(1S,3'R,4'S,5'S,5'R)-5-chloro-6-[(5-ethylthiophen-2-yl)methyl]-3',4',5',6'-tetrahydro-6'-(hydroxymethyl)-spiro[isobenzofuran-1(3H),2'-[2H]pyran]-3',4',5'-triol;
or a pharmaceutically acceptable salt or a solvate thereof.

**9.** The compound according to claim 1 selected from (1S,3'R,4'S,5'S,6'R)-5-chloro-6-[(4-ethylphenyl)methyl]-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-(2-naphthylmethyl)-spiro[isobenzofuran-1(3H),2'-[2H]pyran]-3',4',5'-triol;
(1S,3'R,4'S,5'S,6'R)-5-chloro-6-[(4-(2-fluoroethyl)phenyl)methyl]-3',4',5',6'-tetrahydro-6'-(hydroxymethyl)-spiro[isobenzofuran-1(3H),2'-[2H]pyran]-3',4',5'-trio;
(1S,3'R,4'S,5'S,6'R)-5-chloro-3',4',5',6'-tetrahydro-6'-(hydroxymethyl)-6-[(4-propylphenyl)methyl]-spiro[isobenzofuran-1(3H),2'-[2H]pyran]-3',4',5'-triol;
(1S,3'R,4'S,5'S,6'R)-5-chloro-6-[(4-ethoxyphenyl)methyl]-3',4',5',6'-tetrahydro-6'-(hydroxymethyl)-spiro[isobenzofuran-1(3H),2'-[2H]pyran]-3',4',5'-triol;
(1S,3'R,4'S,5'S,6'R)-5-chloro-3',4',5',6'-tetrahydro-6'-(hydroxymethyl)-6-[(4-isopropylphenyl)methyl]-spiro[isobenzofuran-1(3H),2'-[2H]pyran]-3',4',5'-triol; and
(1S,3'R,4'S,5'S,6'R)-3',4',5',6'-tetrahydro-6'-(hydroxymethyl)-6-[(4-isopropylphenyl)methyl]-5-methyl-spiro[isobenzofuran-1(3H),2'-[2H]pyran]-3',4',5'-triol;
or a pharmaceutically acceptable salt or a solvate thereof.

**10.** The compound according to any one of claims 1 to 9 or a pharmaceutically acceptable salt or a solvate thereof used as a $Na^+$-glucose cotransporter inhibitor.

**11.** The compound according to any one of claims 1 to 9 or a pharmaceutically acceptable salt or a solvate thereof used for prevention or treatment of diabetes, hyperglycemia, diabetic complications and obesity.

**12.** A pharmaceutical composition comprising the compound according to any one of claims 1 to 9, or a pharmaceutically acceptable salt thereof.

**13.** A pharmaceutical composition for use in preventing or treating diabetes, hyperglycemia, diabetic complications induced thereby, or obesity, which comprises the compound according to any one of claims 1 to 9, or a pharmaceutically acceptable salt thereof.

**14.** A pharmaceutical composition accordig to claim 13 wherein diabetes is insulin-dependent diabetes (Type 1 diabetes) or non-insulin-dependent diabetes (Type 2 diabetes).

## INTERNATIONAL SEARCH REPORT

International application No.
PCT/JP2007/064806

A. CLASSIFICATION OF SUBJECT MATTER
*C07H19/01*(2006.01)i, *A61K31/7048*(2006.01)i, *A61P3/04*(2006.01)i, *A61P3/10*(2006.01)i, *A61P43/00*(2006.01)i

According to International Patent Classification (IPC) or to both national classification and IPC

B. FIELDS SEARCHED

Minimum documentation searched (classification system followed by classification symbols)
C07H19/01, A61K31/7048, A61P3/04, A61P3/10, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
CAOLD(STN), CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| P,X | WO 2006/080421 A1 (Chugai Pharmaceutical Co., Ltd.), 03 August, 2006 (03.08.06), Full text (Family: none), | 1-14 |
| A | WO 2005/012326 A1 (TANABE SEIYAKU CO., LTD.), 10 February, 2005 (10.02.05), & JP 2007-518683 A & US 2005/0233988 A1 & US 2006/0217323 A1 & US 2006/0229260 A1 & US 2006/0234954 A1 & US 2006/0293251 A1 & EP 1651658 A1 & CA 2534024 A & NO 2006000220 A & AU 2004260761 A1 & CN 1829729 A & BR 2004013232 A | 1-14 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>22 August, 2007 (22.08.07) | Date of mailing of the international search report<br>04 September, 2007 (04.09.07) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No.
PCT/JP2007/064806

C (Continuation). DOCUMENTS CONSIDERED TO BE RELEVANT

| Category* | Citation of document, with indication, where appropriate, of the relevant passages | Relevant to claim No. |
|---|---|---|
| A | WO 01/68660 A1 (Kissei Pharmaceutical Co., Ltd.), 20 September, 2001 (20.09.01), & JP 3773450 B2 & JP 2006-143735 A & EP 1270584 A1 & US 2004/0053855 A1 & US 2005/0080022 A1 & AU 200141146 A & NO 200200424 A & ZA 2002007418 A & BR 2001009323 A & BG 107102 A & CA 2402609 A & HU 200300057 A & NZ 521369 A & HK 1055973 A | 1-14 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 2000080041 A
- WO 01068660 A
- WO 04007517 A
- WO 0106860 A
- US 2001041674 A
- US 2002137903 A
- WO 01027128 A
- WO 02083066 A
- WO 04013118 A
- WO 03099836 A
- WO 04080990 A
- US 20050209166 A
- WO 05085237 A
- WO 05085265 A
- WO 05012318 A
- WO 05012326 A
- US 20060063722 A
- US 20060035841 A
- US 20060074031 A
- WO 06002912 A
- WO 06008038 A
- WO 06010557 A
- JP 2006301284 W
- WO 2006080421 A
- US 5712396 A
- US 5739135 A
- US 5712279 A
- US 5760246 A
- JP 2006030128 W

### Non-patent literature cited in the description

- *J. Clin. Invest.,* 1994, vol. 93, 397
- *Stroke,* 1983, vol. 14, 388
- *Prog. Med.,* 1985, vol. 5, 2157-2161
- molecular design. Iyakuhin no Kaihatsu. Hirokawa Shoten, 1990, vol. 7, 163-198
- **GREENE ; WUTS.** Protective Groups in Organic Synthesis
- *Carbohydr. Res.,* 1994, 243
- *J. Prakt. Chem.,* vol. 39 (2), 402
- *J. Org. Chem.,* 1975, vol. 40 (21), 3101
- *J. Org. Chem.,* 2005, 756
- **D. G. HALL.** Boronic Acids: Preparation And Applications In Organic Synthesis And Medicines. WILEY-VCH
- *Eur. J. Org. Chem.,* 2003, 821
- *Tetrahedron: Asymmetry,* 1993, vol. 4 (10), 2183